# EUROPEAN PATENT APPLICATION

(11) **EP 4 389 223 A1**
(43) Date of publication of application: **26.06.2024**
(21) Application number: 22215045.0
(22) Date of filing: 20.12.2022
(51) Int. Cl.: A61P 35/00, C07D 413/14, A61K 31/422, A61K 31/496, A61K 31/506, A61K 31/454, A61K 31/5377, A61K 31/5513

(54) **SYNTHESIS, PHARMACOLOGY AND USE OF NEW WATER-SOLUBLE AND SELECTIVE FMS-LIKE TYROSINE KINASE 3 (FLT3) INHIBITORS**

(71) Applicant: Universität Regensburg, 93053 Regensburg (DE)
(72) Inventor: The designation of the inventor has not yet been filed
(74) Representative: Engelhard, Markus

(57) **Abstract**

The present application relates to compounds of formula (I), which inhibit FLT3. The present application further relates to a composition, preferably pharmaceutical composition, comprising a compound of formula (I) or a pharmaceutically acceptable salt thereof and comprising a pharmaceutically acceptable excipient and/or carrier. The present application also relates to a compound of formula (I) or composition thereof for use in medicine. The present application also relates to a compound of formula (I) or composition thereof for use in a method of preventing or treating cancer, preferably blood cancer, more preferably leukemia, even more preferably acute myeloid leukemia (AML). Furthermore, the present application relates to a method of preparing a compound of formula (I).

## Description

### FIELD OF THE INVENTION

The present invention relates to compounds for inhibiting FLT3. The present invention further relates to a composition, preferably pharmaceutical composition, comprising a compound or a pharmaceutically acceptable salt thereof and comprising a pharmaceutically acceptable excipient and/or carrier. The present invention also relates to a compound or composition for use in medicine. The present invention also relates to a compound or composition for use in a method of preventing or treating cancer, preferably blood cancer, more preferably leukemia, even more preferably acute myeloid leukemia (AML). Furthermore, the present invention relates to a method of preparing a compound.

### BACKGROUND OF THE INVENTION

The class III receptor tyrosine kinases (RTK) are required for normal hematopoiesis and they are frequently dysregulated in AML. Therefore, RTK are targets for tailored intervention strategies against AML. Enzymes belonging to this protein family are FMS-like tyrosine kinase 3 (FLT3), macrophage colony stimulating factor receptor (CSF1R, c-FMS), stem cell factor receptor (SCFR, KIT), and plateletderived growth factor receptor (PDGFRα/β). Each of these factors consists of five immunoglobulinelike domains in the extracellular domain, a transmembrane domain (TMD), a juxtamembrane domain (JM), and two intracellular kinase domains, divided by a kinase insert domain.

FLT3 is expressed on hematopoietic stem and progenitor cells and plays an indispensable role in stem cell development and cell differentiation. FLT3 is activated upon the binding of its ligand. After the ligand-induced dimerization and autophosphorylation of the receptor, the signal transduction pathway is initiated. Mutations in FLT3 can be found in about one-third of all patients with AML and such aberrations are associated with poor prognosis. These lesions cause an aberrant activation of FLT3 and its downstream signaling pathway. Of note, recent evidence shows that mutant FLT3 is an oncogenic driver in AML. Different activating FLT3 mutations are known. 1) Activating FLT3 internal tandem duplications (FLT3-ITD) locate within the JM domain of FLT3. These are seen in 15-35% of the patients suffering from AML. Point mutations in the activation loop of the kinase domain (FLT3-KD) occur in 6-8% of patients with AML. Point mutations in the JM are detectable in 2% of AML patients. The most common of these mutations, FLT3-ITD, induces the loss of the auto-inhibitory functions whereby the kinase becomes constitutively active. This leads to altered intracellular signaling and autonomous cell growth. Due to the frequency of FLT3 mutations in AML, the inhibition of FLT3 and its downstream signaling pathways have attracted significant attention in the discovery of new anti-cancer drugs. Most of the potential therapeutics currently under development are direct inhibitors of the FLT3 receptor. Inhibitors of FLT3 are classified into the first and the second generation. The first generation of small molecule inhibitors include midostaurin (PKC412), lestaurtinib (CEP-701), semaxanib (SU 5416) and sunitinib (SU 11248). They are generally multi-kinase inhibitors and their efficacies are limited, not durable, and at the expense of undesired side effects.

Therefore, a second generation of TKi against FLT3-ITD has been developed to yield more selective and highly potent agents for treating AML and to overcome resistance. Prominent representatives for the second generation are tandutinib (MLN518), KW-2449 and quizartinib (AC 220). During therapy with FLT3 TKIs it becomes apparent that a significant proportion of a secondary FLT3-TKD mutation emerges, and this positions FLT3-ITD as a driver of leukemogenesis. Mutations in position D835 within the activation loop, the gatekeeper F691, or N676 have independently been confirmed to cause a reduction or a loss of response to FLT3 TKi. Mutations at the activation loop residue D835 stabilize an enzymatically active kinase conformation which cannot accommodate type II FLT3 inhibitors (i.e. such mutations fix the Asp-Phe-Gly motif in the so-called DFG-in conformation), and gatekeeper mutations impair the binding of AC220. These type II FLT3 TKi appear to bind only to the so-called DFG-out conformation of the inactive kinase; type I inhibitors bind the ATP binding site and adjacent residues. Especially bulky hydrophobic substitutions (D→Y/V/I/F) at D835 disrupt the interaction of this site with S838 within FLT3-ITD and thereby confer resistance to AC220 and other type II FLT3 TKi. Intense efforts have yielded compounds that also target FLT3-ITD with mutations in its activation loop. Examples are crenolanib, G-749, TIT-3002, as well as dual kinase inhibitors (e.g., CCT137690, against FLT3 and Aurora kinase). The benzamidine quinolone crenolanib, which was originally developed as PDGFR inhibitor, is currently the best described FLT3 TKi against therapy-associated FLT3-ITD mutants. Independent groups have shown that crenolanib exerts type I TKi properties against FLT3-ITD and mutants thereof, i.e. the ability to target kinases in the active, DFG-in conformation. Crenolanib shows activity against wild-type FLT3-ITD and FLT3-ITD with secondary KD mutations at D835 or F691 *in vitro* and *in vivo.* Although type I TKi often fail to exert inhibitor specificity, crenolanib inhibits FLT3-ITD and its KD mutants about 100-fold more potently than KIT. The latter is important because a combined knock-out of FLT3 and KIT is detrimental for normal hematopoiesis, and because the potent inhibition of FLT3 plus KIT by AC220 caused severe myelosuppression in leukemic patients. Crenolanib delayed the outgrowth of MV4-11 cells in a xenograft mouse model and only by combination with the type II TKI sorafenib, a significant decrease in leukemic burden and prolonged survival was observed compared with either type I or II TKI alone.

Recently the benzofuranylmethanone marbotinib was shown to be effective against FLT3-ITD positive cell lines MV4-11 in xenotransplantation mouse models and to exhibit activity in therapy-associated FLT3-ITD mutants in cell cultures (Mahboobi, Sellmer et al. 2019). However, there remains the need for improved FLT3 inhibitors. Particularly, there remains the need for compounds with enhanced bioavailability. Furthermore, there remains the need for compounds with increased solubility. There also remains the need for compounds and compositions that efficiently treat cancer such as blood cancer. Moreover, there remains the need for compounds with improved tolerability.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a compound having the general formula I: 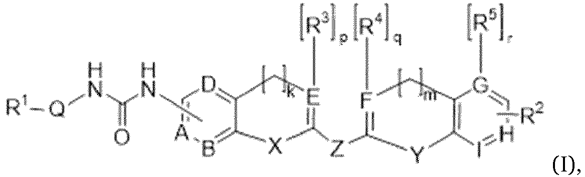 wherein
**A, B, D, H,** and **I** are independently selected from CH and N, preferably CH;
**E, F,** and **G** are independently selected from C and N, preferably C;
**k** and **m** are independently selected from 0 and 1, preferably 0;
**p, q,** and **r** are independently selected from 0 and 1, wherein at least one of **p, q** and **r** is 1, preferably each of **p, q** and **r** is 1; wherein, if p is 1, then **E** is C, and if p is 0, then **E** is N; wherein, if q is 1, then **F** is C, and if q is 0, then **F** is N; wherein, if r is 1, then G is C, and if r is 0, then G is N;
**X** and **Y** are independently selected from NH, O, and S, preferably from NH and O; wherein, preferably, X is O and/or Y is NH;
**Z** is selected from C=O, C=S, and CHOH, preferably is C=O;
**Q** is selected from the group consisting of five- or six-membered aromatic systems and five- or six-membered heteroaromatic systems, preferably is isoxazole;
**R¹** is selected from hydrogen and alkyl, preferably C₁-C₆ alkyl, more preferably *tert*-butyl;
**R²** is selected from the group consisting of hydrogen, alkyl, preferably C₁-C₆ alkyl, OH, ester, carbamate, alkylamino, preferably C₁-C₆ alkylamino, alkoxy, preferably C₁-C₆ alkoxy, 2-hydroxysuccinyl, an amino acid residue, preferably a basic amino acid residue, and any structure of the following group **R⁶** 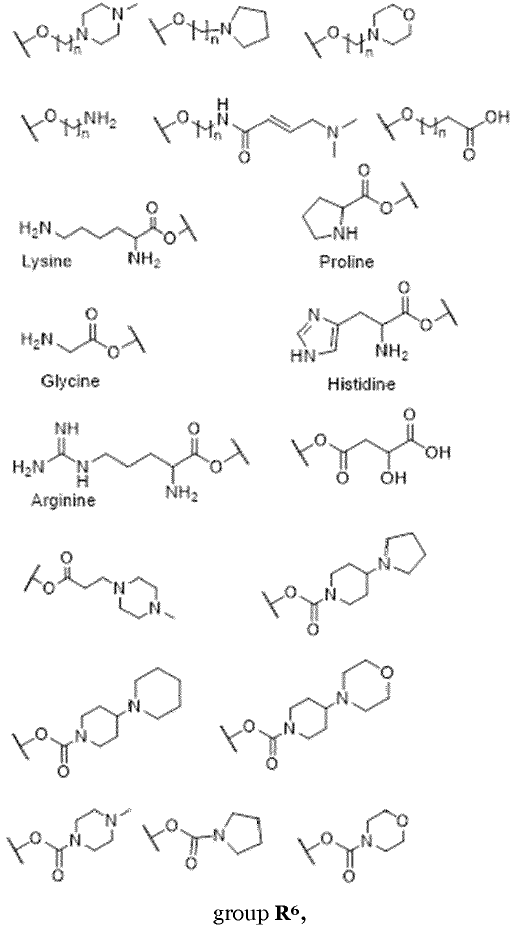
wherein **n** is independently selected from 0, 1, 2, 3, 4, 5, and 6,
wherein each of said alkyl, OH, ester, carbamate, alkylamino, and alkoxy is optionally substituted with a substituent selected from the group consisting of amino, alkylamino, cyclic alkylamino, cyclic diaminoalkyl, preferably piperazinyl or 1-alkylpiperazinyl, and heterocyclic alkylamino, preferably morpholinyl;
**R³** is selected from the group consisting of hydrogen, alkyl, preferably C₁-C₆ alkyl, alkylamino, preferably C₁-C₆ alkylamino, cyclic alkylamino, cyclic diaminoalkyl, such as piperazinyl or 1-alkylpiperazinyl, heterocyclic alkylamino, such as morpholinyl, and any structure of the following group **R⁷** 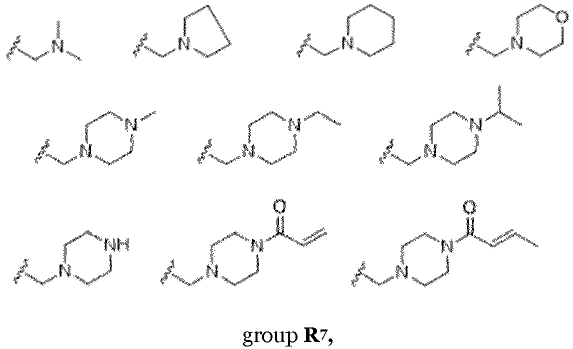
wherein each of said alkyl, alkylamino, cyclic alkylamino, cyclic diaminoalkyl, and heterocyclic alkylamino is optionally substituted;
**R⁴** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁸** 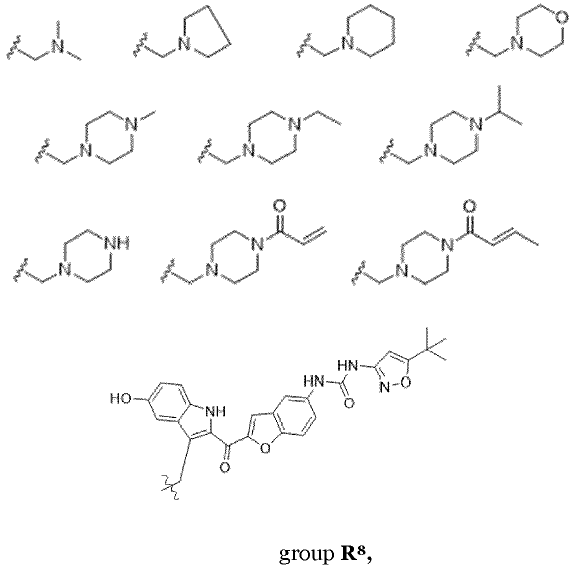
wherein each of said alkyl, alkylamino, amino, dialkylamino, cyclic alkylamino, cyclic diaminoalkyl, and heterocyclic alkylamino is optionally substituted;
**R⁵** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁹** 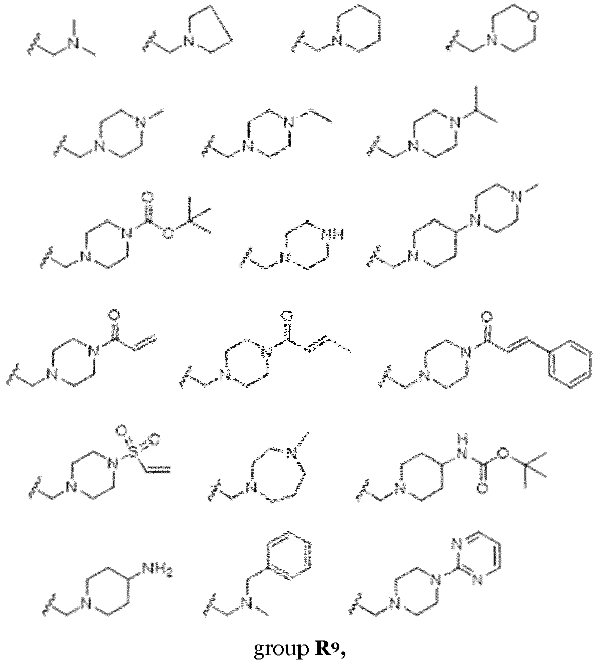
wherein, preferably, **R⁵** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R¹⁰** 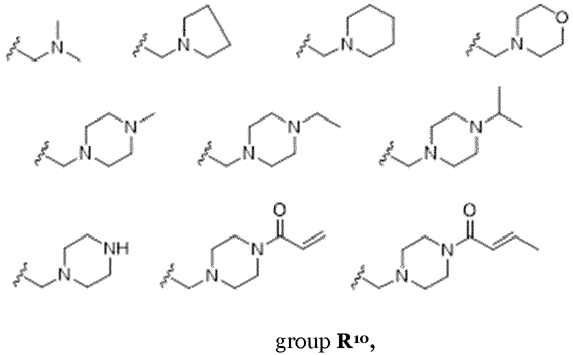
wherein each of said alkyl, alkylamino, amino, dialkylamino, cyclic alkylamino, cyclic diaminoalkyl, and heterocyclic alkylamino is optionally substituted;
under the proviso that at least one of **R³**, **R⁴**, and **R⁵** is not hydrogen;
and pharmaceutically acceptable salts or solvates thereof.

In one embodiment, the compound of the present invention has the general formula II 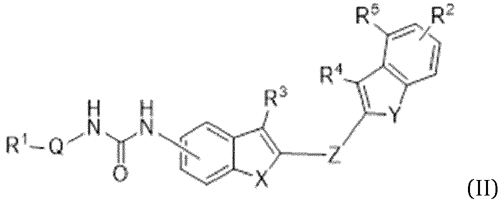 wherein
**X** and **Y** are independently selected from NH, O, and S, preferably from NH and O; wherein, preferably, X is O and/or Y is NH;
**Z** is selected from C=O, C=S, and CHOH, preferably is C=O;
**Q** is selected from the group consisting of five- or six-membered aromatic systems and five- or six-membered heteroaromatic systems, preferably isoxazole;
**R¹** is selected from hydrogen and alkyl, preferably C₁-C₆ alkyl, more preferably tert-butyl;
**R²** is selected from the group consisting of hydrogen, alkyl, preferably C₁-C₆ alkyl, OH, ester, carbamate, alkylamino, preferably C₁-C₆ alkylamino, alkoxy, preferably C₁-C₆ alkoxy, 2-hydroxysuccinyl, an amino acid residue, preferably a basic amino acid residue, and any structure of the following group **R⁶** 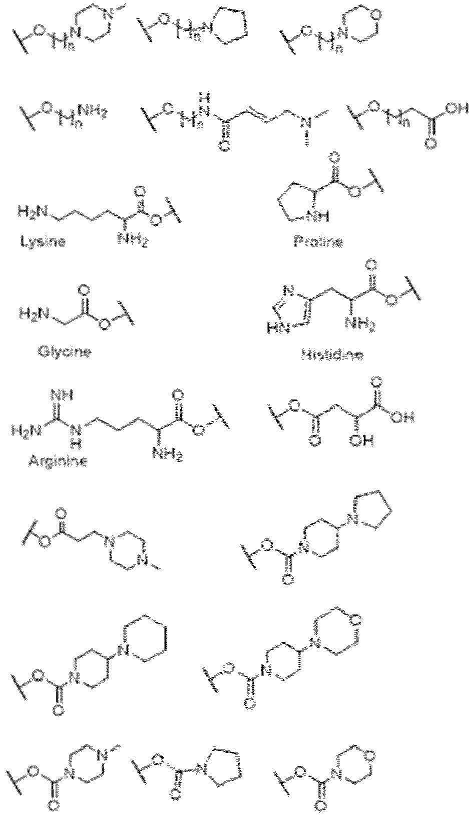 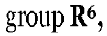
wherein **n** is independently selected from 0, 1, 2, 3, 4, 5, and 6,
wherein each of said alkyl, OH, ester, carbamate, alkylamino, and alkoxy is optionally substituted with a substituent selected from the group consisting of amino, alkylamino, cyclic alkylamino, cyclic diaminoalkyl, preferably piperazinyl or 1-alkylpiperazinyl, and heterocyclic alkylamino, preferably morpholinyl;
**R³** is selected from the group consisting of hydrogen, alkyl, preferably C₁-C₆ alkyl, alkylamino, preferably C₁-C₆ alkylamino, cyclic alkylamino, cyclic diaminoalkyl, such as piperazinyl or 1-alkylpiperazinyl, heterocyclic alkylamino, such as morpholinyl, and any structure of the following group **R⁷** 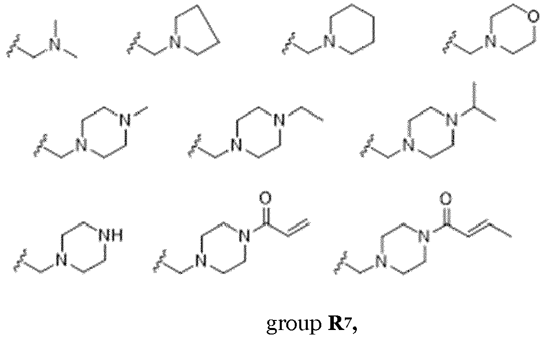
wherein each of said alkyl, alkylamino, cyclic alkylamino, cyclic diaminoalkyl, and heterocyclic alkylamino is optionally substituted;
**R⁴** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁸** 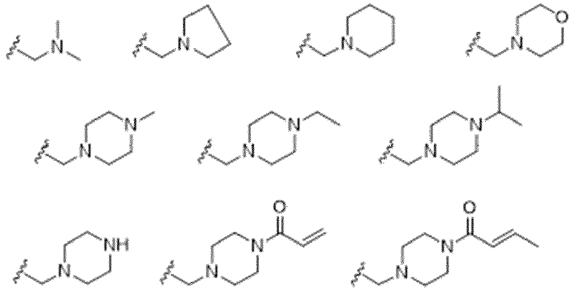 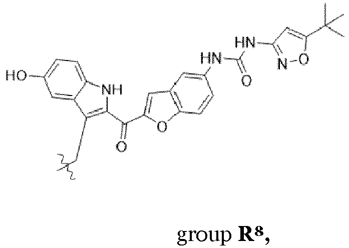
wherein each of said alkyl, alkylamino, amino, dialkylamino, cyclic alkylamino, cyclic diaminoalkyl, and heterocyclic alkylamino is optionally substituted;
**R⁵** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁹** 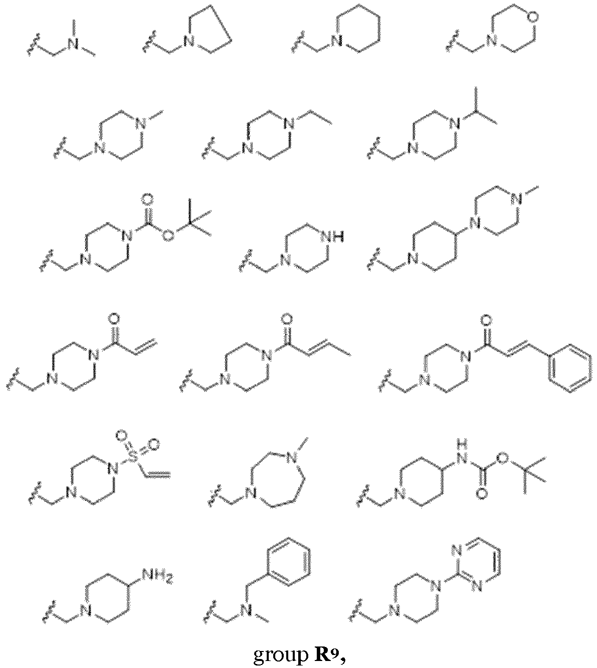
wherein, preferably, **R⁵** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R¹⁰** 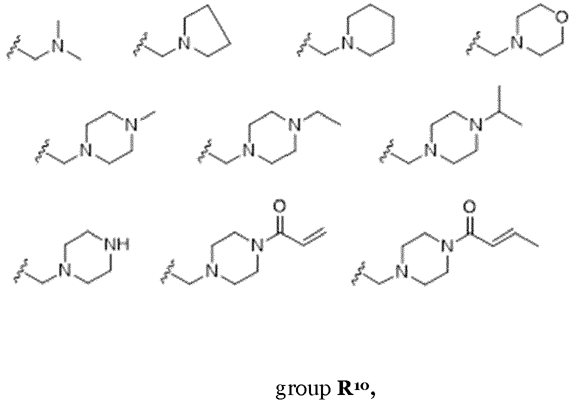
wherein each of said alkyl, alkylamino, amino, dialkylamino, cyclic alkylamino, cyclic diaminoalkyl, and heterocyclic alkylamino is optionally substituted;
under the proviso that at least one of **R³**, **R⁴**, and **R⁵** is not hydrogen;
and pharmaceutically acceptable salts or solvates thereof.

In one embodiment, **Q** is isoxazole; and/or **R¹** is selected from C₁-C₆ alkyl, preferably is *tert*-butyl.

In one embodiment, **X** is O; **Y** is NH; and/or **Z** is C=O. In a preferred embodiment, **Q** is isoxazole; **R¹** is selected from C₁-C₆ alkyl, preferably is *tert*-butyl; **X** is O; **Y** is NH; and **Z** is C=O.

In one embodiment, **R²** is OH, carbamate, alkylamino, or an amino acid residue; preferably is OH or carbamate, more preferably is OH or

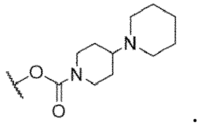

In one embodiment, each of p, q, and r is 1, and wherein **R³** is selected from hydrogen and alkyl, preferably selected from hydrogen and C₁-C₆ alkyl, more preferably selected from hydrogen and methyl; under the proviso that, if **R³** is hydrogen, then at least one of **R⁴** and **R⁵** is not hydrogen.

In one embodiment, the compound of the present invention has the general formula III 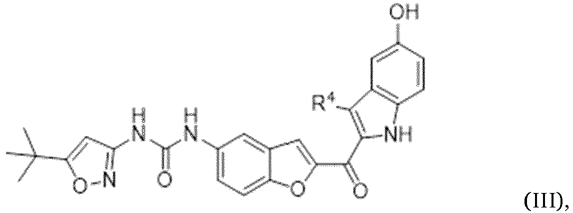 wherein
**R⁴** is selected from the group consisting of alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁸** 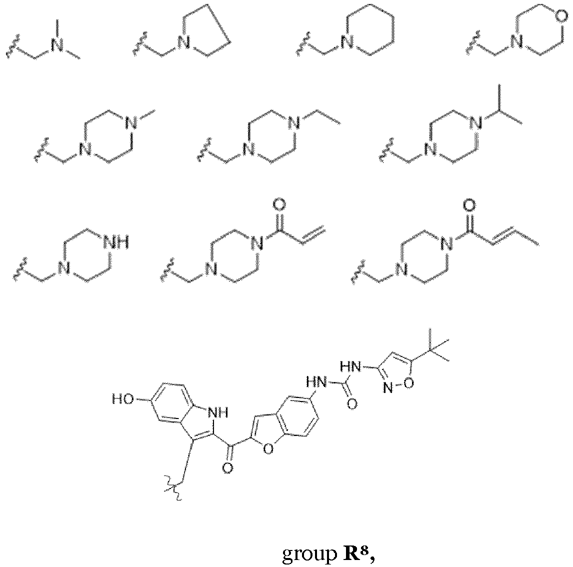
wherein each of said alkyl, alkylamino, amino, dialkylamino, cyclic alkylamino, cyclic diaminoalkyl, and heterocyclic alkylamino is optionally substituted;
wherein, preferably, **R⁴** is selected from alkyl, preferably C₁-C₆ alkyl, more preferably methyl, and any structure of the above group **R⁸**;
wherein, more preferably, **R⁴** is selected from alkyl, preferably C₁-C₆ alkyl, more preferably methyl, and any structure of the following group **R¹¹** 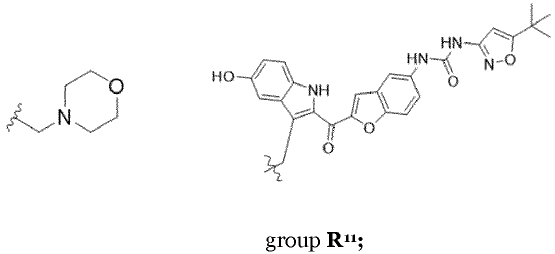
and pharmaceutically acceptable salts or solvates thereof.

In one embodiment, the compound of the present invention has the general formula IV 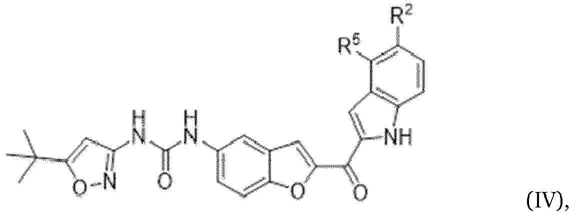 wherein
**R²** is selected from the group consisting of OH and carbamate, preferably is selected from OH and 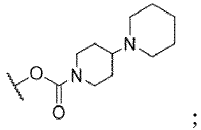
**R⁵** is selected from the group consisting of alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁹** 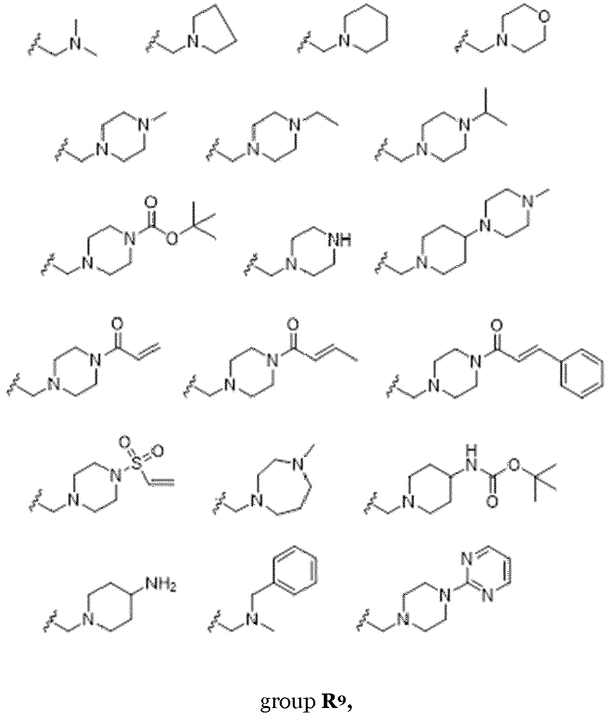
wherein, preferably, **R⁵** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R¹⁰** 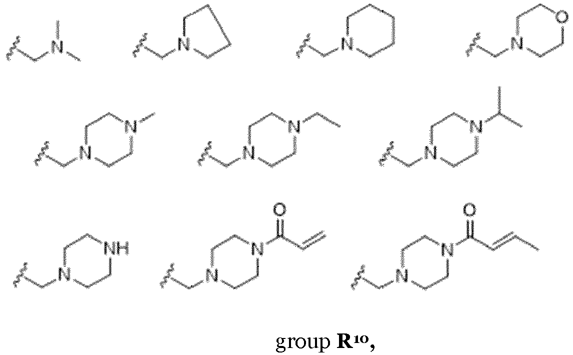
wherein each of said alkyl, alkylamino, amino, dialkylamino, cyclic alkylamino, cyclic diaminoalkyl, and heterocyclic alkylamino is optionally substituted;
wherein, more preferably, **R⁵** is selected from any structure of the above group **R⁹;**
wherein, even more preferably, **R⁵** is selected from any structure of the above group **R¹⁰;**
and pharmaceutically acceptable salts or solvates thereof.

In one embodiment, **R³** is hydrogen; and
wherein **R⁵** is hydrogen, and **R⁴** is selected from alkyl, preferably C₁-C₆ alkyl, more preferably methyl, and any structure of the following group **R⁸** 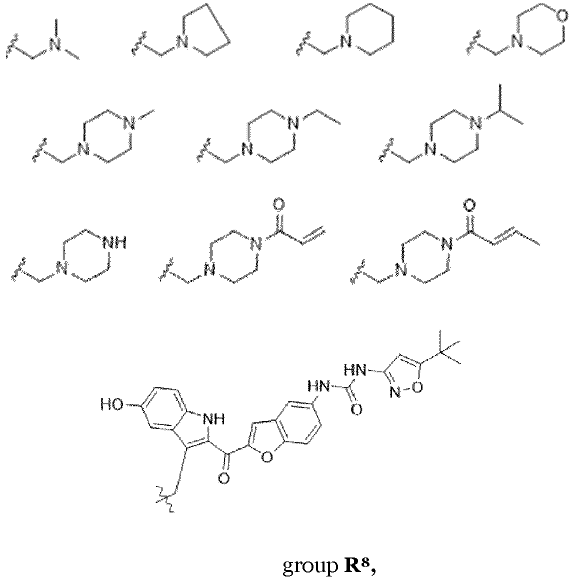
**R⁴** preferably being selected from alkyl, preferably C₁-C₆ alkyl, more preferably methyl, and any structure of the following group **R¹¹** 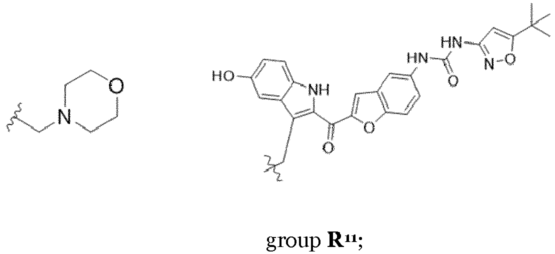 or
wherein **R⁴** is hydrogen, and **R⁵** is selected from any structure of the following group **R⁹** 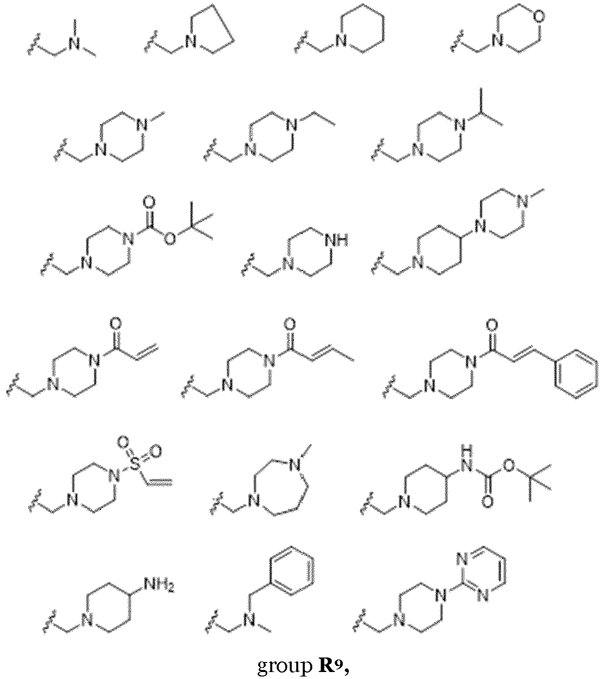
wherein, preferably, **R⁵** is selected from any structure of the following group **R¹⁰** 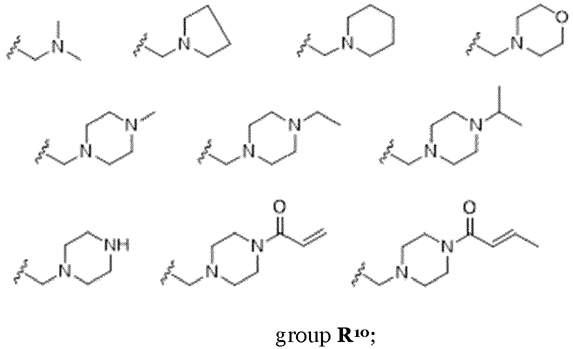
and pharmaceutically acceptable salts or solvates thereof.

In one embodiment, said salts are selected from hydrochlorides, trifluoroacetates, sulfates, phosphates, mesylates, tosylates, formiates, and acetates, preferably selected from hydrochlorides and trifluoroacetates; and/or, if **R²** = OH, a salt of the respective phenolate comprising sodium, potassium, calcium, zinc or another pharmaceutically acceptable cation forming a soluble salt.

In one embodiment, the compound of the invention has one of the structures as shown in Table 1 or a pharmaceutically acceptable salt or solvate thereof.

In a further aspect, the present invention relates to a composition, preferably pharmaceutical composition, comprising a compound or a pharmaceutically acceptable salt thereof, as defined herein, and a pharmaceutically acceptable excipient and/or carrier.

In one embodiment, said composition further comprises a further active agent, preferably a drug.

In a further aspect, the present invention relates to the compound, as defined herein, or the composition, as defined herein, for use in medicine.

In a further aspect, the present invention relates to the compound, as defined herein, or the composition, as defined herein, for use in a method of preventing or treating cancer, preferably blood cancer, more preferably leukemia, even more preferably acute myeloid leukemia (AML).

In one embodiment, said method of preventing or treating cancer comprises administering said compound or said composition to a patient, preferably a patient characterized by a mutation in the *Flt₃* gene.

In a further aspect, the present invention relates to a method of preparing a compound, as defined herein, comprising the steps:
(1a) heating a solution of a starting material, preferably a starting material having a structure represented by 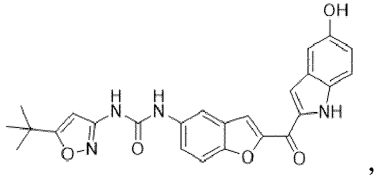 in a solvent, preferably dimethylformamide, to a temperature in a range of from 60 °C to 100 °C, preferably of from 70 °C to 90 °C, more preferably of about 80 °C; and
   adding an aldehyde, preferably formaldehyde, paraformaldehyde, or acetaldehyde, and a primary or secondary amine, preferably dimethylamine, piperidine, pyrrolidine, morpholine, or 1-methylpiperazine, to said solution;
(1b) deprotecting a benzyloxy group of a first intermediate compound obtained in step (1a);
(1c) reduction of a nitro group of said first intermediate compound;
(1d) reaction of a second intermediate compound obtained in step (1b) and/or (1c) with an isocyanate, preferably an isocyanate having a structure represented by 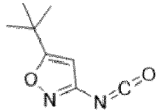 ; and
(1e) obtaining a compound, as defined herein;
or comprising the steps:
(2aa) heating a solution of a starting material, preferably a starting material having a structure represented by 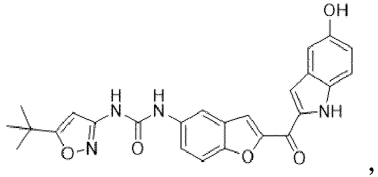 in a solvent, preferably dimethylformamide or ethanol, to a temperature in a range of from 40 °C to 100 °C, preferably of from 50 °C to 90 °C, more preferably of from about 60 °C to about 80 °C; and
   adding an aldehyde, preferably formaldehyde, paraformaldehyde, or acetaldehyde, and a primary or secondary amine, preferably dimethylamine, piperidine, pyrrolidine, morpholine, or 1-methylpiperazine, to said solution; or
(2ab) mixing, preferably stirring, a primary or secondary amine and an aldehyde, preferably formaldehyde, paraformaldehyde, or acetaldehyde, dissolved in a carboxylic acid, preferably glacial acetic acid, and adding a starting material, preferably a starting material having a structure represented by 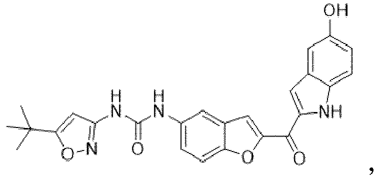 dissolved in a carboxylic acid, preferably glacial acetic acid; and
(2b) adding to a solution obtained in step (2aa) or in step (2ab), optionally in a mixture of dichloromethane:pyridine, a solution of [1,4'-bipiperidine]-1'-carbonylchloride, optionally in a mixture of dichloromethane:pyridine; and
(2c) obtaining a compound, as defined herein;
or comprising the steps:
(3a) acidic cleavage of a tert-butoxycarbonyl group of 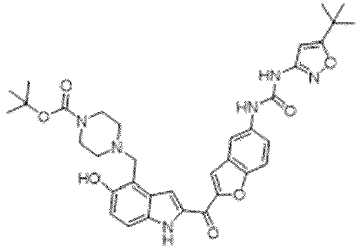 , preferably using trifluoro acetic acid;
(3b) reaction with an acid chloride or an acidic anhydride, preferably with acryloyl chloride or crotonic acid chloride; optionally in the presence of a base, preferably triethylamine, diisopropylethylamine, or pyridine; and
(3c) obtaining a compound, as defined herein;
or comprising the steps:
(4a) hydrogenolytic cleavage of a benzyloxy group of a compound and reduction of a nitro group of said compound; wherein, optionally, said compound has a structure represented by 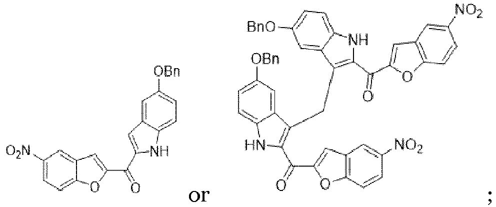
(4b) reaction of said compound with an isocyanate, preferably an isocyanate having a structure represented by 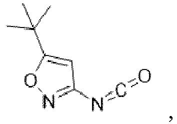 to obtain an urea derivative of said compound; wherein, optionally, said urea derivative has a structure represented by 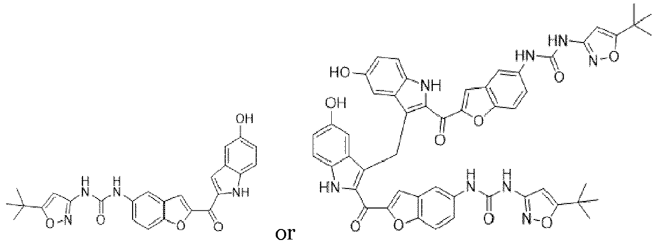 ; and
(4c) obtaining a compound, as defined herein;
or comprising the steps:
(5a) aromatic nitration of a starting material, preferably a compound having a structure represented by 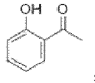 , to obtain a first intermediate compound, preferably a first intermediate compound having a structure represented by 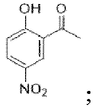 wherein, preferably, said aromatic nitration is performed using nitric acid and acetic acid;
(5b) reaction of 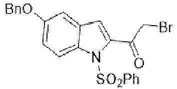 with said first intermediate compound in the presence of a base, preferably K₂CO₃, to obtain a second intermediate compound, preferably a second intermediate compound having a structure represented by 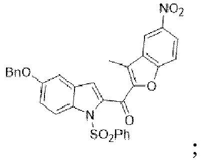
(5c) cleavage of a phenylsulfonyl-protection group of said second intermediate compound by a base, preferably NaOH, to obtain a third intermediate compound, preferably a third intermediate compound having a structure represented by 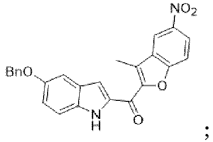
(5d) catalytic hydrogenolytic cleavage of a benzyloxy group of said third intermediate compound and reaction of a nitro group of said third intermediate compound, preferably to obtain a fourth intermediate compound having a structure represented by 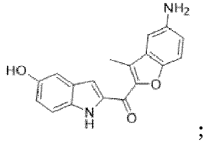
(5e) reaction with an isocyanate, preferably an isocyanate having a structure represented by 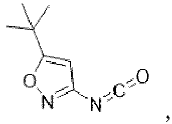 to obtain an urea derivative, preferably a compound having a structure represented by 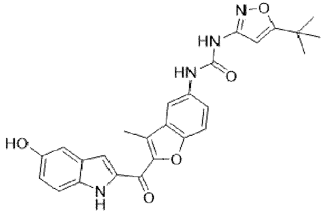 ; and
(5f) obtaining a compound, as defined herein;
or comprising the steps:
(6a) transformation of a compound to a pharmaceutically acceptable salt by treatment with an acid, preferably an acid selected from the group consisting of hydrochloric acid, trifluoroacetic acid, and a sulfonic acid such as methanesulfonic acid; and
(6b) obtaining a compound, as defined herein.

In a further aspect, the present invention relates to a method of preventing or treating cancer, preferably blood cancer, more preferably leukemia, even more preferably acute myeloid leukemia (AML), comprising administering a compound, as defined herein, or a composition, as defined herein, to a patient in need thereof, preferably a patient characterized by a mutation in the Flt₃ gene.

In one embodiment, said administering comprises administering an effective amount of a compound, as defined herein, or a composition, as defined herein, to a patient in need thereof.

In a further aspect, the present invention relates to a use of a compound, as defined herein, or a composition, as defined herein, for the manufacture of a medicament; preferably for the manufacture of a medicament for preventing or treating cancer, preferably blood cancer, more preferably leukemia, even more preferably acute myeloid leukemia (AML).

### DETAILED DESCRIPTION

It is an aim of the present invention to provide improved FLT3 inhibitors. Furthermore, it is an aim of the present invention to provide compounds for use in the treatment of cancer, such as acute myeloid leukemia (AML). It is also an aim of the invention to provide compounds with improved solubility. Furthermore, it is an aim of the invention to provide compounds, particularly FLT3 inhibitors, with enhanced bioavailability. The compounds of the present invention are enhanced tyrosine kinase inhibitors, in particular FLT3 inhibitors.

In one embodiment, the compound of the present invention has the general formula (I), (II), (III), or (IV). The inventors have found that a compound having the general formula (I), (II), (III), or (IV) is highly effective in inhibiting FLT3. Furthermore, the inventors have found that, advantageously, a compound having the general formula (I), (II), (III), or (IV) has increased solubility and enhanced bioavailability compared to compounds such as marbotinib. In one embodiment, **p, q,** and **r** are independently selected from 0 and 1, wherein at least one of **p, q** and **r** is 1. In one embodiment, at least two of **p, q** and **r** are 1. In a preferred embodiment, each of **p, q** and **r** is 1. In one embodiment, each of **p, q** and **r** is 1, and at least one of **R³**, **R⁴**, and **R⁵** is not hydrogen. In a preferred embodiment, each of **p, q** and **r** is 1, one of **R³**, **R⁴**, and **R⁵** is not hydrogen, and two of **R³**, **R⁴**, and **R⁵** are hydrogen. The inventors have found that the solubility of the compound is unexpectedly high, if each of **p, q** and **r** is 1, and one of **R³**, **R⁴**, and **R⁵** is not hydrogen.

The inventors have found that the solubility of a compound of the present invention is advantageously enhanced compared to compounds such as marbotinib, e.g. in the following scenarios:
In one embodiment, **R³** and **R⁴** are hydrogen and **R⁵** is selected from group **R⁹** or group **R¹⁰**. In one embodiment, **R³** and **R⁴** are hydrogen and **R⁵** is selected from group **R⁹**. In one embodiment, **R³** and **R⁴** are hydrogen and **R⁵** is selected from group **R¹⁰**. In one embodiment, **R³** and **R⁵** are hydrogen and **R⁴** is selected from alkyl e.g. methyl, group **R⁸**, and group **R¹¹**. In one embodiment, **R³** and **R⁵** are hydrogen and **R⁴** is selected from alkyl and group **R⁸**. In one embodiment, **R³** and **R⁵** are hydrogen and **R⁴** is selected from alkyl and group **R¹¹**. In one embodiment, **R⁴** and **R⁵** are hydrogen and **R³** is selected from alkyl and group **R⁷**.

In one embodiment, when referring to the expression "under the proviso that at least one of **R³**, **R⁴**, and **R⁵** is not hydrogen", such expression is meant to be understood as referring to a scenario in which at least one of **p, q** and **r** is 1, and the corresponding substituent (i.e. **R³** if p is 1, **R⁴** if q is 1, or **R⁵** if r is 1) is selected from substituents other than hydrogen, and also referring to a scenario in which the expression is used interchangeably with "under the proviso that each of **p, q** and **r** is 1, and at least one of **R³**, **R⁴**, and **R⁵** is not hydrogen". In one embodiment, when referring to **R³**, **R⁴**, and/or **R⁵** as being "not hydrogen", it is meant to be understood that such expression (e.g. "is not hydrogen") refers to the respective substituent, i.e. **R³**, **R⁴**, and/or **R⁵,** as being present, but being selected from substituents other than hydrogen. For example, when referring to **"Rs** is not hydrogen", such expression means that **r** is 1, and **R⁵** is selected from substituents other than hydrogen, e.g. selected from group **R⁹**.

Advantageously, the compound of the invention has high efficiency and good solubility, particularly enhanced solubility compared to marbotinib. For example, the inventors have found that the following compounds exhibit high efficiency and enhanced solubility:
In one embodiment, each of **p, q** and **r** is 1, each of **R³** and **R⁵** is hydrogen, and **R⁴** is selected from alkyl e.g. methyl, group **R⁸**, and group **R¹¹**. In one embodiment, each of **p, q** and **r** is 1, each of **R⁴** and **R⁵** is hydrogen, and **R³** is selected from alkyl e.g. methyl and group **R⁷**. In one embodiment, each of **p, q** and **r** is 1, each of **R³** and **R⁴** is hydrogen, and **R⁵** is selected from group **R⁹** or group **R¹⁰**. It is meant to be understood that, when referring to **R³, R⁴,** and/or **R⁵** (e.g. when defining **R³, R⁴,** and/or **R⁵**), **p, q** and **r,** respectively, are to be understood as being 1. For example, when referring to a definition of **R³**, it is meant to be understood that, in such a scenario, **p** is 1.

The term "alkyl" refers to a monovalent straight, branched or cyclic chain, saturated aliphatic hydrocarbon radical having a number of carbon atoms in the specified range. Thus, for example, "C₁-C₆ alkyl" refers to any of the hexyl alkyl and pentyl alkyl isomers as well as n-, iso-, sec-, and t-butyl, n- and isopropyl, cyclic propyl, ethyl and methyl. In one embodiment, 1-alkylpiperazinyl is 1-methylpiperazinyl. In one embodiment, alkyl is C₁-C₆ alkyl, preferably methyl. The term "optionally substituted" as used herein is meant to indicate that a hydrogen atom where present and attached to a member atom within a group, or several such hydrogen atoms, may be replaced by a suitable group, such as alkyl, dimethylamine, pyrrolidine, piperidine, morpholine, or piperazine. In one embodiment, the term "optionally substituted" relates to optionally substituted with any of dimethylamine, pyrrolidine, piperidine, morpholine, and piperazine. In one embodiment, the term "heteroaromatic system" refers to (i) optionally substituted 5- and 6-membered heteroaromatic rings and (ii) optionally substituted 9- and 10-membered bicyclic, fused ring systems in which at least one ring is aromatic, wherein the heteroaromatic ring or the bicyclic, fused ring system contains from 1 to 4 heteroatoms independently selected from N, O, and S, where each N is optionally in the form of an oxide and each S in a ring which is not aromatic is optionally S(O) or S(O)₂. Suitable 5- and 6-membered heteroaromatic rings include, for example, isoxazolyl, pyridyl, pyrrolyl, pyrazinyl, pyrimidinyl, pyridazinyl, triazinyl, thienyl, furanyl, imidazolyl, pyrazolyl, triazolyl, tetrazolyl, oxazolyl, oxadiazolyl, thiazolyl, isothiazolyl, and thiadiazolyl. In one embodiment, a five- or six-membered heteroaromatic system is preferably isoxazolyl. In one embodiment, an amino acid residue, e.g. **R²** being an amino acid residue, is bound via the carbonyl group.

In one embodiment, the compound of the invention consists of any one structure as shown in Table 1. In one embodiment, the compound of the present invention is selected from the compounds depicted in Table 1. Each of the compounds may be used as defined herein, and/or may be at least one active ingredient in pharmaceutical compositions or formulations described herein, which can be used in the methods of treatment or are for use in the treatment of cancer as described herein. In one embodiment, the compound of the invention is a FLT3 inhibitor. The compounds of the present invention are efficient inhibitors of FLT3. Furthermore, the compounds of the invention have enhanced solubility and efficiency compared to compounds such as marbotinib. The compound of the invention may be combined, e.g. co-administered, with a further active agent, e.g. a drug other than the compound of the invention. For example, the compound of the invention may be combined with a histone deacetylase inhibitor (HDAC). The synergism of inhibition of tyrosine kinases and histone deacetylases has been shown, e.g. for FLT3. In one embodiment, a further active agent, e.g. comprised in a composition of the invention, is a histone deacetylase inhibitor.

The inventors have found that, advantageously, the compound of the invention, e.g. a compound having the general formula (I), (II), (III), or (IV), is highly potent. Particularly, a compound of the invention is more potent than the compound Marbotinib. For example, the inventors have found that the compound is particularly potent if two of **R³**, **R⁴**, and **R⁵** are hydrogen and one of **R³**, **R⁴**, and **R⁵** is selected from alkyl and any of the groups specified above, e.g. selected from alkyl and the following group:

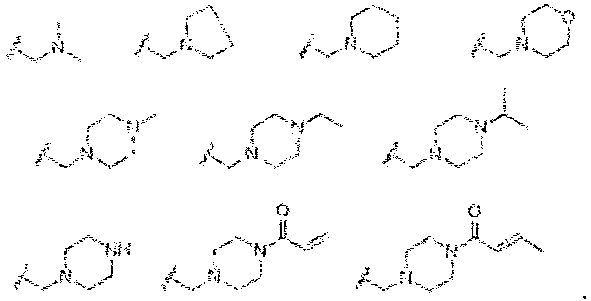

In one embodiment, the compound of the invention has a structure of any compound shown in Table 1. In one embodiment, the compound of the invention is any compound shown in Table 1. In one embodiment, the compound of the invention is selected from compound (cpd.) nos. 24a, 24b, 26, 29, 30a, 30b, 30c, 31a, 31b, 31c, 32a, 32b, 32c, 33a, 33b, 33c, 34a, 34b, 34c, 35a, 36a, 38a, 38b, 38c, 39a, 39b, 39c, 40a, 40b, 40c, 41a, 41b, 41c, 42a, 42b, 42c, 46a, 46b, 47a, 47b, 52a, 53b, 54a, 55b, 56a, 56b, 57a, 57b, 58a, 58b, 61a, 64a, 69a, 69b, 70a, 70b, and 76, as shown in Table 1. For example, the compound may be selected from compound nos. 33b, 33c, 34b, 34c, 41b, 41c, 42b, and 42c, as shown in Table 1. In a preferred embodiment, the compound of the invention has a structure of any one of compound nos. 33c, 34c, 41c, or 42c as shown in Table 1, preferably has a structure of compound no. 34c as shown in Table 1. In a preferred embodiment, the compound of the invention is any compound selected from compound nos. 33c, 34c, 41c, and 42c as shown in Table 1, preferably is compound no. 34c as shown in Table 1. The inventors have found that the compounds shown in Table 1 exhibit an advantageous solubility and potency, e.g. advantageous compared to marbotinib. Particularly, the inventors have found that compounds 33c, 34c, 41c, and 42c exhibit highly advantageous solubility and potency, e.g. advantageous compared to marbotinib. Furthermore, the inventors have found that the compounds of the invention, for example compound no. 34c as shown in Table 1, are highly effective in vivo. Advantageously, the compounds of the invention, for example the compounds shown in Table 1, have a good bioavailability. In one embodiment, the compound is provided in the form of a trifluoroacetate or hydrochloride.

In one embodiment, the composition, preferably pharmaceutical composition, of the invention comprises a compound of general formulae (I), (II), (III) and/or (IV). In one embodiment, the composition, preferably pharmaceutical composition, of the invention comprises a compound having a structure as shown in Table 1. In one embodiment, the composition, preferably pharmaceutical composition, comprises more than one alternative falling under the definitions of formulae (I), (II), (III) and/or (IV).

In one embodiment, the compound or composition is for use in a method of preventing or treating cancer, preferably blood cancer, more preferably leukemia, even more preferably acute myeloid leukemia (AML), and said method may comprise a co-administration of the compound of the invention with a further active agent, e.g. a further drug. In one embodiment, in said use in a method of preventing or treating cancer, the compound of the invention is co-administered with a further active agent, e.g. with a histone deacetylase inhibitor. In one embodiment, the compound or composition is administered to a patient in need thereof. In one embodiment, the term "patient" relates to a human or an animal, preferably a human. In one embodiment, the patient is characterized by a mutation in the *Flt₃* gene. In one embodiment, the compound or composition is for use in the treatment of a cancer patient having a mutation in the *Flt₃* gene.

In one embodiment, the compound or composition is for use in a method of preventing or treating cancer in a patient that is subjected to treatment with at least one further drug, particularly with at least one anti-cancer drug, more preferably with at least one HDAC inhibitor and/or transcription inhibitor. In one embodiment, said HDAC inhibitor is selected from the group consisting of Suberoylanilid Hydroxamic Acid (SAHA, Vorinostat), Panobinostat (LBH589)), and Benzamide (z.B. MS-275, MGCD-0103). In one embodiment, the transcription inhibitor is a cyclin dependent kinase (CDK) inhibitor such as a CDK1, CDK2, CDK3, CDK4, CDK5, CDK6, CDK7, CDK8, CDK9, CDK19, CDK11, and/or CDK12 inhibitor. In one embodiment, the CDK inhibitor is THZ1, E9, YLK-01-116, THZ-5-31-1, dinaciclib, DCA, or palbociclib. In one embodiment, the transcription inhibitor is a bromodomain-containing protein inhibitor (e.g., BRD2, BRD3, BDR4), a CREB binding protein inhibitor, or a E1A binding protein p300 (EP300) inhibitor. In one embodiment, the compound or composition of the invention is for use in the inhibition of FLT3.

In one embodiment, the method of preventing or treating comprises preventing or treating said patient by administering a therapeutically effective amount of any of the compounds, salts, solvates, and/or compositions of the invention. The present invention provides the use of compounds according to the invention as tyrosine kinase inhibitors, preferably Flt₃ inhibitor. Further, the present invention provides the use of a pharmaceutically acceptable salt or solvate of the compound according to the invention as tyrosine kinase inhibitor, preferably Flt₃ inhibitor. The administration of the compounds according to this invention and compositions such as pharmaceutical compositions according to the invention may be performed in any of the generally accepted modes of administration available in the art. Illustrative examples of suitable modes of administration include intravenous, oral, nasal, inhalable, parenteral, topical, transdermal, and rectal delivery. Oral and intravenous delivery forms are preferred. In one embodiment, an injectable composition comprising a therapeutically effective amount of the compound of the invention is provided, including salts, esters, isomers, solvates, hydrates and polymorphs thereof, at least one vehicle comprising water, aqueous solvents, organic solvents, hydro-alcoholic solvents, oily substances, or mixtures thereof, and optionally one or more pharmaceutically acceptable excipients.

The compositions of the invention such as pharmaceutical compositions may be formulated in the form of a dosage form for oral, intravenous, oral, nasal, inhalable, parenteral, topical, transdermal, or rectal administration. The formulations may comprise further pharmaceutically acceptable excipients, such as buffers, solvents, preservatives, disintegrants, stabilizers, carriers, diluents, fillers, binders, lubricants, glidants, colorants, pigments, taste masking agents, sweeteners, flavorants, plasticizers, and/or any acceptable auxiliary substances such as absorption enhancers, penetration enhancers, surfactants, cosurfactants, and specialized oils. The proper excipient(s) is (are) selected based in part on the dosage form, the intended mode of administration, the intended release rate, and manufacturing reliability. In one embodiment, excipient(s) are selected from polymers, waxes, calcium phosphates, sugars, and combinations thereof. Polymers include cellulose and cellulose derivatives such as HPMC, hydroxypropyl cellulose, hydroxyethyl cellulose, microcrystalline cellulose, carboxymethylcellulose, sodium carboxymethylcellulose, calcium carboxymethylcellulose, and ethylcellulose; polyvinylpyrrolidones; polyethylenoxides; polyalkylene glycols such as polyethylene glycol and polypropylene glycol; and polyacrylic acids including their copolymers and crosslinked polymers thereof, e.g., Eudragit^{®}(Rohm), polycarbophil, and chitosan polymers. Waxes include white beeswax, microcrystalline wax, carnauba wax, hydrogenated castor oil, glyceryl behenate, glycerylpalmito stearate, and saturated polyglycolyzed glycerate. Calcium phosphates include dibasic calcium phosphate, anhydrous dibasic calcium phosphate, and tribasic calcium phosphate. Sugars include simple sugars, such as lactose, maltose, mannitol, fructose, sorbitol, saccharose, xylitol, isomaltose, and glucose, as well as complex sugars (polysaccharides), such as maltodextrin, amylodextrin, starches, and modified starches.

The compositions of the present invention may be formulated into various types of dosage forms, for instance as solutions or suspensions, or as tablets, capsules, granules, pellets or sachets for oral administration. In one embodiment, the composition is in the form of a solid oral dosage form, preferably a tablet. The tablet is preferably a tablet for swallowing. It may optionally be coated with a film coat comprising any suitable inert coating material. The above lists of excipients and forms are not exhaustive. The pharmaceutical composition of the present invention can be manufactured according to standard methods known in the art. The composition of the invention may be provided in the form of granulates, e.g. obtained by dry compaction or wet granulation. These granulates can subsequently be mixed with e.g., suitable disintegrating agents, glidants and lubricants and the mixture can be compressed into tablets or filled into sachets or capsules of suitable size. Tablets can also be obtained by direct compression of a suitable powder mixture, i.e., without any preceding granulation of the excipients. Suitable powder or granulate mixtures are also obtainable by spray drying, lyophilization, melt extrusion, pellet layering, coating of the active pharmaceutical ingredient or any other suitable method. The so obtained powders or granulates can be mixed with one or more suitable ingredients and the resulting mixtures can either be compressed to form tablets or filled into sachets or capsules. The above-mentioned methods known in the art also include grinding and sieving techniques permitting the adjustment of desired particle size distributions. Injectable compositions of the present invention may contain a buffer (for example, sodium dihydrogen phosphate, disodium hydrogen phosphate and the like), an isotonizing agent (for example, glucose, sodium chloride and the like), a stabilizer (for example, sodium hydrogen sulfite and the like), a soothing agent (for example, glucose, benzyl alcohol, mepivacaine hydrochloride, xylocaine hydrochloride, procaine hydrochloride, carbocaine hydrochloride and the like), a preservative (for example, p-oxybenzoic acid ester such as methyl p-oxybenzoate and the like, thimerosal, chlorobutanol, benzyl alcohol and the like) and the like, if necessary. In addition, the injectable composition may contain vitamins and the like. Further, injectable compositions may contain an aqueous solvent, if necessary. Examples of the aqueous solvent include purified water for injection, physiological saline solution, and glucose solution. In injectable compositions of the present invention, the pharmaceutical compound may be solid. As used herein, the "solid" comprises crystals and amorphous substances which have conventional meanings. The form of the solid component is not particularly limited, but powder is preferred in view of dissolution rate.

As discussed above, the present invention provides the compound and/or composition for use in a method of preventing or treating cancer. Treatment includes any of amelioration, alleviation, prevention from worsening, and curing a cancer, particularly blood cancer, such as leukemia, especially such as acute myeloid leukemia (AML). In one embodiment, a method of preventing or treating cancer comprises a step of administering a therapeutically effective amount of a compound according to the invention or a composition of the invention to a patient in need thereof. A "therapeutically effective amount" or "effective amount" of a compound according to the invention preferably refers to the amount necessary to achieve the therapeutic outcome. The dosage of the compounds according to the invention is carried out in the order of magnitude customary for FLT3 inhibitors. For example, the customary dose in the case of systemic therapy (p.o.) may be between 0.03 and 60 mg/kg body weight per day, (i. v.) may be between 0.03 and 60 mg/kg/h. In one embodiment, the customary dose in the case of systemic therapy (p.o.) is between 0.3 and 30 mg/kg per day, (i. v.) is between 0.3 and 30 mg/kg/h. The choice of the optimal dosage regime and duration of medication, particularly the optimal dose and manner of administration of the active compounds necessary in each case can be determined by a person skilled in the art on the basis of his/her expert knowledge.

In one embodiment, the compound of the invention is obtainable or obtained by a method of preparing a compound of the invention. For example, the method of preparing a compound of the invention may comprise steps (1a), (1b), (1c), (1d), and (1e) as defined above. For example, the method of preparing a compound may comprise steps (2aa), (2ab), (2b), and (2c) as defined above. For example, the method of preparing a compound may comprise steps (3a), (3b), and (3c) as defined above. For example, the method of preparing a compound may comprise steps (4a), (4b), and (4c) as defined above. For example, the method of preparing a compound may comprise steps (5a), (5b), (5c), (5d), (5e), and (5f) as defined above. For example, step (5a) may comprise using a starting material such as 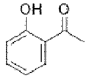 , salicylaldehyde (e.g. for a formation of unsubstituted benzofuran), or another aromatic ketone with ortho-substituted hydroxy-group. For example, the method of preparing a compound may comprise steps (6a) and (6b). In one embodiment, the method of preparing a compound of the invention comprises or consists of the steps shown in any of the schemes described herein. In one embodiment, the method of preparing a compound of the invention comprises or consists of reactions as shown in any of schemes 1-14. In one embodiment, the method of preparing a compound of the invention comprises scheme 1, 2, 3, 4, 5, 6, 7, 8, 9, 10, 11, 12, 13, and/or 14, as shown herein.

The terms "of the [present] invention", "in accordance with the invention", "according to the invention" and the like, as used herein are intended to refer to all aspects and embodiments of the invention described and/or claimed herein. As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, mo%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The present invention is now further described by reference to the following figures. All methods mentioned in the figure descriptions below were carried out as described in detail in the examples.
**Figure 1** shows A) UV spectra of solutions of 41c with different concentrations. Absorption of solutions of 8 concentations between 5 and 40 µM are measured in a wavelenght range between 200 and 500 nm. B) a plot of the concentration of the solutions against the measured absorbance values at the wavelength maximum. Graph was used as a calibration line for the saturated solutions of the corresponding compound 41c.
**Figure 2** shows Mean Relative (%) body weight of the oral treatment groups from MTD study + SEM.
**Figure 3** shows an overview over the progression of the relative tumor volume of all 5 treatment groups.
**Figure 4** shows an overview over the progression of the relative body weight of all 5 treatment groups.
**Figure 5** shows the change in relative tumor volume in the **34c** (20 mg/kg), **34c** (50 mg/kg), and midostaurin (50 mg/kg). The first curve (●) shows mice that received no drug. The second curve (▪) shows mice treated with midostaurin (50 mg/kg). The third curve (ó) shows **34c** group (20 mg/kg). The fourth curve (Δ) shows mice treated with **34c** (50 mg/kg).
**Figure 6** shows the change in relative tumor volume in the **34c** (20 mg/kg), **34c** (50 mg/kg), and quizartinib (AC220) (50 mg/kg). The first curve (●) shows mice that received no drug. The second curve (▪) shows mice treated with **34c** (20 mg/kg), and the third curve (ó) shows mice treated with **34c** (50 mg/kg). The fourth curve (Δ) shows mice treated with quizartinib (50 mg/kg).

In the following, reference is made to the examples, which are given to illustrate, not to limit the present invention.

### EXAMPLES

### Example 1: Exemplary compounds of the invention

The exemplary compounds shown in table 1 were prepared using a method of the invention, as described herein. In one embodiment, the compound of the invention has a structure of any one of the compounds shown in Table 1.

**Table 1: Synthesized compounds of the invention**

| Name | Chemical Name | No | Structure |
|---|---|---|---|
| Regetinib 1 | 1-(5-(*tert*-Butyl)isoxazol-3-yl)-3-(2-(5-hydroxy-3-(morpholinomethyl)-1*H*-indole-2-carbonyl)benzofuran-5-yl)urea | 24a | 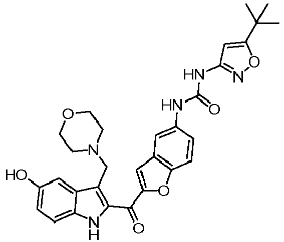 |
| Regetinib 1 trifluoroacetate | 1-(5-(*tert*-Butyl)isoxazol-3-yl)-3-(2-(5-hydroxy-3-(morpholinomethyl)-1*H*-indole-2-carbonyl)benzofuran-5-yl)urea 2,2,2-trifluoroacetate | 24b | 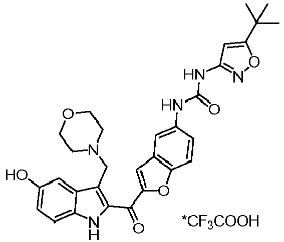 |
| Regetinib 2 | 1-(5-(*tert*-Butyl)isoxazol-3-yl)-3-(2-(5-hydroxy-3-methyl-1*H-*indole-2-carbonyl)benzofuran-5-yl)urea | 26 | 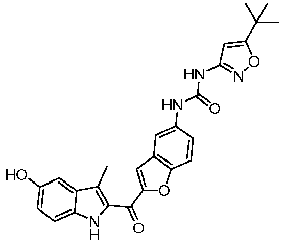 |
| Regetinib 3 | 1,1'-((3,3'-Methylenebis(5-hydroxy-1*H*-indole-3,2-diyl-2-carbonyl))bis(benzofuran-2,5-diyl))bis(3-(5-(tert-butyl)isoxazol-3-yl)urea) | 29 | 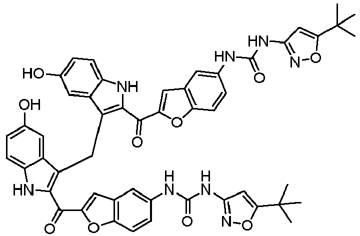 |
| Regetinib 4 | 1-(5-(*tert*-Butyl)isoxazol-3-yl)-3-(2-(5-hydroxy-4-(morpholinomethyl)-1*H*-indole-2-carbonyl)benzofuran-5-yl)urea | 30a | 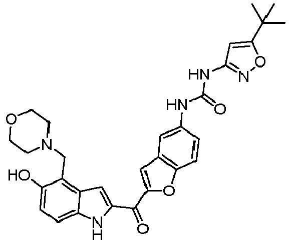 |
| Regetinib 4 trifluoroacetate | 1-(5-(*tert*-Butyl)isoxazol-3-yl)-3-(2-(5-hydroxy-4-(morpholinomethyl)-1*H*-indole-2-carbonyl)benzofuran-5-yl)urea 2,2,2-trifluoroacetate | 30b | 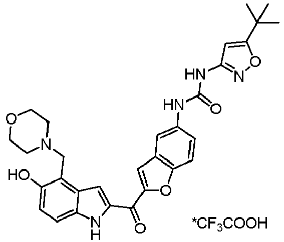 |
| Regetinib 5 | 1-(5-(*tert*-Butyl)isoxazol-3-yl)-3-(2-(5-hydroxy-4-(piperidin-1-ylmethyl)-1*H*-indole-2-carbonyl)benzofuran-5-yl)urea | 31a | 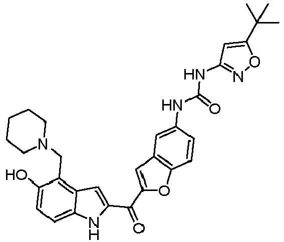 |
| Regetinib 5 trifluoroacetate | 1-(5-(*tert*-Butyl)isoxazol-3-yl)-3-(2-(5-hydroxy-4-(piperidin-1-ylmethyl)-1*H*-indole-2-carbonyl)benzofuran-5-yl)urea 2,2,2-trifluoroacetate | 31b | 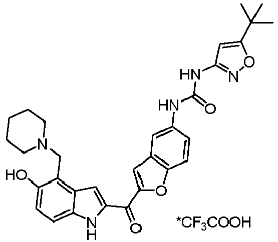 |
| Regetinib 6 | 1-(5-(*tert*-Butyl)isoxazol-3-yl)-3-(2-(5-hydroxy-4-(pyrrolidin-1-ylmethyl)-1*H*-indole-2-carbonyl)benzofuran-5-yl)urea | 32a | 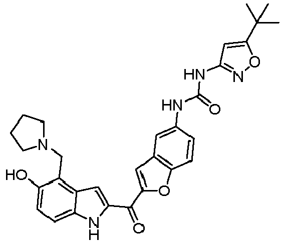 |
| Regetinib 6 trifluoroacetate | 1-(5-(*tert*-Butyl)isoxazol-3-yl)-3-(2-(5-hydroxy-4-(pyrrolidin-1-ylmethyl)-1*H*-indole-2-carbonyl)benzofuran-5-yl)urea 2,2,2-trifluoroacetate | 32b | 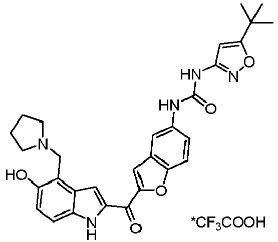 |
| Regetinib 7 | 1-(5-(*tert*-Butyl)isoxazol-3-yl)-3-(2-(5-hydroxy-4-((4-methylpiperazin-1-yl)methyl)-1*H-*indole-2-carbonyl)benzofuran-5-yl)urea | 33a | 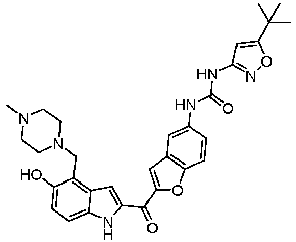 |
| Regetinib 7 trifluoroacetate | 1-(5-(*t*ert-Butyl)isoxazol-3-yl)-3-(2-(5-hydroxy-4-((4-methylpiperazin-1-yl)methyl)-1*H-*indole-2-carbonyl)benzofuran-5-yl)urea bis(2,2,2-trifluoroacetate) | 33b | 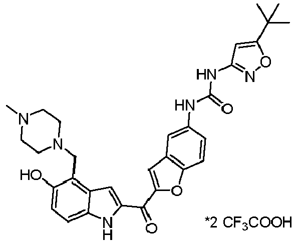 |
| Regetinib 8 | 1-(5-*tert*-Butylisoxazol-3-yl)-3-(2-(4-((dimethylamino)methyl)-5-hydroxy-1*H*-indole-2-carbonyl)benzofuran-5-yl)urea | 34a | 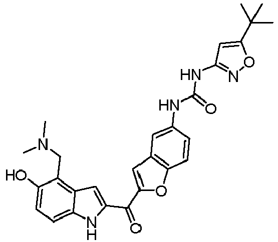 |
| Regetinib 8 trifluoroacetate | 1-(5-*tert*-Butylisoxazol-3-yl)-3-(2-(4-((dimethylamino)methyl)-5-hydroxy-1*H*-indole-2-carbonyl)benzofuran-5-yl)urea 2,2,2-trifluoroacetate | 34b | 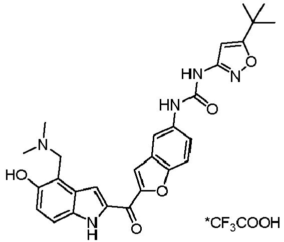 |
| Regetinib 9 | 1-(5-(*tert*-Butyl)isoxazol-3-yl)-3-(2-(5-hydroxy-4-((4-methyl-1,4-diazepan-1-yl)methyl)-1H-indole-2-carbonyl)benzofuran-5-yl)urea | 35a | 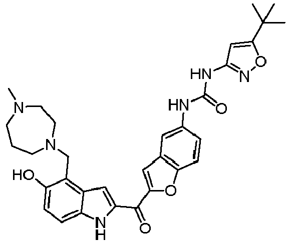 |
| Regetinib 10 | 1-(2-(4-((Benzyl(methyl)amino)methyl)-5-hydroxy-1H-indole-2-carbonyl)benzofuran-5-yl)-3-(5-(tert-butyl)isoxazol-3-yl)urea | 36a | 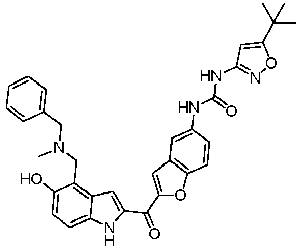 |
| Regetinib 11 | 2-(5-(3-(5-(*tert*-Butyl)isoxazol-3-yl)ureido)benzofuran-2-carbonyl)-4-(morpholinomethyl)-1*H*-indol-5-yl[1,4'-bipiperidine]-1'-carboxylate | 38a | 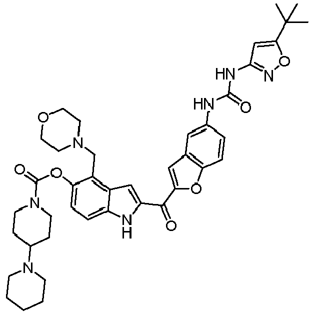 |
| Regetinib 11 trifluoroacetate | 2-(5-(3-(5-(*tert*-Butyl)isoxazol-3-yl)ureido)benzofuran-2-carbonyl)-4-(morpholinomethyl)-1*H*-indol-5-yl[1,4'-bipiperidine]-1'-carboxylate bis(2,2,2-trifluoroacetate) | 38b | 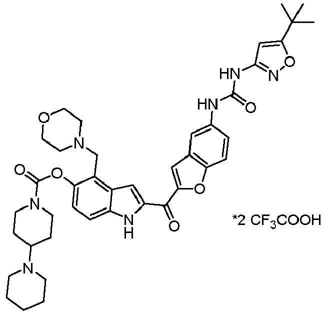 |
| Regetinib 12 | 2-(5-(3-(5-(*tert*-Butyl)isoxazol-3-yl)ureido)benzofuran-2-carbonyl)-4-(piperidin-1-ylmethyl)-1*H*-indol-5-yl [1,4'-bipiperidine]-1'-carboxylate | 39a | 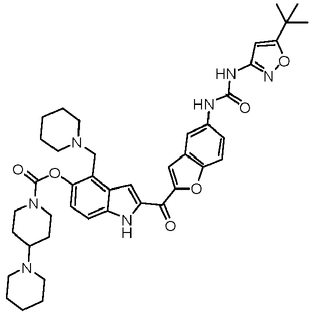 |
| Regetinib 12 trifluoroacetate | 2-(5-(3-(5-(*tert*-Butyl)isoxazol-3-yl)ureido)benzofuran-2-carbonyl)-4-(piperidin-1-ylmethyl)-1*H*-indol-5-yl [1,4'-bipiperidine]-1'-carboxylate bis(2,2,2-trifluoroacetate) | 39b | 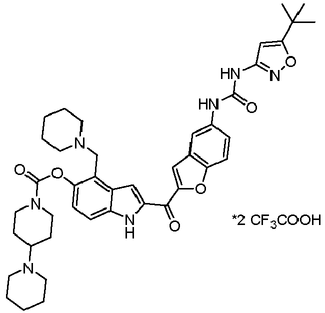 |
| Regetinib 13 | 2-(5-(3-(5-(*tert*-Butyl)isoxazol-3-yl)ureido)benzofuran-2-carbonyl)-4-(pyrrolidin-1-ylmethyl)-1*H*-indol-5-yl [1,4'-bipiperidine]-1'-carboxylate | 40a | 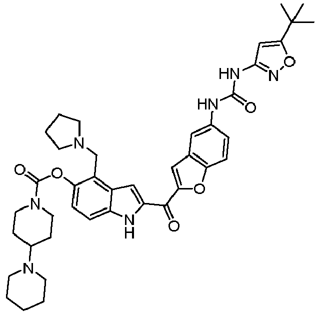 |
| Regetinib 13 trifluoroacetate | 2-(5-(3-(5-(*tert*-Butyl)isoxazol-3-yl)ureido)benzofuran-2-carbonyl)-4-(pyrrolidin-1-ylmethyl)-1*H*-indol-5-yl [1,4'-bipiperidine]-1'-carboxylate bis(2,2,2-trifluoroacetate) | 40b | 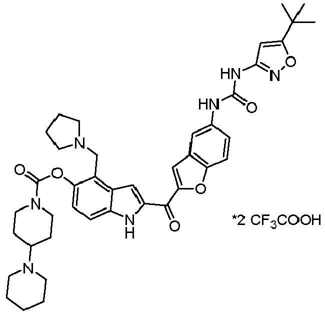 |
| Regetinib 14 | 2-(5-(3-(5-(*tert*-Butyl)isoxazol-3-yl)ureido)benzofuran-2-carbonyl)-4-((4-methylpiperazin-1-yl)methyl)-1*H*-indol-5-yl[1,4'-bipiperidine]-1'-carboxylate | 41a | 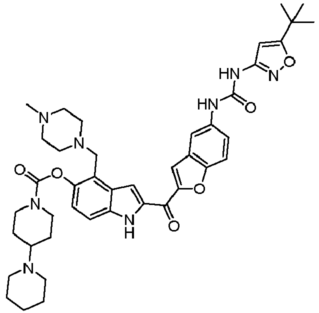 |
| Regetinib 14 trifluoroacetate | 2-(5-(3-(5-(*tert*-Butyl)isoxazol-3-yl)ureido)benzofuran-2-carbonyl)-4-((4-methylpiperazin-1-yl)methyl)-1*H*-indol-5-yl [1,4'-bipiperidine]-1'-carboxylate tris(2,2,2-trifluoroacetate) | 41b | 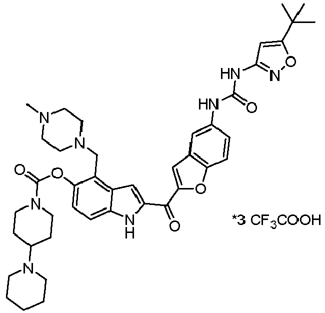 |
| Regetinib 15 | 2-(5-(3-(5-(*tert*-Butyl)isoxazol-3-yl)ureido)benzofuran-2-carbonyl)-4-((dimethylamino)methyl)-1*H-*indol-5-yl[1,4'-bipiperidine]-1'-carboxylate | 42a | 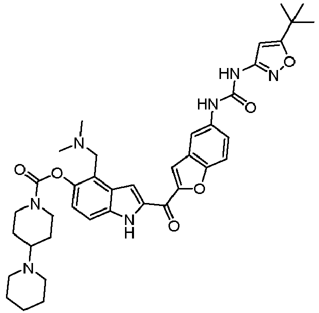 |
| Regetinib 15 trifluoroacetate | 5-(*tert*-Butyl)isoxazol-3-yl)ureido)benzofuran-2-carbonyl)-4-((dimethylamino)methyl)-1*H-*indol-5-yl[1,4'-bipiperidine]-1'-carboxylate bis(2,2,2-trifluoroacetate) | 42b | 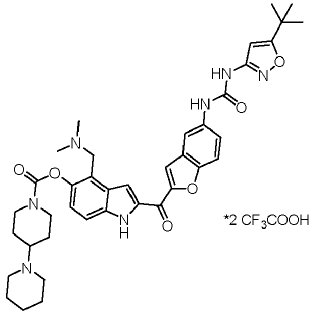 |
| Regetinib 4 hydrochloride | 1-(5-(*tert-*Butyl)isoxazol-3-yl)-3-(2-(5-hydroxy-4-(morpholinomethyl)-1*H*-indole-2-carbonyl)benzofuran-5-yl)urea hydrochloride | 30c | 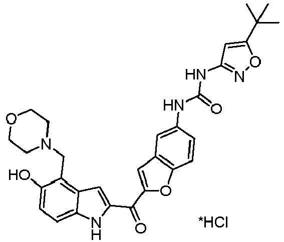 |
| Regetinib 5 hydrochloride | 1-(5-(*tert*-Butyl)isoxazol-3-yl)-3-(2-(5-hydroxy-4-(piperidin-1-ylmethyl)-1*H*-indole-2-carbonyl)benzofuran-5-yl)urea hydrochloride | 31c | 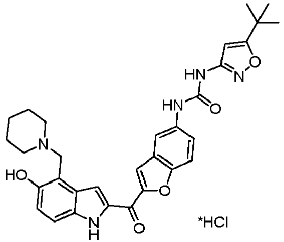 |
| Regetinib 6 hydrochloride | 1-(5-(*tert*-Butyl)isoxazol-3-yl)-3-(2-(5-hydroxy-4-(pyrrolidin-1-ylmethyl)-1*H*-indole-2-carbonyl)benzofuran-5-yl)urea hydrochloride | 32c | 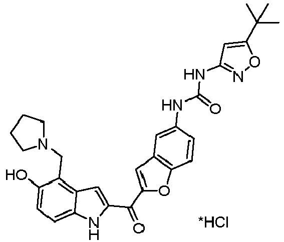 |
| Regetinib 7 hydrochloride | 1-(5-(*tert*-Butyl)isoxazol-3-yl)-3-(2-(5-hydroxy-4-((4-methylpiperazin-1-yl)methyl)-1*H-*indole-2-carbonyl)benzofuran-5-yl)urea hydrochloride | 33c | 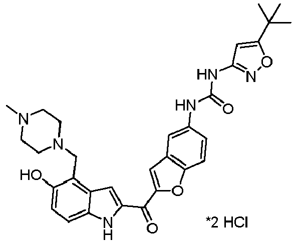 |
| Regetinib 8 hydrochloride | 1-(5-(*tert*-Butyl)isoxazol-3-yl)-3-(2-(4-(dimethylamino)methyl)-5-hydroxy-1*H*-indole-2-carbonyl)benzofuran-5-yl)urea hydrochloride | 34c | 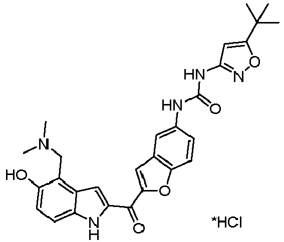 |
| Regetinib 11 hydrochloride | 2-(5-(3-(5-(*tert*-Butyl)isoxazol-3-yl)ureido)benzofuran-2-carbonyl)-4-(morpholinomethyl)-1*H*-indol-5-yl[1,4'-bipiperidine]-1'-carboxylate dihydrochloride | 38c | 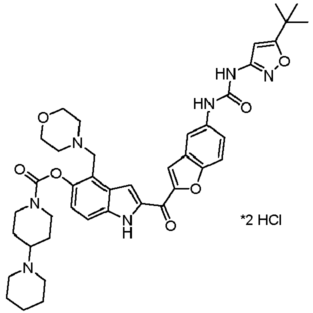 |
| Regetinib 12 hydrochloride | 2-(5-(3-(5-(*tert*-Butyl)isoxazol-3-yl)ureido)benzofuran-2-carbonyl)-4-(piperidin-1-ylmethyl)-1*H*-indol-5-yl [1,4'-bipiperidine]-1'-carboxylate dihydrochloride | 39c | 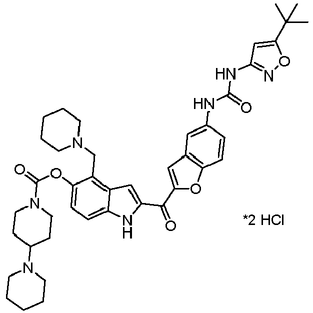 |
| Regetinib 13 hydrochloride | 2-(5-(3-(5-(*tert*-Butyl)isoxazol-3-yl)ureido)benzofuran-2-carbonyl)-4-(pyrrolidin-1-ylmethyl)-1*H*-indol-5-yl [1,4'-bipiperidine]-1'-carboxylate dihydrochloride | 40c | 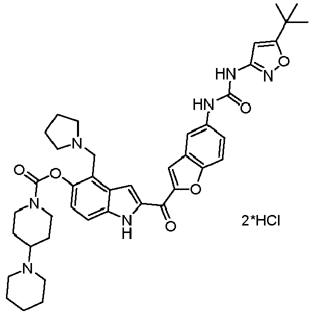 |
| Regetinib 14 hydrochloride | 2-(5-(3-(5-(*tert*-Butyl)isoxazol-3-yl)ureido)benzofuran-2-carbonyl)-43-((4-methylpiperazin-1-yl)methyl)-1*H-*indol-5-yl[1,4'-bipiperidine]-1'-carboxylate trihydrochloride | 41c | 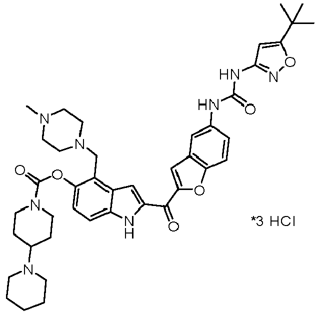 |
| Regetinib 15 hydrochloride | 2-(5-(3-(5-(*tert*-Butyl)isoxazol-3-yl)ureido)benzofuran-2-carbonyl)-4-((dimethylamino)methyl)-1*H-*indol-5-yl[1,4'-bipiperidine]-1'-carboxylate dihydrochloride | 42c | 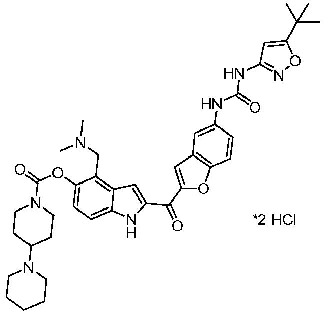 |
| Regetinib 16 | 1-(5-(*tert*-Butyl)isoxazol-3-yl)-3-(2-(5-hydroxy-4-((4-(4-methylpiperazin-1-yl)piperidin-1-yl)methyl)-1*H*-indole-2-carbonyl)benzofuran-5-yl)urea | 46a | 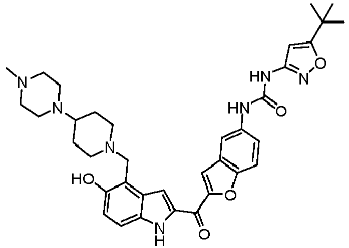 |
| Regetinib 16 trifluoroacetate | 1-(5-(*tert*-Butyl)isoxazol-3-yl)-3-(2-(5-hydroxy-4-((4-(4-methylpiperazin-1-yl)piperidin-1-yl)methyl)-1*H*-indole-2-carbonyl)benzofuran-5-yl)urea tris(2,2,2-trifluoroacetate) | 46b | 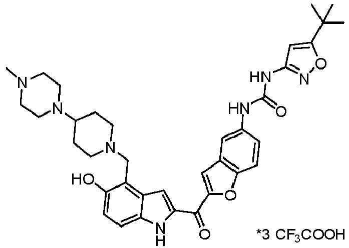 |
| Regetinib 17 | 2-(5-(3-(5-(*tert*-Butyl)isoxazol-3-yl)ureido)benzofuran-2-carbonyl)-4-((4-(4-methylpiperazin-1-yl)piperidin-1-yl)methyl)-1H-indol-5-yl[1,4'-bipiperidin]-1'-carboxylate | 47a | 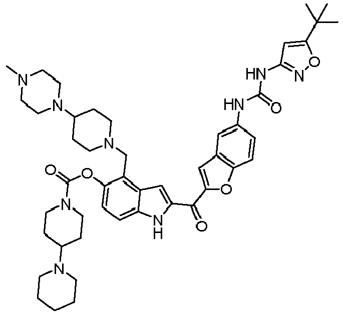 |
| Regetinib 17 trifluoroacetate | 2-(5-(3-(5-(*tert*-Butyl)isoxazol-3-yl)ureido)benzofuran-2-carbonyl)-4-((4-(4-methylpiperazin-1-yl)piperidin-1-yl)methyl)-1H-indol-5-yl[1,4'-bipiperidin]-1'-carboxylate tetrakis(2,2,2-trifluoroacetate) | 47b | 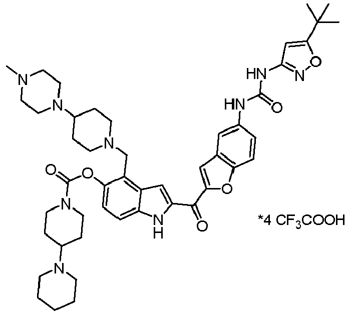 |
| Regetinib 18 | *tert*-Butyl (1-((2-(5-(3-(5-(*tert-*butyl)isoxazol-3-yl)ureido)benzofuran-2-carbonyl)-5-hydroxy-1*H*-indol-4-yl)methyl)piperidin-4-yl)carbamate | 52a | 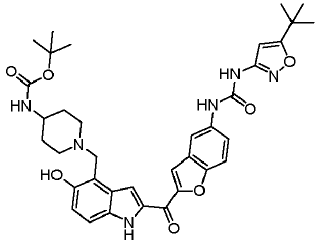 |
| Regetinib 19 | 1-(2-(4-((4-Aminopiperidin-1-yl)methyl)-5-hydroxy-1*H*-indole-2-carbonyl)benzofuran-5-yl)-3-(5-(tert-butyl)isoxazol-3-yl)urea bis(2,2,2-trifluoroacetate) | 53b | 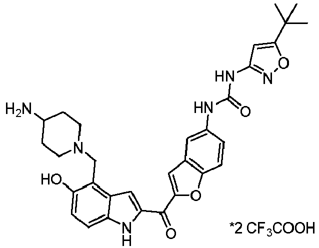 |
| Regetinib 20 | *tert*-Butyl 4-((2-(5-(3-(5-(*tert-*butyl)isoxazol-3-yl)ureido)benzofuran-2-carbonyl)-5-hydroxy-1*H*-indol-4-yl)methyl)piperazine-1-carboxylate | 54a | 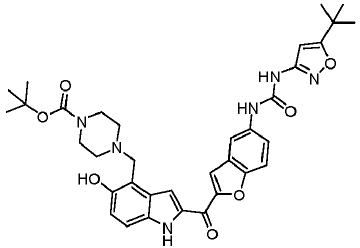 |
| Regetinib 21 | 1-(5-(*tert*-Butyl)isoxazol-3-yl)-3-(2-(5-hydroxy-4-(piperazin-1-ylmethyl)-1*H*-indole-2-carbonyl)benzofuran-5-yl)urea bis(2,2,2-trifluoroacetate) | 55b | 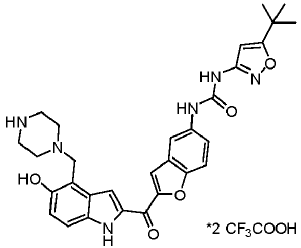 |
| Regetinib 22 | 1-(2-(4-((4-Acryloylpiperazin-1-yl)methyl)-5-hydroxy-1*H*-indole-2-carbonyl)benzofuran-5-yl)-3-(5-(tert-butyl)isoxazole-3-yl)urea | 56a | 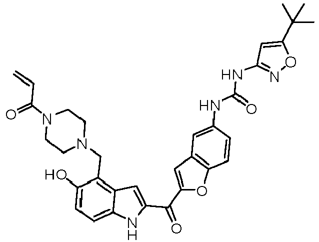 |
| Regetinib 22 trifluoroacetate | 1-(2-(4-((4-Acryloylpiperazin-1-yl)methyl)-5-hydroxy-1*H*-indole-2-carbonyl)benzofuran-5-yl)-3-(5-(tert-butyl)isoxazole-3-yl)urea bis(2,2,2-trifluoroacetate) | 56b | 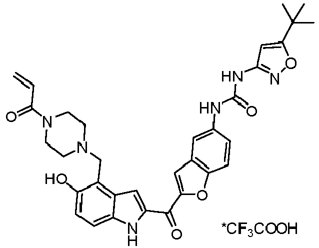 |
| Regetinib 23 trifluoroacetate | 1-(2-(4-((4-(But-2-enoyl)piperazin-1-yl)methyl)-5-hydroxy-1*H*-indole-2-carbonyl)benzofuran-5-yl)-3-(5-(*tert*-butyl)isoxazole-3-yl)urea bis(2,2,2-trifluoroacetate) | 57b | 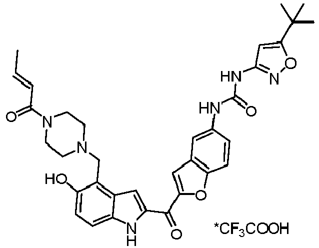 |
| Regetinib 24 | 1-(5-(*tert*-Butyl)isoxazol-3-yl)-3-(2-(4-((4-cinnamoylpiperazin-1-yl)methyl)-5-hydroxy-1*H*-indole-2-carbonyl)benzofuran-5-yl)urea | 58a | 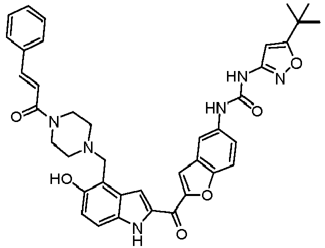 |
| Regetinib 24 trifluoroacetate | 1-(5-(*tert*-Butyl)isoxazol-3-yl)-3-(2-(4-((4-cinnamoylpiperazin-1-yl)methyl)-5-hydroxy-1*H*-indole-2-carbonyl)benzofuran-5-yl)urea bis(2,2,2-trifluoroacetate) | 58b | 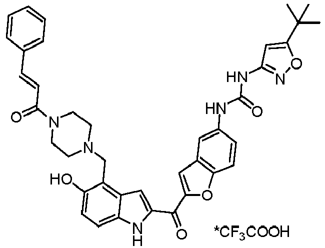 |
| Regetinib 25 | 1-(5-(*tert*-Butyl)isoxazol-3-yl)-3-(2-(5-hydroxy-4-((4-(vinylsulfonyl)piperazin-1-yl)methyl)-1*H*-indole-2-carbonyl)benzofuran-5-yl)urea | 61a | 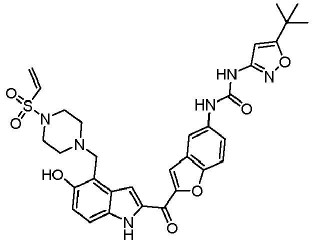 |
| Regetinib 26 | 1-(5-(*tert*-Butyl)isoxazol-3-yl)-3-(2-(5-hydroxy-4-((4-(pyrimidin-2-yl)piperazin-1-yl)methyl)-1*H-*indole-2-carbonyl)benzofuran-5-yl)urea | 64a | 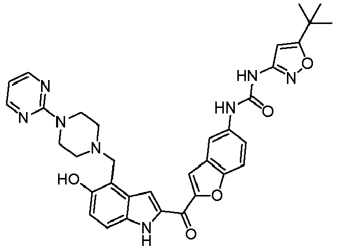 |
| Regetinib 27 | 1-(5-(*tert*-Butyl)isoxazol-3-yl)-3-(2-(4-((4-ethylpiperazin-1-yl)methyl)-5-hydroxy-1H-indole-2-carbonyl)benzofuran-5-yl)urea | 69a | 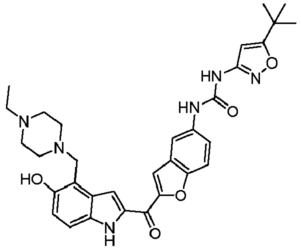 |
| Regetinib 27 trifluoroacetate | 1-(5-(*tert*-Butyl)isoxazol-3-yl)-3-(2-(4-((4-ethylpiperazin-1-yl)methyl)-5-hydroxy-1H-indole-2-carbonyl)benzofuran-5-yl)urea bis(2,2,2-trifluoroacetate | 69b | 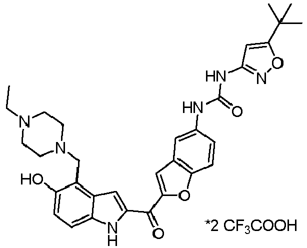 |
| Regetinib 28 | 1-(5-(*tert*-Butyl)isoxazol-3-yl)-3-(2-(5-hydroxy-4-((4-isopropylpiperazin-1-yl)methyl)-1H-indole-2-carbonyl)benzofuran-5-yl)urea | 70a | 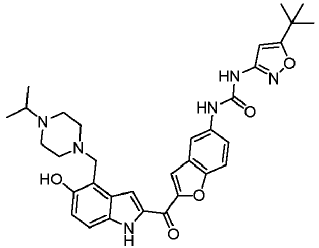 |
| Regetinib 28 trifluoroacetate | 1-(5-(*tert*-Butyl)isoxazol-3-yl)-3-(2-(5-hydroxy-4-((4-isopropylpiperazin-1-yl)methyl)-1H-indole-2-carbonyl)benzofuran-5-yl)urea bis(2,2,2-trifluoroacetate) | 70b | 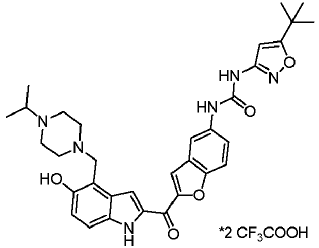 |
| Regetinib 29 | 1-(5-(*tert*-Butyl)isoxazol-3-yl)-3-(2-(5-hydroxy-1*H*-indole-2-carbonyl)-3-methylbenzofuran-5-yl)urea | 76 | 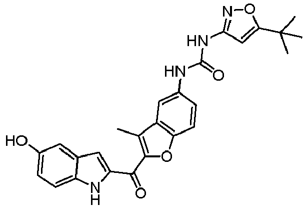 |

### Example 2: Synthesis of the compounds of the invention

Compounds of the invention were prepared as described below.

### Synthesis of Arylmethanone-Derivative 13 as an intermediate

To obtain the desired aryl-methanone derivative **13** also referred to as Marbotinib, a sequence described (Mahboobi; Sellmer et al. 2020) was used as follows:
5-Benzyloxyindole was protected by use of benzenesulfonyl chloride as described (Mahboobi et al. 2002; Sellmer et al. 2020) to get **14**. After acetylation **15** was obtained, which was brominated in alpha-position to the carbonyl-group using CuBr₂ or phenyltrimethylammonium tribromide to give **16**. Reaction of **16** with 2-hydroxy-5-nitrobenzaldehyde (**17**) resulted in the formation of (5-(benzyloxy)-1-(phenylsulfonyl)-1*H*-indol-2-yl)(5-nitrobenzofuran-2-yl)methanone as an intermediate. After alkaline cleavage of the phenylsulfonyl-protecting group **18** was obtained. By use of ammonium formate and Pd/C the nitro- and Bn- groups were reduced. Finally, reaction of **19** with 5-(*tert*-butyl)-3-isocyanatoisoxazole **20** in THF led to target compound **13** (Marbotinib) (Scheme 1).

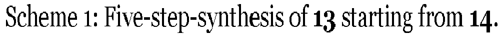

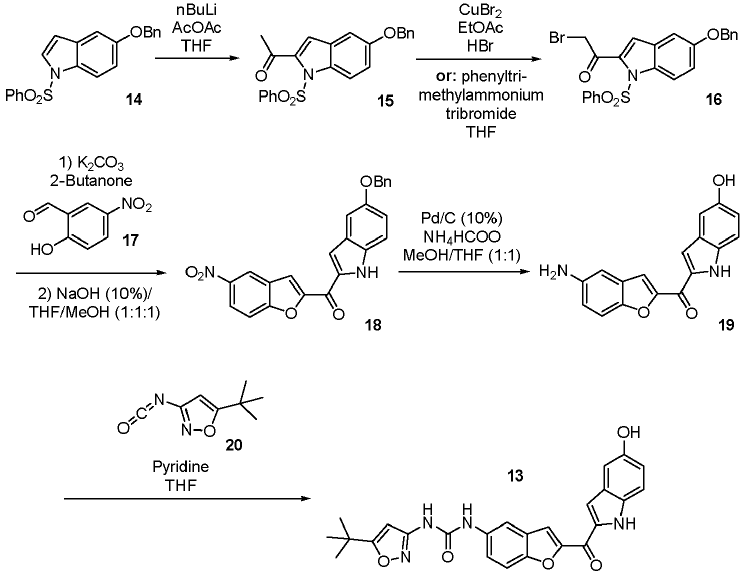

### Synthesis of derivatives with aminomethyl group in position 3 of the indole subunit

In order to obtain an aminomethyl group in position 3 of the indole subunit of Marbotinib, Mannich reaction with paraformaldehyde and morpholine was performed to synthesize **21**, using **18** as starting material as shown in Scheme 2. Deprotection of the benzyl group with BBr₃ and subsequent reduction of the nitro group with Raney-Ni and hydrazine resulted in **23**. A solution of 5-(*tert*-butyl)-3-isocyanatoisoxazole **20** in THF was used the same way as described for Marbotinib to obtain compound **24a** (Sellmer et al. 2020).

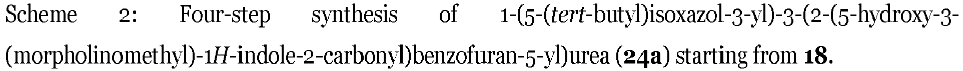

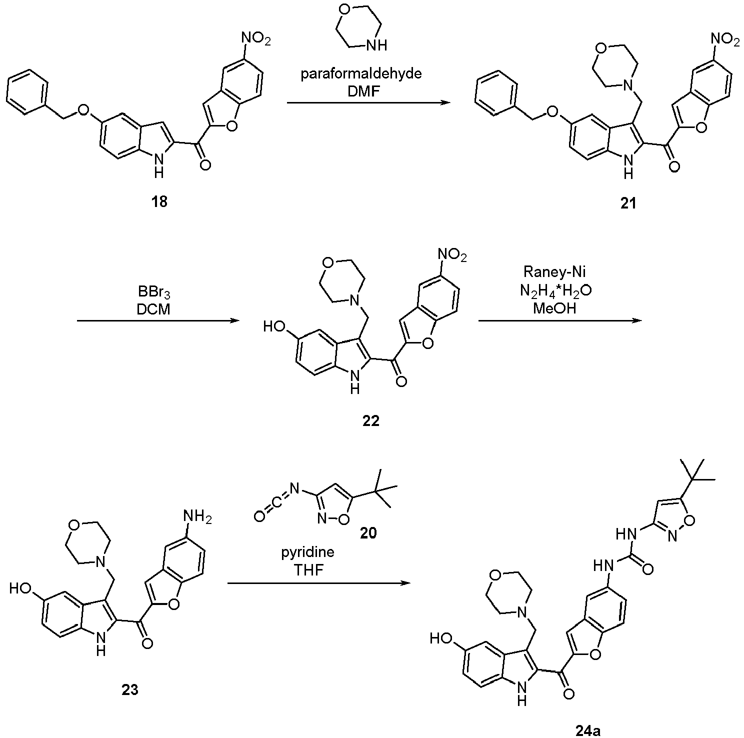

### Synthesis of derivatives with methyl group in position 3 of the indole subunit

Compound **26** with a methyl group in position 3 of the indole subunit was synthesized by hydrogenation of **21** and subsequent reaction with 5-(*tert*-butyl)-3-isocyanatoisoxazole **20** as described for Marbotinib (Scheme 3) (Sellmer et al. 2020).

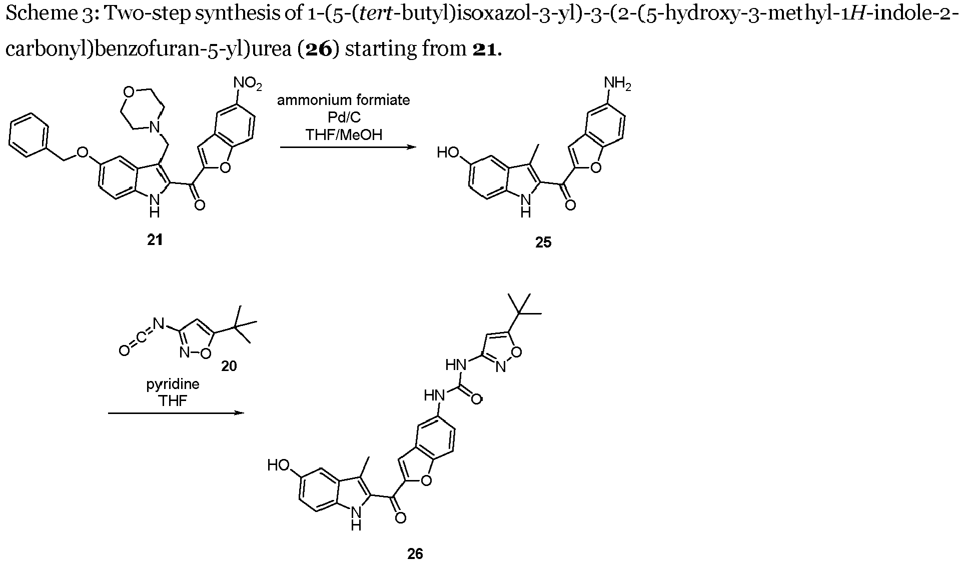

### Synthesis of a dimer compound connected via position 3 of the indole subunit

By reacting **18** with dimethylamine and paraformaldehyde dimerization could be observed where connection of both monomers is done via position 3 of each indole subunit with a methyl group as shown in Scheme 4. The dimer product **27** was hydrogenolysed to obtain **28** and reacted with **20** to get the desired compound **29** (Sellmer et al. 2020).

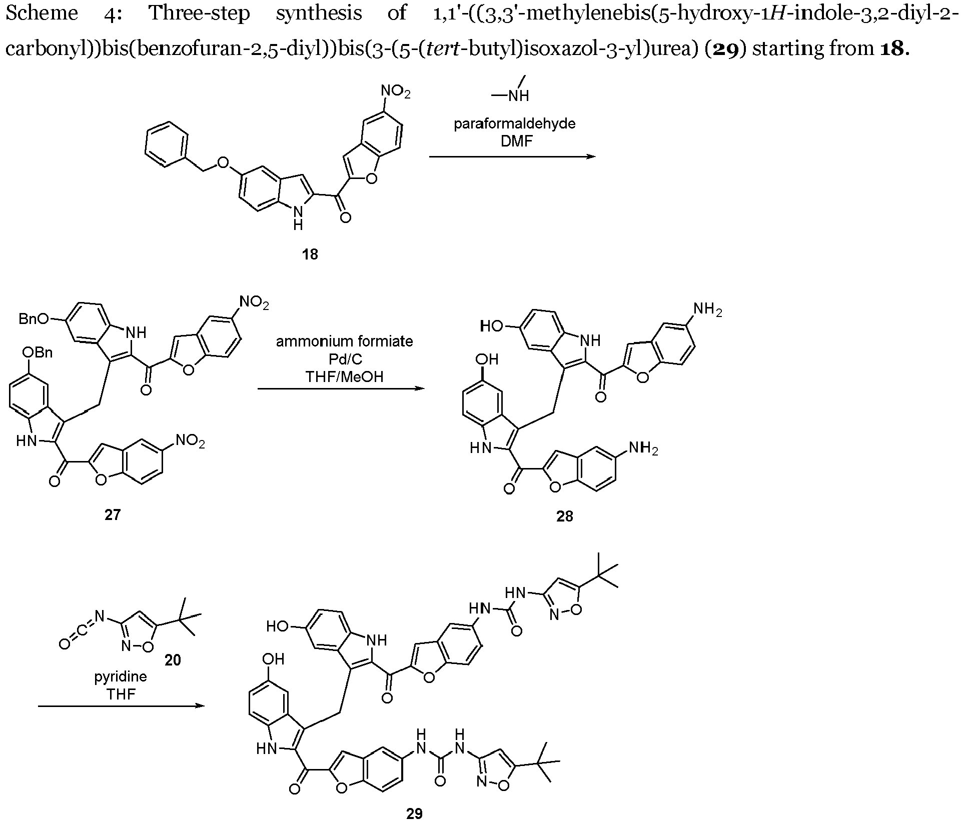

### Synthesis of derivatives with aminomethyl group in position 4 of the indole subunit

In order to obtain an aminomethyl group in position 4 of the indole subunit of Marbotinib (**13**), Mannich reaction was done with formaldehyde (37% in water or paraformaldehyde) and the corresponding amine in different solvents (Bandini and Eichholzer 2009; Isgör Y G et. al. 2008; Harriman; Monti and Johnson 1970) starting from Marbotinib (**13**). Morpholine, piperidine, pyrrolidine, N-methyl-piperazine, dimethylamine, 1-methyl-1,4-diazepane or *N*-methyl-1-phenylmethanamine were used to synthesize **30a** - **36a** (Scheme 5).

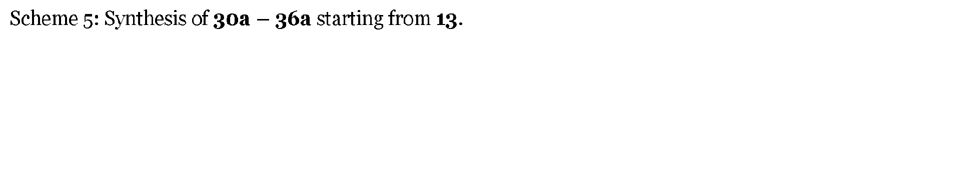

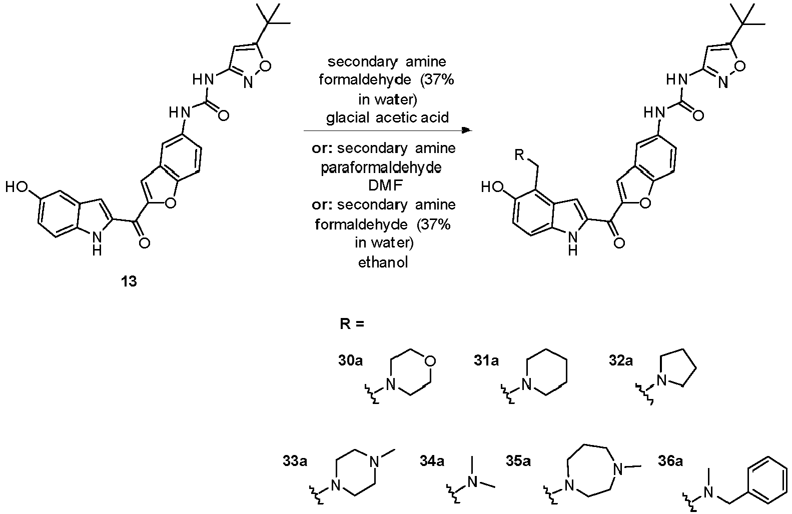

### Synthesis of carbamates with aminomethyl group in position 4 of the indole subunit

Carbamates of some derivatives (**38a**, **39a, 40a, 41a** and **42**a) were synthesized by reaction of the alcohols **30a**, **31a, 32a, 33a** and **34a** with [1,4'-bipiperidine]-1'-carbonyl chloride **37** using pyridine as a base (Scheme 6) as already done for Marbotinib (**13**) (Sellmer et al. 2020).

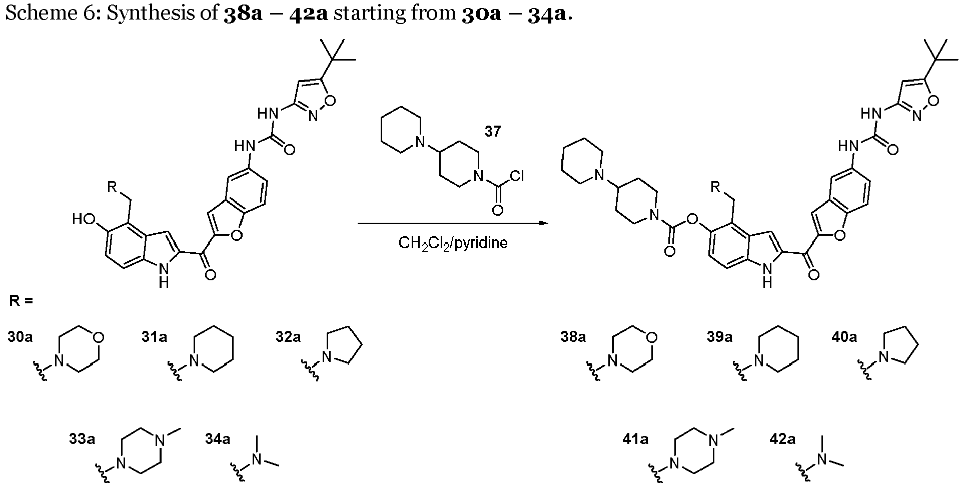

### Synthesis of the hydrochlorides

In order to increase water solubility and consequently biological availability of the synthesized compounds, some derivatives (**30a**, **31a, 32a, 33a** and **34a**) and their carbamates (**38a**, **39a, 40a**, **41a** and **42a**) were treated with HCl (solution in water or diethylether) to form the corresponding hydrochlorides (Scheme 7) (Sellmer et al. 2020).

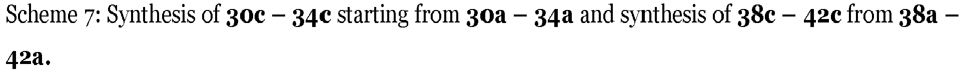

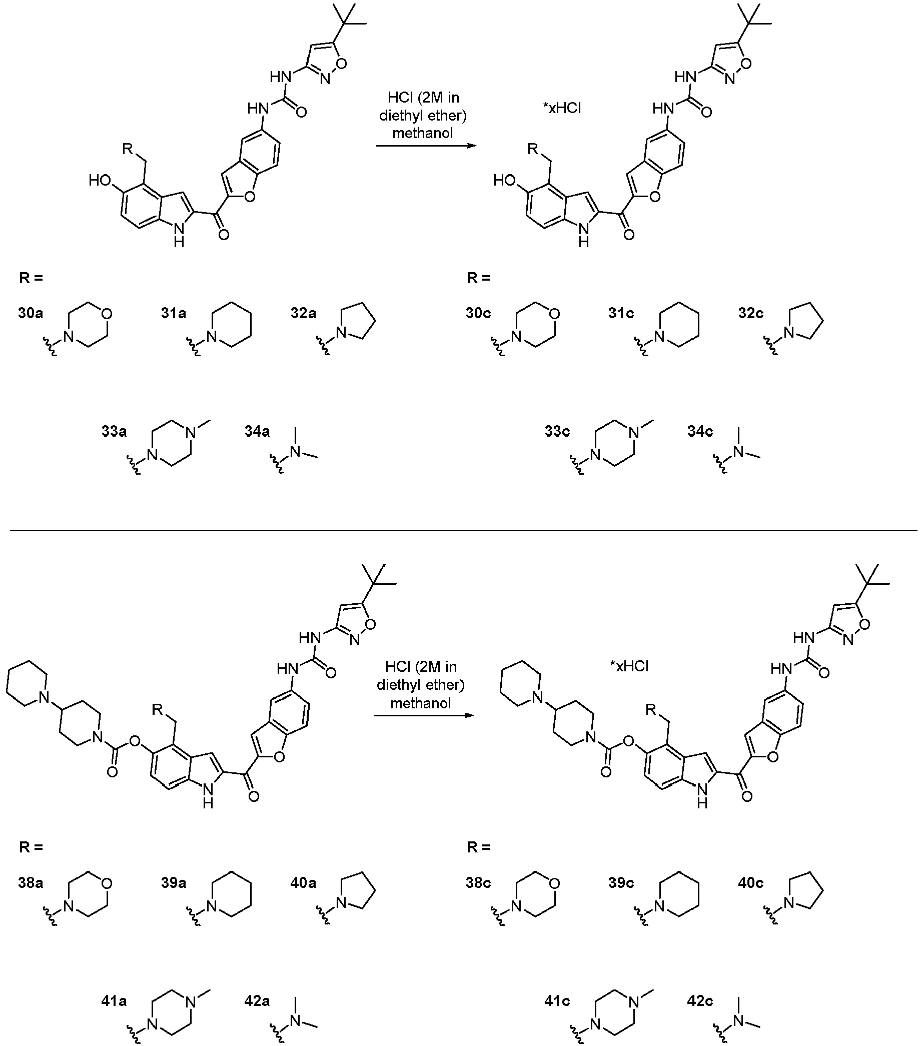

### Synthesis of 1-(5-(tert-Butyl)isoxazol-3-yl)-3-(2-(5-hydroxy-4-((4-(4-methylpiperazin-1-yl)piperidin-1-yl)methyl)-1H-indole-2-carbonyl)benzofuran-5-yl)urea and the corresponding carbamate

**45** was synthesized over two steps from 1-benzylpiperidin-4-one **43** and *N*-methylpiperazine as shown in Scheme 8. Resulting 1-Methyl-4-(piperidin-4-yl)piperazine **45** was further used for already mentioned Mannich reaction to obtain **46a** as shown above. The corresponding carbamate **47a** was obtained the same way as shown for Marbotinib **(13)** (Sellmer et al. 2020).

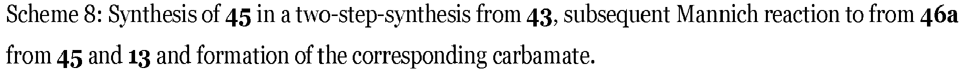

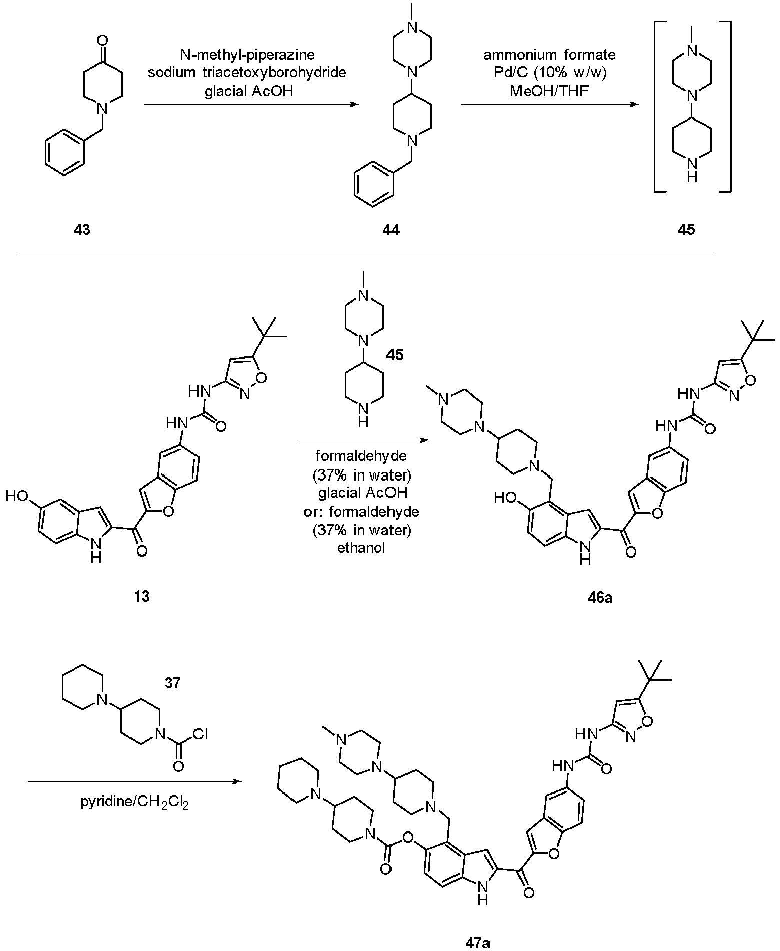

### Synthesis of 1-(2-(4-((4-aminopiperidin-1-yl)methyl)-5-hydroxy-1H-indole-2-carbonyl)benzofuran-5-yl)-3-(5-(tert-butyl)isoxazol-3-yl)urea bis(2,2,2-trifluoroacetic acid)

tert-Butyl piperidin-4-ylcarbamate **51** was synthesized in a four-step-synthesis starting from **43.** Ketoxime **48** was obtained by reaction of ketone **43** with hydroxylamine. Reduction with lithium aluminum hydride led to amine **49,** which was protected with a *boc*-group to form **50.** In a last step the benzyl group was removed by catalytic hydrogenation and *tert*-butyl piperidin-4-ylcarbamate **51** could be obtained, which was used for the Mannich reaction to form **52a** as described above. The desired target compound **53b** was reached by subsequent acidic cleavage of the *boc*-group (Scheme 9).

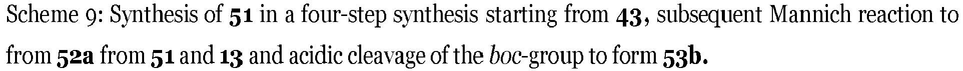

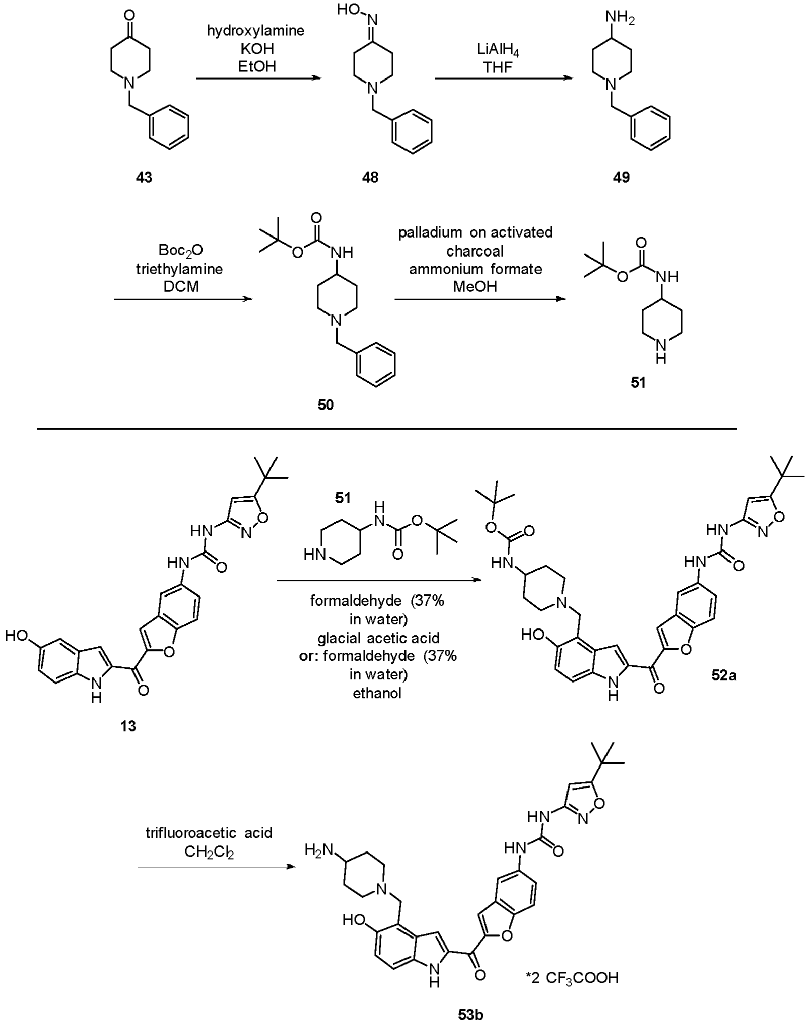

### Synthesis of enone compounds

**54a** could be obtained by using *tert*-butyl piperazine-1-carboxylate for the Mannich reaction as described above. Subsequent acidic cleavage of the *boc*-group (Scheme 10) led to **55b** as an intermediate and reaction of **55b** with different acid chlorides using DIPEA as base resulted in enone compounds **56a - 58a.**

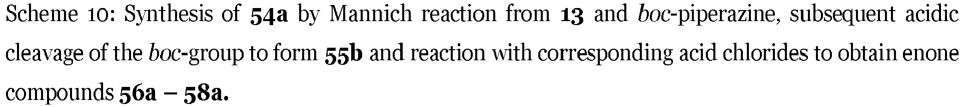

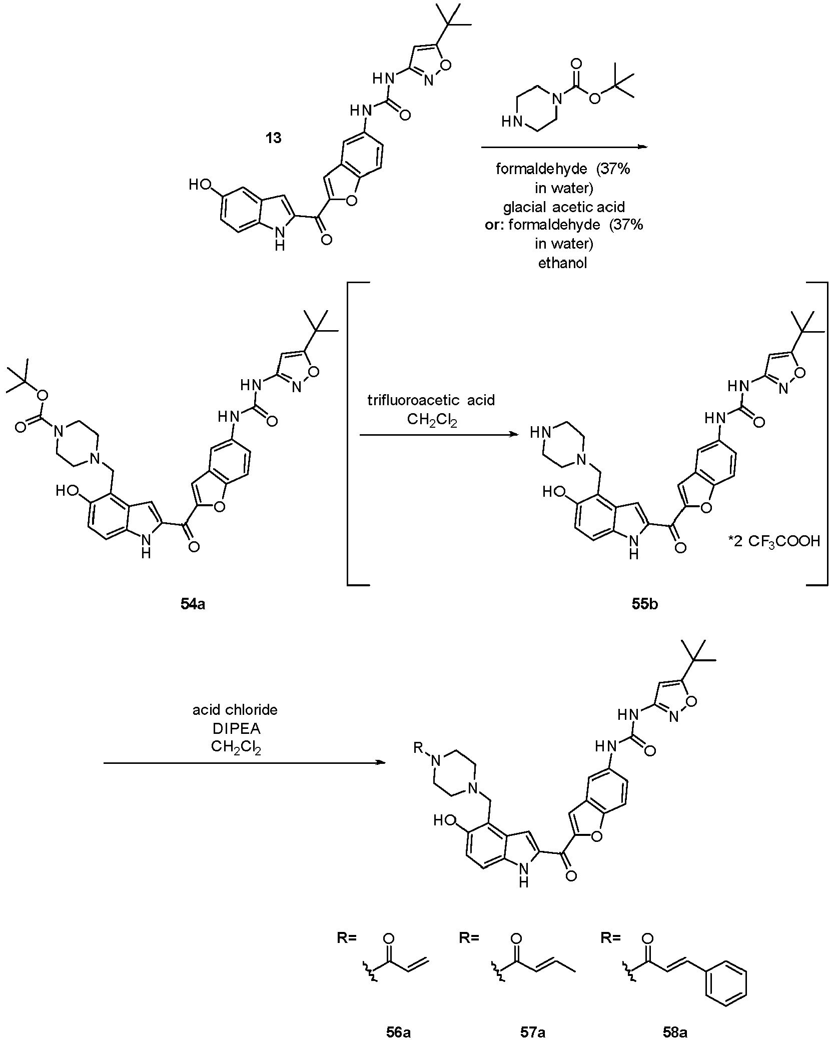

### Synthesis of 1-(5-(tert-butyl)isoxazol-3-yl)-3-(2-(5-hydroxy-4-((4-(vinylsulfonyl)piperazin-1-yl)methyl)-1H-indole-2-carbonyl)benzofuran-5-yl)urea

Enone-analog compound **61a** was obtained by Mannich reaction of 1-(vinylsulfonyl)piperazine **60** with Marbotinib **(13)** as described above. Two-step-synthesis starting from *tert*-butyl piperazine-1-carboxylate and 2-chloroethane-1-sulfonyl chloride using triethylamine as a base led to **59,** which was deprotected subsequently to form **60** as shown in Scheme 11.

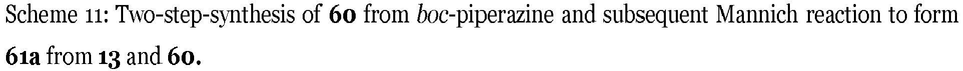

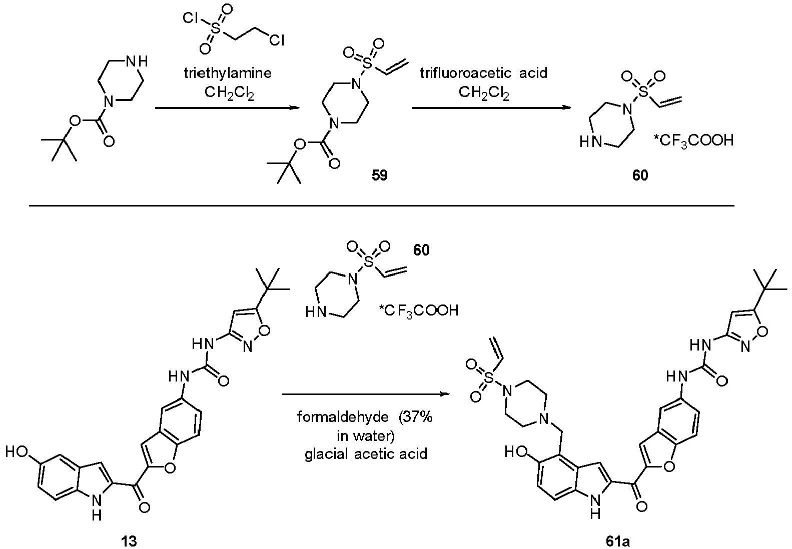

### Synthesis of 1-(5-(tert-Butyl)isoxazol-3-yl)-3-(2-(5-hydroxy-4-((4-(pyrimidin-2-yl)piperazin-1-yl)methyl)-1H-indole-2-carbonyl)benzofuran-5-yl)urea

Combination of piperazine with aromatic pyrimidine led to compound **63,** which was reacted with Marbotinib **(13)** as described above to get **64a** (Scheme 12).

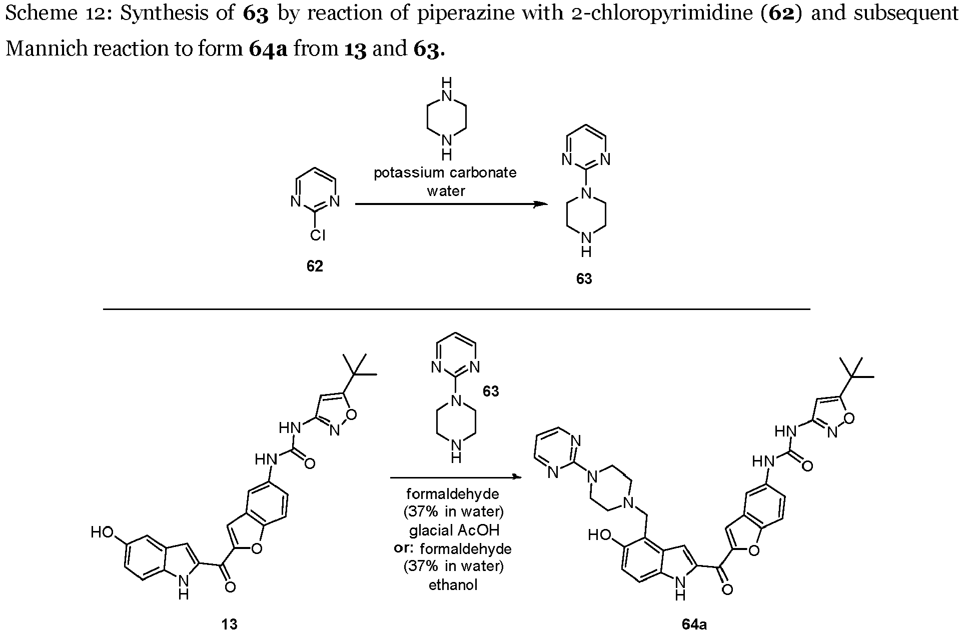

### Synthesis of 1-(5-(tert-Butyl)isoxazol-3-yl)-3-(2-(4-((4-ethylpiperazin-1-yl)methyl)-5-hydroxy-1H-indole-2-carbonyl)benzofuran-5-yl)urea and 1-(5-(tert-Butyl)isoxazol-3-yl)-3-(2-(5-hydroxy-4-((4-isopropylpiperazin-1-yl)methyl)-1H-indole-2-carbonyl)benzofuran-5-yl)urea

In order to reach two more piperazine derivatives **69a** and **70a,** the amines **67** and **68** were synthesized by *N*-alkylation of *tert*-butyl piperazine-1-carboxylate similar to lit. and subsequent acidic cleavage of the protection group as shown in Scheme 13. Reaction of these amines with Marbotinib as described above led to **69a** and **70a.**

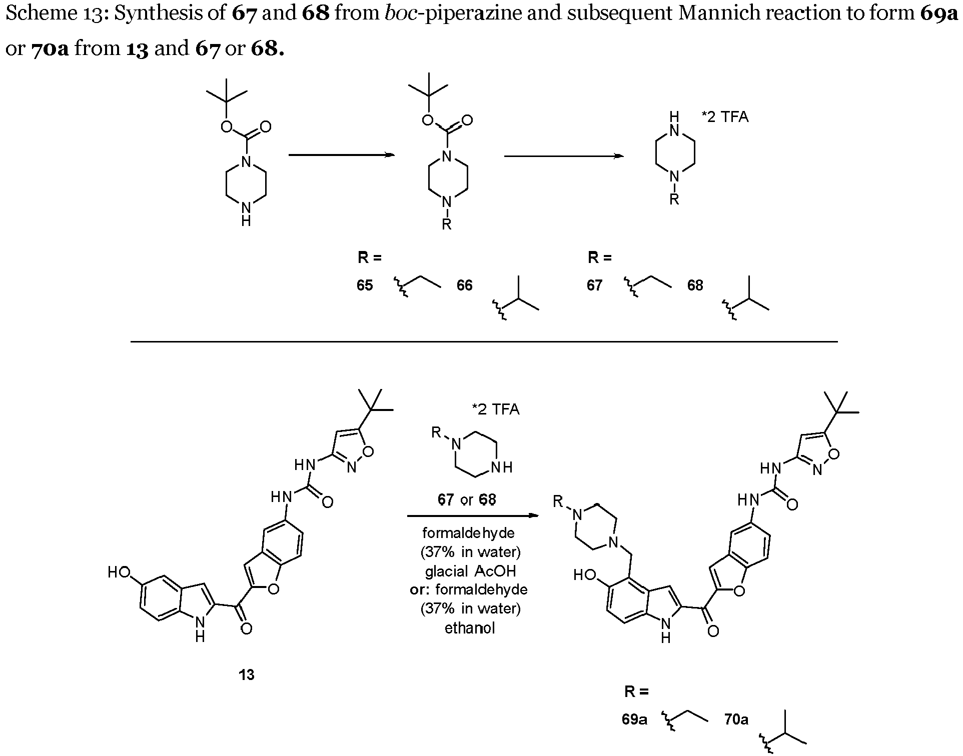

### Synthesis of derivatives with methyl group in position 3 of the benzofuran subunit

Compound **76** with a methyl group in position 3 of the benzofuran subunit was synthesized as shown in Scheme 14 starting with nitration of **71.** Reaction of α-brominated **16** with **72** resulted in the formation of **73** as an intermediate. The phenylsulfonyl-protecting group was removed to obtain **74** and simultaneous reduction of nitro-group and benzyl-group led to **75.** In a last step, reaction of **75** with 5-(*tert*-butyl)-3-isocyanatoisoxazole resulted in the formation of compound **76.** All steps were performed in a similar way as described for Marbotinib **(13)** (Sellmer et al. 2020).

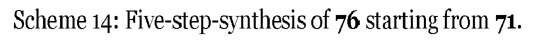

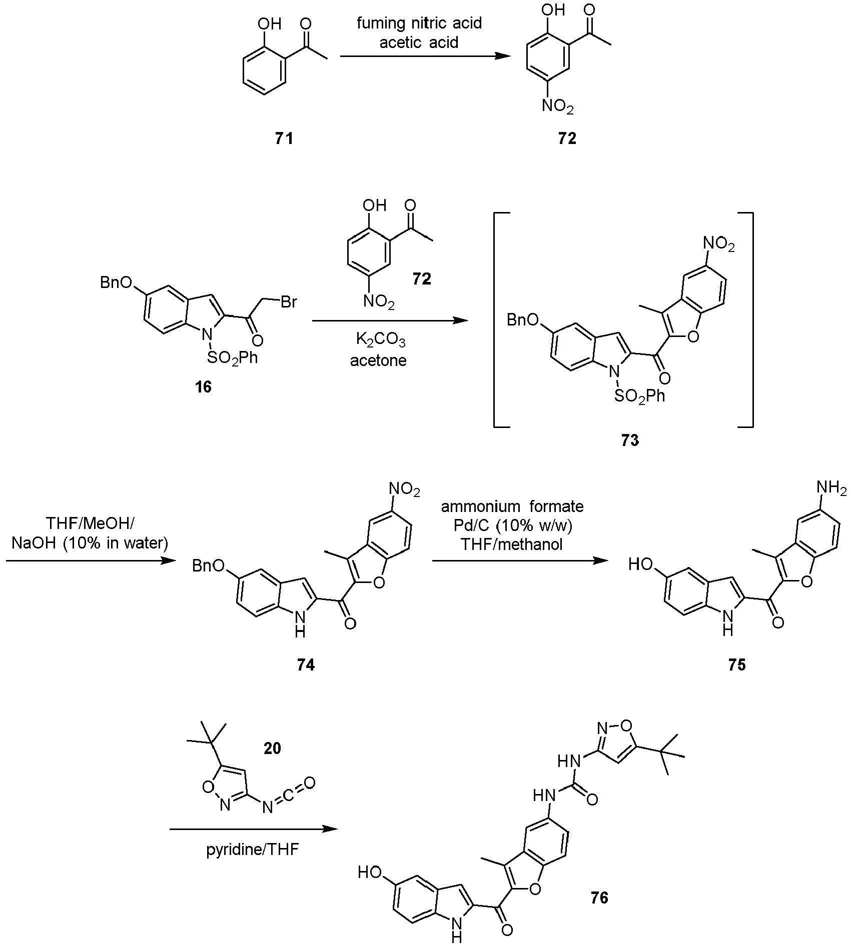

### Example 3: Experimental Section

### Materials and Methods

### General information

In general, NMR spectra were recorded with a Bruker Avance 300 MHz spectrometer at 300 K, using TMS as an internal standard. Melting points were determined with a Büchi B-545. MS spectra were measured with a Finnigan MAT SSQ 710A, a Finnigan MAT 95 or respectively a ThermoQuest Finnigan TSQ 7000. All reactions were carried out under nitrogen atmosphere if necessary. Some compounds were additionally purified by preparative HPLC system from waters (Eschbom, Germany) consisting of a Binary Gradient Module (waters 2545), a detector (waters 2489 UV/visible detector), a manual injector (waters Prep inject) and a collector (waters Fraction Collector III). A Kinetex XB C18, 5 µm, 250 × 21 mm (Phenomenex) was used as a RP column. Before injection all solutions were filtrated with syringe filters (0.45 µm, nylon). The flow rate was 18 or 20 mL/min and the detection wavelength 220 nm. The mobile phase contained the solvents acetonitrile and 0.1% aq. TFA. The eluate was lyophilized using a freeze-dryer (Christ ALPHA 2-4 LD-2).

### Cell lines

MV4-11 cells are human acute myeloid leukemia cell lines that carry a FLT3-ITD mutation (American Type Culture Collection, ATCC, Manassas, VA, United States). Cells were cultured in IMDM medium plus 100 µg/mL penicillin, 100 pg/mL streptomycin and 10% FBS. All cells were kept at 37 °C, 5 % CO₂.

### General procedure 1:

Reaction was performed according to lit. (Isgör Y G et. al. 2008). A solution of the starting material in dimethylformamide was heated to 80 °C. At this temperature paraformaldehyde (3.0 eq.) and the corresponding amine (3.0 eq.) were added. The completion of the reaction was controlled by TLC (DCM:MeOH 95:5). The reaction was stopped by addition of water. After extraction with EtOAc (3x) the combined organic layers were dried (Na₂SO₄) and the solvent was removed under reduced pressure. The crude product was purified by column chromatography (DCM/MeOH 10:1) to obtain the product.

### General procedure 2:

Reaction was performed similar as described for Marbotinib **(13)** (Sellmer et al. 2020). A solution of phosgen (20% in toluene, 8.80 eq.) was added dropwise to a solution of 5-(*tert*-butyl)isoxazol-3-amine (2.20 eq.) at o °C. Pyridine (16 eq.) was added and the reaction mixture was stirred overnight at room temperature. The solvent was evaporated, the residue was resolved in THF (10 mL/g) and filtered. The solution of 5-(*tert*-Butyl)-3-isocyanatoisoxazole (2.20 eq.) in THF was added to a solution of the corresponding amine (1.00 eq.) in THF (100 mL/g). Pyridine (25 eq.) was added, and the reaction mixture was stirred at room temperature overnight. After completion of the reaction ethyl acetate was added, the organic layer was washed with water twice and dried (Na₂SO₄). The solvent was removed under reduced pressure.

### General procedure 3:

Reaction was performed similar as described for Marbotinib **(13)** (Sellmer et al. 2020). The starting material was dissolved in a mixture of THF and methanol (1:1) and heated to reflux. Ammonium formate (4.00 eq.) and palladium on activated charcoal (10% w/w) were added and the reaction mixture was refluxed until complete consumption of the starting material. The catalyst was removed by filtering the reaction mixture over a pad of celite.

### General procedure 4A:

Reaction was performed according to lit. (Bandini and Eichholzer 2009). The corresponding amine and formaldehyde (37% aq.) were stirred in glacial acetic acid for 10 minutes. The starting material in glacial acetic acid was added and the resulting solution was stirred at room temperature. The reaction was stopped by addition of NaOH (30% aq.) or NaHCO₃ (sat. in water). After extraction with DCM or ethyl acetate (3x) the combined organic layers were dried (Na₂SO₄) and the solvent was removed under reduced pressure. The crude product was purified by column chromatography (DCM/MeOH/7 N NH₃ in MeOH 95:4:1) and crystallization from petroleum ether and DCM.

### General procedure 4B:

Reaction was performed similar to lit. (Monti and Johnson 1970). A suspension of the starting material in EtOH (17 mL/g) was heated to 60 °C. The corresponding amine (1.10 equiv.) and formaldehyde (37% aq., 1.20 equiv.) were added, and the reaction mixture was stirred at this temperature for 4 h. After addition of another 1.1 equiv. of the amine and 1.2 equiv. formaldehyde stirring was continued overnight. The mixture was cooled, diluted with EtOAc, and washed with NaHCO₃ (sat. in water) and water. The solvent was removed, and the crude product was purified by column chromatography (DCM/MeOH 10:1) to obtain the product.

### General procedure 5:

Reaction was performed similar as described for Marbotinib **(13)** (Sellmer et al. 2020). To a solution of the corresponding starting material in a mixture of DCM:pyridine (1:1, 10 mL) was added [1,4'-bipiperidine]-1'-carbonylchloride (1.3 eq.) in DCM:pyridine (1:1, 5 mL). After completion of reaction the reaction mixture was diluted with 10 ml DCM. The organic layer was washed with water (2×10 ml), dried (MgSO₄) and the solvent evaporated under reduced pressure. The crude product was purified by column chromatography (DCM/MeOH/7 N NH₃ in MeOH 90:9:1).

### General procedure 6:

The corresponding starting material was dissolved in MeOH. After addition of 2M HCl in diethyl ether (10 eq.) the product was crystallized by evaporation of the solvent at room temperature.

### General procedure 7:

Reaction was performed as follows: A solution of the starting material in DCM (150 mL/g) was cooled to o °C and trifluoroacetic acid (10 mL/g) was added. The reaction mixture was warmed to room temperature and stirred for 15 minutes. DCM and trifluoroacetic acid were removed under reduced pressure to obtain the product.

### General procedure 8:

Reaction was performed similar to lit. (Laras et al. 2009). To a solution of the residue in THF (150 mL/g) was added *N,N-*Diisopropylethylamine (4.00 eq.). The solution was cooled to o °C, the corresponding acid chloride (1.20 eq.) was added, and the reaction mixture was warmed up to room temperature. After completion of the reaction, HCl (1M in water) was added carefully. The product was extracted with ethyl acetate twice, the organic layer was dried (Na₂SO₄) and the solvent was removed under reduced pressure. The crude product was purified by column chromatography (DCM/MeOH/7 N NH₃ in MeOH 95:4:1) and crystallization from petroleum ether and DCM.

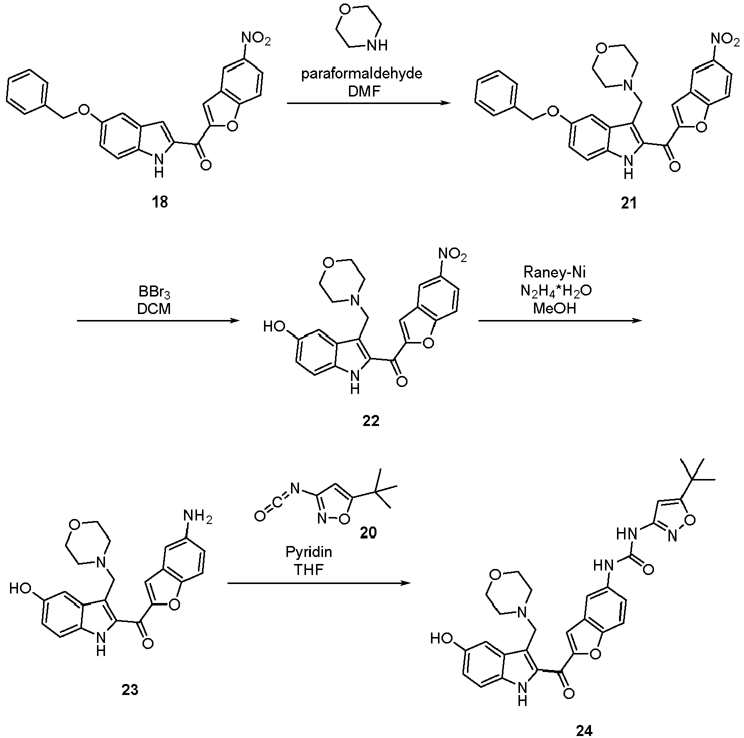

### (5-(Benzyloxy)-3-(morpholinomethyl)-1H-indol-2-yl)(5-nitrobenzofuran-2-yl)methanone (21)

**C₂₉H₂₅N₃O₆ (*M* = 511.53 g/mol)**

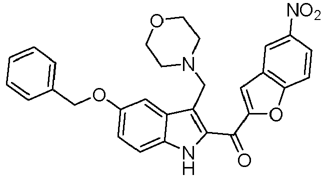

### Synthesis of 21 from 18 and morpholine as described in general procedure 1. Yield: 34%.

**¹H-NMR** (DMSO-d₆, 300 MHz): δ = 11.79 (s, 1H), 8.83 (d, *J* = 2.4 Hz, 1H), 8.43 (dd, *J* = 9.0, 2.4 Hz, 1H), 8.10 - 8.02 (m, 2H), 7.52 - 7.47 (m, 2H), 7.47 - 7.37 (m, 4H), 7.36 - 7.29 (m, 1H), 7.10 (dd, *J* = 9.1, 2.3 Hz, 1H), 5.16 (s, 2H), 3.90 (s, 2H), 3.42 - 3.37 (m, 4H), 2.24 (br s, 4H).

**Mp:** decomposition from 196 °C.

### (5-Hydroxy-3-(morpholinomethyl)-1H-indol-2-yl)(5-nitrobenzofuran-2-yl)methanone (22)

**C₂₂H₁₉N₃O₆ (*M* = 421.41 g/mol)**

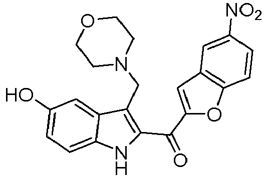

Reaction was performed: A solution of **21** in DCM (200 mL/g) was cooled to -78 °C. BBr₃ (5.00 eq.) was added dropwise, and the reaction mixture was stirred at -78 °C for 1 h. After completion of the reaction DCM was removed under reduced pressure and NaHCO₃ (sat. in water) was added. The product was extracted with ethyl acetate (3x), the organic layer was dried (Na₂SO₄), and the solvent was removed under reduced pressure. The crude product was purified with cc (dichloromethane: ammonia (7M in methanol) 99:1). Yield: 70%.

**¹H-NMR** (DMSO-*d*₆, 400 MHz): δ = 11.62 (s, 1H), 9.07 (s, 1H), 8.83 (d, *J* = 2.4 Hz, 1H), 8.42 (dd, *J* = 9.1, 2.5 Hz, 1H), 8.05 (d, *J* = 9.2 Hz, 1H), 8.02 (s, 1H), 7.36 (d, *J* = 8.8 Hz, 1H), 7.19 (d, *J* = 2.2 Hz, 1H), 6.92 (dd, *J* = 8.8, 2.3 Hz, 1H), 3.86 (s, 2H), 3.45 (t, *J* = 4.5 Hz, 4H), 2.28 (t, *J* = 4.5 Hz, 4H).

**Mp:** decomposition from 240 °C.

### (5-Aminobenzofuran-2-yl)(5-hydroxy-3-(morpholinomethyl)-1H-indol-2-yl)methanone (23)

**C₂₂H₂₁N₃O₄ (*M* = 391.43 g/mol)**

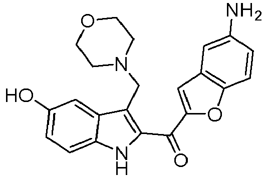

Reaction was performed similar to lit. (Orús et al. 2002). Raney-Ni (10% w/w) was added to a solution of **22** in MeOH (200 mL/g). Hydrazine hydrate (3.00 eq.) was added dropwise, and the reaction mixture was stirred at room temperature for 1 h. After completion of the reaction the reaction mixture was filtered over a pad of celite, and the filtrate was further used. The solvent was removed under reduced pressure and the residue was dissolved in ethyl acetate. The organic layer was washed with water twice, dried (Na₂SO₄), and the solvent was removed under reduced pressure. The crude product was used without further purification.

**¹H-NMR** (DMSO-*d*₆, 400 MHz): δ = 11.45 (s, 1H), 8.98 (s, 1H), 7.64 (d, *J* = 0.9 Hz, 1H), 7.44 (dd, *J* = 9.6, 0.9 Hz, 1H), 7.33 (d, *J* = 8.8 Hz, 1H), 7.18 (d, *J* = 2.3 Hz, 1H), 6.90 - 6.84 (m, 3H), 5.09 (s, 2H), 3.85 (s, 2H), 3.49 (t, *J* = 4.6 Hz, 4H), 2.31 (t, *J* = 4.6 Hz, 4H).

### (5-Aminobenzofuran-2-yl)(5-hydroxy-3-(morpholinomethyl)-1H-indol-2-yl)methanone (24a)

**C₃₀H₃₁N₅O₆ (*M* = 557.61 g/mol)**

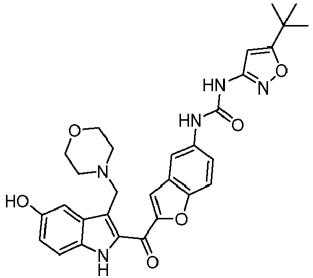

Synthesis of **24a** from **23** as described in general procedure 2. The crude product was purified by cc (DCM:ammonia (7M in MeOH) 95:5). Yield: 20% (over 2 steps). Additional purification was done by prep. HPLC to achieve a TFA salt of the corresponding amine.

**¹H-NMR** (DMSO-d₆, 400 MHz): δ = 11.53 (s, 1H), 9.55 (s, 1H), 9.01 (s, 1H), 8.98 (s, 1H), 8.06 (d, *J* = 2.1 Hz, 1H), 7.84 (d, *J* = 0.9 Hz, 1H), 7.72 (d, *J* = 9.0 Hz, 1H), 7.50 (dd, *J* = 9.0, 2.3 Hz, 1H), 7.35 (d, *J* = 8.8 Hz, 1H), 7.19 (d, *J* = 2.3 Hz, 1H), 6.89 (dd, *J* = 8.8, 2.3 Hz, 1H), 6.52 (s, 1H), 3.86 (s, 2H), 3.48 (t, *J* = 4.6 Hz, 4H), 2.31 (t, *J* = 4.6 Hz, 4H), 1.30 (s, 9H).

### (5-Aminobenzofuran-2-yl)(5-hydroxy-3-(morpholinomethyl)-1H-indol-2-yl)methanone 2,2,2-trifluoroacetate (24b)

**C₃₂H₃₂F₃N₅O₈ (*M* = 671.63 g/mol)**

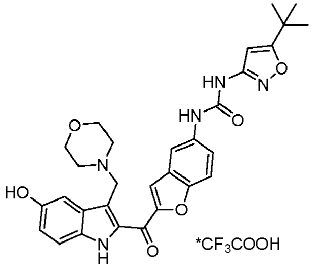

**¹H-NMR** (DMSO-*d*₆, 300 MHz): δ =

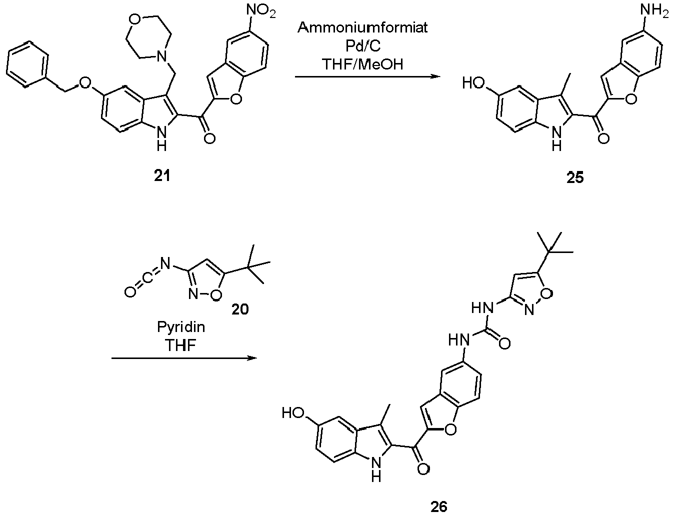

### (5-Aminobenzofuran-2-yl)(5-hydroxy-3-methyl-1H-indol-2-yl)methanone (25)

**C₁₈H₁₄N₂O₃ (*M* = 306.32 g/mol)**

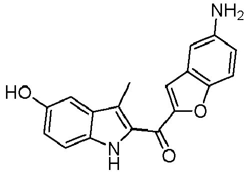

Synthesis of **25** from **21** as described in general procedure 3. The crude product was purified by cc (DCM:MeOH 10:1). Yield: 45%.

**¹H-NMR** (DMSO-*d*₆, 300 MHz): δ 11.26 (s, 1H), 9.02 (s, 1H), 7.56 (d, *J* = 0.9 Hz, 1H), 7.50 - 7.40 (m, 1H), 7.35 (d, *J* = 8.7 Hz, 1H), 6.95 - 6.83 (m, 4H), 5.20 (br s, 2H), 2.45 (s, 3H).

### 1-(5-(tert-Butyl)isoxazol-3-yl)-3-(2-(5-hydroxy-3-methyl-1H-indole-2-carbonyl)benzofuran-5-yl)urea (26)

**C₂₆H₂₄N₄O₅ (*M* = 472.50 g/mol)**

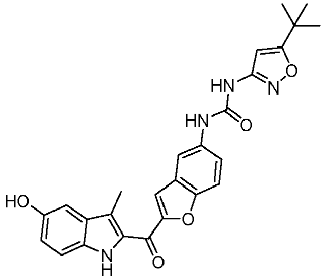

Synthesis of **26** from **25** as described in general procedure 2. The crude product was purified by cc (DCM:MeOH 10:1). Yield: 49%. Additional purification was done by prep. HPLC.

**¹H-NMR** (DMSO-d₆, 300 MHz): δ = δ 11.33 (s, 1H), 9.56 (s, 1H), 9.04 (s, 1H), 8.97 (s, 1H), 8.07 (d, *J* = 2.1 Hz, 1H), 7.77 (d, *J* = 0.9 Hz, 1H), 7.73 (d, *J* = 9.0 Hz, 1H), 7.50 (dd, *J* = 9.0, 2.3 Hz, 1H), 7.36 (dd, *J* = 8.5, 1.0 Hz, 1H), 6.94 - 6.88 (m, 2H), 6.52 (s, 1H), 2.47 (s, 3H), 1.30 (s, 9H).

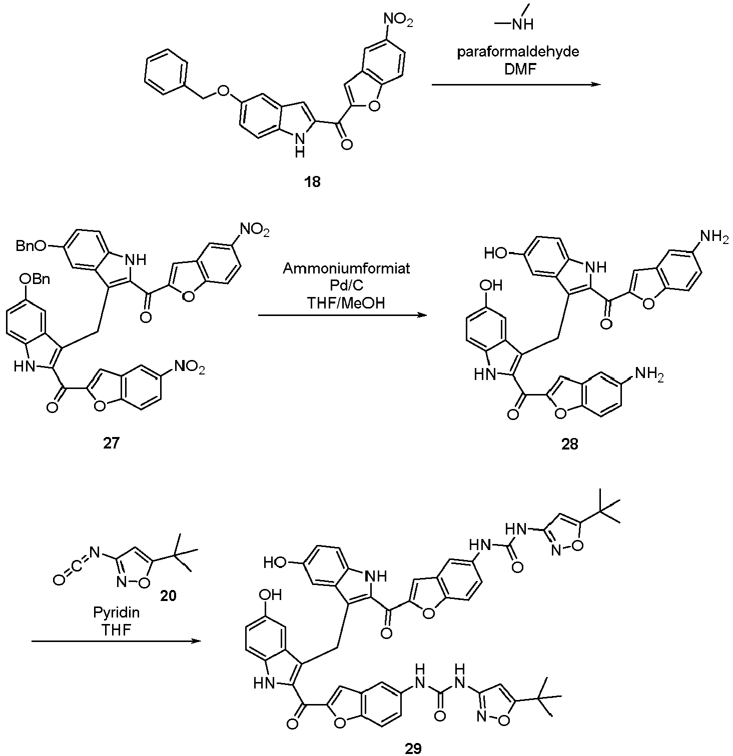

### (Methylenebis(5-(benzyloxy)-1H-indole-3,2-diyl))bis((5-nitrobenzofuran-2-yl)methanone) (27)

**C₄₉H₃₂N₄O₁₀ (*M* = 836.81 g/mol)**

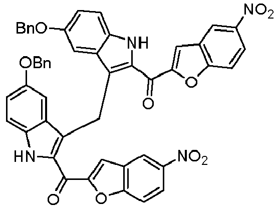

Synthesis of **27** from **18** and dimethylamine as described in general procedure 1. Yield: 22%. **¹H-NMR** (DMSO-*d*₆, 400 MHz): δ = 11.65 (s, 2H), 8.66 (d, *J* = 2.4 Hz, 2H), 8.26 (dd, *J* = 9.3, 2.4 Hz, 2H), 7.88 (d, *J* = 8.9 Hz, 2H), 7.88 (d, *J* = 0.9 Hz, 2H), 7.39 (dd, *J* = 8.6, 0.9 Hz, 2H), 7.27 - 7.22 (m, 6H), 7.16 (dd, *J* = 6.6,3.1 Hz, 4H), 6.97 - 6.93 (m, 4H), 5.00 (s, 2H), 4.64 (s, 4H).

**Mp:** decomposition from 224 °C.

### (Methylenebis(5-hydroxy-1H-indole-3,2-diyl))bis((5-aminobenzofuran-2-yl)methanone) (28)

**C₃₅H₂₄N₄O₆ (*M* = 596.60 g/mol)**

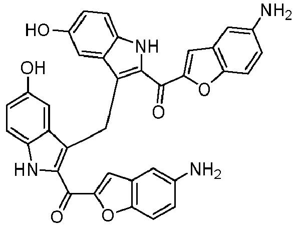

Synthesis of **28** from **27** as described in general procedure 3. The crude product was purified by cc (DCM:MeOH 10:1). Yield: 70%.

**¹H-NMR** (DMSO-*d*₆, 400 MHz): δ = 11.31 (s, 2H), 8.74 (s, 2H), 7.60 (s, *J* = 0.9, 2H), 7.42 (dt, *J* = 8.4, 1.1 Hz, 2H), 7.30 (d, *J* = 8.8 Hz, 2H), 6.90 - 6.84 (m, 4H), 6.76 (dd, *J* = 8.8, 2.3 Hz, 2H), 6.58 (d, *J* = 2.3 Hz, 2H), 5.09 (s, 4H), 4.85 (s, 2H).

### 1,1'-((3,3'-Methylenebis(5-hydroxy-1H-indole-3,2-diyl-2-carbonyl))bis(benzofuran-2,5-diyl))bis(3-(5-(tert-butyl)isoxazol-3-yl)urea) (29)

**C₅₁H₄₄N₈O₁₀ (*M* = 928.96 g/mol)**

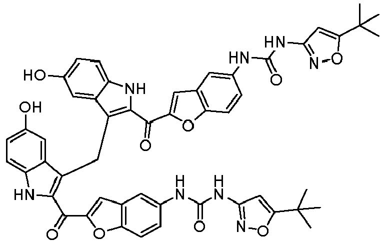

Synthesis of **29** from **28** as described in general procedure 2. The crude product was purified by cc (DCM:MeOH 10:1). Yield: 40%.

**¹H-NMR** (DMSO-*d*₆, 300 MHz): δ = 11.39 (s, 2H), 9.53 (s, 2H), 8.93 (s, 2H), 8,81 (s, 2H), 8.04 (d, *J* = 2.1 Hz, 2H), 7.75 (d, *J* = 1.0 Hz, 2H), 7.68 (d, *J* = 8.9 Hz, 2H), 7.46 (dd, *J* = 9.0, 2.3 Hz, 2H), 7.32 (d, *J* = 8.8 Hz, 2H), 6.80 (dd, *J* = 8.8, 2.3 Hz, 2H), 6.60 (d, *J* = 2.3 Hz, 2H), 6.53 (s, 2H), 4.88 (s, 2H), 1.30 (s, 18H).

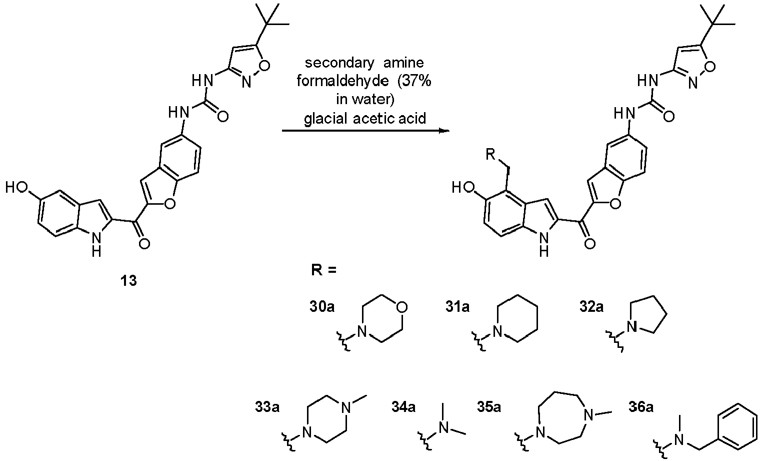

### 1-(5-(tert-Butyl)isoxazol-3-yl)-3-(2-(5-hydroxy-4-(morpholinomethyl)-1H-indole-2-carbonyl)benzofuran-5-yl)urea (30a)

**C₃₀H₃₁N₅O₆ (*M* = 557.61 g/mol)**

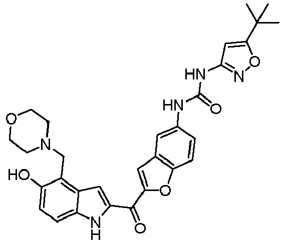

Synthesis of **30a** from **13** and morpholine as described in general procedure 1. Yield: 50%.

Synthesis of **30a** from **13** and morpholine as described in general procedure 4A. Yield: 28%.

Synthesis of **30a** from **13** and morpholine as described in general procedure 4B. Yield: 87%.

Additional purification was done by prep. HPLC to achieve a TFA salt of the corresponding amine. **¹H-NMR** (DMSO-*d*₆, 300 MHz): δ = 11.86 (s, 1H), 10.15 (s, 1H), 9.59 (s, 1H), 9.01 (s, 1H), 8.10 (d, *J* = 1.6 Hz, 1H), 8.00 (s, 1H), 7.83 - 7.70 (m, 2H), 7.51 (dd, *J* = 9.0, 2.0 Hz, 1H), 7.27 (d, *J* = 8.9 Hz, 1H), 6.88 (d, *J* = 8.8 Hz, 1H), 6.53 (s, 1H), 3.99 (s, 2H), 3.64 (s, 4H), 2.55 (s, 4H), 1.30 (s, 9H).

**¹H-NMR** (DMSO-*d*₆, 600 MHz): δ = 11.84 (d, 1H , *J* = 1.6 Hz), 10.16 (s, 1H), 9.57 (s, 1H), 9.00 (s, 1H), 8.10 (d, *J* = 2.2 Hz, 1H), 8.00 (d, *J* = 1.0 Hz, 1H), 7.78 - 7.73 (m, 2H), 7.51 (dd, *J* = 9.0, 2.2 Hz, 1H), 7.28 (d, *J* = 8.9 Hz, 1H), 6.89 (d, *J* = 8.8 Hz, 1H), 6.53 (s, 1H), 3.99 (s, 2H), 3.64 (s, 4H), 2.56 (s, 4H), 1.30 (s, 9H).

**¹³C-NMR** (DMSO-*d*₆, 151 MHz): δ = 180.65, 173.53, 158.90, 153.26, 152.09, 151.73, 151.08, 135.74, 134.10, 133.63, 128.28, 127.74, 121.17, 118.56, 114.88, 112.93, 112.82, 112.50, 112.02, 109.35, 92.94, 66.75, 56.37, 53.35, 32.95, 28.83.

**MP:** decomposition from 241 °C.

### 1-(5-(tert-Butyl)isoxazol-3-yl)-3-(2-(5-hydroxy-4-(morpholinomethyl)-1H-indole-2-carbonyl)benzofuran-5-yl)urea 2,2,2-trifluoroacetate (30b)

**C₃₂H₃₂F₃N₅O₈ (*M* = 671.63 g/mol)**

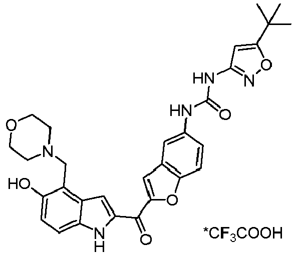

**¹H-NMR** (Methanol-*d*₄, 400 MHz): δ = 7.99 (s, 1H), 7.86 (s, 2H), 7.65 (d, *J* = 8.9 Hz, 1H), 7.55 -7.46 (m, 2H), 7.07 (d, *J* = 8.9 Hz, 1H), 6.34 (s, 1H), 4.74 (s, 2H), 4.16 - 3.96 (m, 2H), 3.92 - 3.72 (m, 2H), 3.62 - 3.51 (m, 2H), 3.49 - 3.36 (m, 2H), 1.35 (s, 9H).

**¹⁹F-NMR** (Methanol-*d*₄, 377 MHz): δ = - 75.30.

### 1-(5-(tert-Butyl)isoxazol-3-yl)-3-(2-(5-hydroxy-4-(piperidin-1-ylmethyl)-1H-indole-2-carbonyl)benzofuran-5-yl)urea (31a)

**C₃₁H₃₃N₅O₅ (*M* = 555.64 g/mol)**

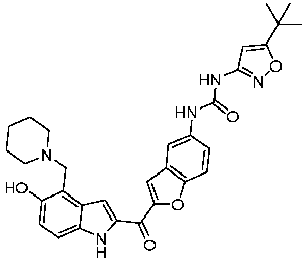

Synthesis of **31a** from **13** and piperidine as described in general procedure 1. Yield: 29%.

Synthesis of **31a** from **13** and piperidine as described in general procedure 4A. Yield: 46%.

Synthesis of **31a** from **13** and piperidine as described in general procedure 4B. Yield: 73%.

Additional purification was done by prep. HPLC to achieve a TFA salt of the corresponding amine.

**¹H-NMR** (DMSO-*d*₆, 400 MHz): δ = 11.84 (s, 1H), 10.89 (br s, 1H), 9.57 (s, 1H), 8.99 (s, 1H), 8.09 (d, *J* = 2.1 Hz, 1H), 8.00 (s, 1H), 7.76 (d, *J* = 9.0 Hz, 1H), 7.71 (s, 1H), 7.51 (dd, *J* = 9.0, 2.2 Hz, 1H), 7.26 (d, *J* = 8.8 Hz, 1H), 6.84 (d, *J* = 8.8 Hz, 1H), 6.52 (s, 1H), 4.00 (s, 2H), 2.55 (s, 4H), 1.56 - 1.53 (m, 4H), 1.52 - 1.42 (m, 2H), 1.31 (s, 9H).

**¹H-NMR** (DMSO-*d*₆, 300 MHz): δ = 11.86 (s, 1H), 9.61 (s, 1H), 9.04 (s, 1H), 8.10 (d, *J* = 2.1 Hz, 1H), 8.01 (d, *J* = 0.7 Hz, 1H), 7.76 (d, *J* = 9.0 Hz, 1H), 7.72 (s, 1H), 7.51 (dd, *J* = 9.0, 2.2 Hz, 1H), 7.27 (d, *J* = 8.8 Hz, 1H), 6.85 (d, *J* = 8.8 Hz, 1H), 6.53 (s, 1H), 4.02 (s, 2H), 2.57 (s, 4H), 1.65 - 1.52 (m, 4H), 1.50 - 1.36 (m, 2H), 1.30 (s, 9H).

**MP:** decomposition from 199 °C.

### 1-(5-(tert-Butyl)isoxazol-3-yl)-3-(2-(5-hydroxy-4-(piperidin-1-ylmethyl)-1H-indole-2-carbonyl)benzofuran-5-yl)urea 2,2,2-trifluoroacetate (31b)

**C₃₃H₃₄F₃N₅O₇ (*M* = 669.65 g/mol)**

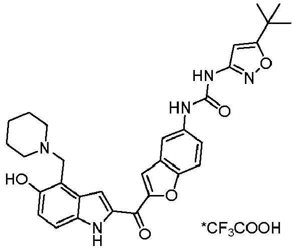

**¹H-NMR** (DMSO-*d*₆, 300 MHz): δ = 12.10 (s, 1H), 9.92 (s, 1H), 9.65 (s, 1H), 9.12 (s, 1H), 8.15 (d, *J* = 1.9 Hz, 1H), 8.06 (s, 1H), 7.87 (d, *J* = 0.6 Hz, 1H), 7.76 (d, *J* = 8.9 Hz, 1H), 7.51 (dd, *J* = 9.0, 2.2 Hz, 2H), 7.49 (d, *J* = 8.9 Hz, 1H), 7.10 (d, *J* = 8.9 Hz, 1H), 6.52 (s, 1H), 4.55 (s, 2H), 3.57 - 3.41 (m, 2H), 3.17 - 2.99 (m, 2H), 1.88 - 1.60 (m, 6H), 1.30 (s, 9H).

**¹H-NMR** (Methanol-*d*₄, 400 MHz): δ = 7.95 (s, 1H), 7.78 (s, 1H), 7.77 (s, 1H), 7.59 (d, *J* = 8.8 Hz, 1H), 7.47 (d, *J* = 8.6 Hz, 1H), 7.43 (d, *J* = 8.9 Hz, 1H), 7.02 (d, *J* = 8.9 Hz, 1H), 6.32 (s, 1H), 4.63 (s, 2H), 3.62 (d, *J* = 12.4 Hz, 2H), 3.17 (t, *J* = 11.3 Hz, 2H), 1.95 (d, *J* = 13.2 Hz, 2H), 1.87 - 1.74 (m, 3H), 1.62 - 1.48 (m, 1H), 1.33 (s, 9H).

**¹⁹F-NMR** (Methanol-*d*₄, 377 MHz): δ = - 75.26.

### 1-(5-(tert-Butyl)isoxazol-3-yl)-3-(2-(5-hydroxy-4-(pyrrolidin-1-ylmethyl)-1H-indole-2-carbonyl)benzofuran-5-yl)urea (32a)

**C₃₀H₃₁N₅O₅ (*M* = 541.61 g/mol)**

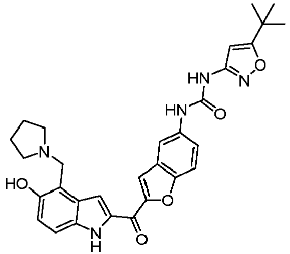

Synthesis of **32a** from **13** and pyrrolidine as described in general procedure 1. Yield: 6%.

Synthesis of **32a** from **13** and pyrrolidine as described in general procedure 4A. Yield: 51%.

Synthesis of **32a** from **13** and pyrrolidine as described in general procedure 4B. Yield: 41%. Additional purification was done by prep. HPLC to achieve a TFA salt of the corresponding amine.

**¹H-NMR** (DMSO-*d*₆, 300 MHz): δ = 11.88 (s, 1H), 9.66 (s, 1H), 9.17 (s, 1H), 8.10 (d, *J* = 2.1 Hz, 1H), 8.02 (d, *J* = 0.7 Hz, 1H), 7.76 (d, *J* = 9.0 Hz, 1H), 7.73 (s, 1H), 7.52 (dd, *J* = 9.0, 2.2 Hz, 1H), 7.28 (d, *J* = 8.9 Hz, 1H), 6.88 (d, *J* = 8.8 Hz, 1H), 6.53 (s, 1H), 4.19 (s, 2H), 2.79 - 2.64 (m, 4H), 1.86 - 1.74 (m, 4H), 1.30 (s, 9H).

**¹³C-NMR** (DMSO-*d*₆, 101 MHz): δ = 180.1, 173.1, 158.4, 152.7, 151.7, 151.2, 151.1, 135.3, 133.7, 133.0, 127.3, 127.2, 120.6, 118.0, 114.5, 112.5, 112.4, 111.9, 111.9, 108.4, 92.5, 53.3, 53.1, 32.4, 28.3, 23.2.

**MP:** decomposition from 203 °C.

### 1-(5-(tert-Butyl)isoxazol-3-yl)-3-(2-(5-hydroxy-4-(pyrrolidin-1-ylmethyl)-1H-indole-2-carbonyl)benzofuran-5-yl)urea 2,2,2-trifluoroacetate (32b)

**C₃₂H₃₂F₃N₅O₇ (*M* = 655.62 g/mol)**

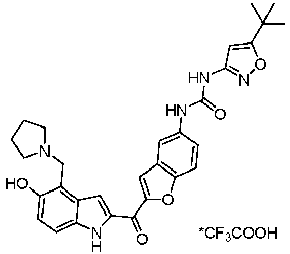

**¹H-NMR** (DMSO-*d*₆, 300 MHz): δ = 12.11 (s, 1H), 9.95 (s, 1H), 9.69 (s, 1H), 9.52 (s, 1H), 9.15 (s, 1H), 8.15 (d, *J* = 1.9 Hz, 1H), 8.07 (s, 1H), 7.92 (d, *J* = 0.9 Hz, 1H), 7.76 (d, *J* = 9.0 Hz, 1H), 7.53 (d, *J* = 2.1 Hz, 1H), 7.48 (d, *J* = 9.4 Hz, 1H), 7.10 (d, *J* = 8.8 Hz, 1H), 6.52 (s, 1H), 4.66 (s, 2H), 3.54 - 3.41 (m, 2H), 3.32 - 3.19 (m, 2H), 2.15 - 2.01 (m, 2H), 1.96 - 1.79 (m, 2H), 1.31 (s, 9H).

**¹H-NMR** (Methanol-*d*₄, 400 MHz): δ = 7.91 (s, 1H), 7.77 (s, 1H), 7.75 (s, 1H), 7.56 (d, *J* = 8.7 Hz, 1H), 7.45 (d, *J* = 8.9 Hz, 1H), 7.41 (d, *J* = 8.9 Hz, 1H), 7.02 (d, *J* = 8.9 Hz, 1H), 6.31 (s, 1H), 4.71 (s, 2H), 3.65 - 3.52 (m, 2H), 3.43 - 3.33 (m, 2H), 2.28 - 2.15 (m, 2H), 2.10 - 1.95 (m, 2H), 1.32 (s, 9H).

**¹⁹F-NMR** (Methanol-*d*₄, 377 MHz): δ = - 75.27.

### 1-(5-(tert-Butyl)isoxazol-3-yl)-3-(2-(5-hydroxy-4-((4-methylpiperazin-1-yl)methyl)-1H-indole-2-carbonyl)benzofuran-5-yl)urea (33a)

**C₃₁H₃₄N₆O₅ (*M* = 570.65 g/mol)**

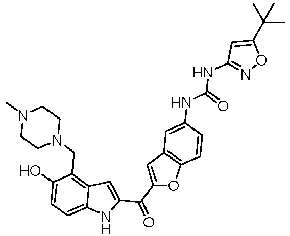

Synthesis of **33a** from **13** and *N*-methyl-piperazine as described in general procedure 1. Yield: 24%.

Synthesis of **33a** from **13** and *N*-methyl-piperazine as described in general procedure 4A. Yield: 94%.

Synthesis of **33a** from **13** and *N*-methyl-piperazine as described in general procedure 4B. Yield: 79%.

Additional purification was done by prep. HPLC to achieve a TFA salt of the corresponding amine.

**¹H-NMR** (DMSO-*d*₆, 400 MHz): δ = 11.83 (s, 1H), 10.51 (br s, 1H), 9.58 (s, 1H), 9.04 (s, 1H), 8.09 (d, *J* = 2.0 Hz, 1H), 8.00 (d, *J* = 0.6 Hz, 1H), 7.76 (d, *J* = 9.0 Hz, 1H), 7.74 (s, 1H), 7.52 (dd, *J* = 9.0, 2.2 Hz, 1H), 7.27 (d, *J* = 8.8 Hz, 1H), 6.86 (d, *J* = 8.8 Hz, 1H), 6.52 (s, 1H), 4.01 (s, 2H), 2.77 - 2.52 (m, 4H), 2.47 - 2.24 (m, 4H), 2.19 (s, 3H), 1.30 (s, 9H).

**¹³C-NMR** (DMSO-*d*₆, 101 MHz): δ = 180.2, 173.0, 158.4, 152.8, 151.6, 151.3, 150.9, 135.3, 133.6, 133.1, 127.5,127.3,120.7,127.3,120.7,118.1,114.4,112.5, 112.2, 112.0, 111.5, 108.6, 92.5, 55.9, 54.7, 52.2, 45.5, 32.5, 28.4.

**MP:** decomposition from 212 °C.

### 1-(5-(tert-Butyl)isoxazol-3-yl)-3-(2-(5-hydroxy-4-((4-methylpiperazin-1-yl)methyl)-1H-indole-2-carbonyl)benzofuran-5-yl)urea bis(2,2,2-trifluoroacetate) (33b)

**C₃₅H₃₆F₆N₆O₉ (*M*** = **798.69 g/mol)**

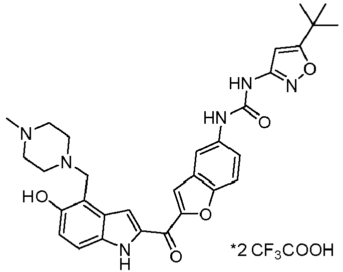

**¹H-NMR** (DMSO-*d*₆, 300 MHz): δ = 11.99 (s, 1H), 9.69 (s, 1H), 9.15 (s, 1H), 8.13 (d, *J* = 2.0 Hz, 1H), 8.03 (s, 1H), 7.81 (s, 1H), 7.76 (d, *J* = 9.0 Hz, 1H), 7.51 (dd, *J* = 9.0, 2.2 Hz, 1H), 7.39 (d, *J* = 9.0 Hz, 1H), 7.03 (d, *J* = 9.0 Hz, 1H), 6.51 (s, 1H), 3.91 - 3.27 (m, 10H), 2.74 (s, 3H), 1.30 (s, 9H).

**¹H-NMR** (Methanol-*d*₄, 400 MHz): δ = 7.92 (s, 1H), 7.79 (s, 2H), 7.61 (d, *J* = 8.9 Hz, 1H), 7.48 (d, *J* = 9.1 Hz, 1H), 7.42 (d, *J* = 8.8 Hz, 1H), 7.02 (d, *J* = 8.9 Hz, 1H), 6.26 (s, 1H), 4.60 (s, 2H), 3.68 - 3.55 (m, 8H), 2.87 (s, 3H), 1.33 (s, 9H).

**¹⁹F-NMR** (Methanol-*d*₄, 377 MHz): δ = - 75.39.

### 1-(5-tert-Butylisoxazol-3-yl)-3-(2-(4-((dimethylamino)methyl)-5-hydroxy-1H-indole-2-carbonyl)benzofuran-5-yl)urea (34a)

**C₂₈H₂₉N₅O₅ (*M* = 515.56 g/mol)**

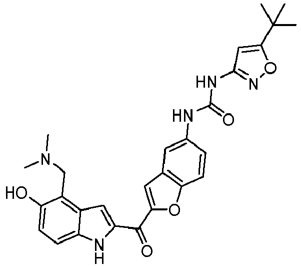

Synthesis of **34a** from **13** and dimethylamine (40% in water or 2M in THF) as described in general procedure 4A. Yield: 69%.

Synthesis of **34a** from **13** and dimethylamine (40% in water or 2M in THF) as described in general procedure 4B. Yield: 48%.

Additional purification was done by prep. HPLC to achieve a TFA salt of the corresponding amine. **¹H-NMR** (DMSO-*d*₆, 400 MHz): δ = 11.84 (s, 1H), 9.58 (s, 1H), 9.03 (s, 1H), 8.09 (d, *J* = 2.1 Hz, 1H), 8.00 (s, 1H), 7.77 (d, *J* = 8.9 Hz, 1H), 7.70 (s, 1H), 7.52 (dd, *J* = 9.0, 2.2 Hz, 1H), 7.29 (d, *J* = 8.8 Hz, 1H), 6.87 (d, *J* = 8.8 Hz, 1H), 6.53 (s, 1H), 3.97 (s, 2H), 2.35 (s, 6H), 1.30 (s, 9H).

**¹³C-NMR** (DMSO-*d*₆, 101 MHz): δ = 180.2, 173.0, 158.4, 152.8, 151.6, 151.3, 151.2, 135.3, 133.6, 133.0, 127.5, 127.3, 120.7, 118.1, 114.4, 112.5, 112.4, 112.0, 111.8, 108.4, 92.5, 57.4, 44.4, 32.5, 28.4.

**MP:** decomposition from 217 °C.

### 1-(5-tert-Butylisoxazol-3-yl)-3-(2-(4-((dimethylamino)methyl)-5-hydroxy-1H-indole-2-carbonyl)benzofuran-5-yl)urea 2,2,2-trifluoroacetate (34b)

**C₃₀H₃₀F₃N₅O₇ (*M* = 629.58 g/mol)**

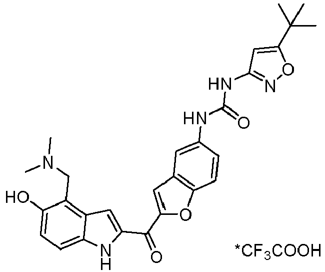

**¹H-NMR** (DMSO-*d*₆, 400 MHz): δ = 12.11 (d, *J* = 2.0 Hz, 1H), 9.97 (s, 1H), 9.64 (s, 1H), 9.24 (s, 1H), 9.08 (s, 1H), 8.15 (d, *J* = 2.0 Hz, 1H), 8.05 (d, *J* = 0.8 Hz, 1H), 7.89 (d, *J* = 1.8 Hz, 1H), 7.76 (d, *J* = 9.2 Hz, 1H), 7.51 (dd, *J* = 9.2, 2.2 Hz, 1H), 7.49 (d, *J =* 8.8 Hz, 1H), 7.10 (d, *J* = 9.0 Hz, 1H), 6.51 (s, 1H), 4.58 (s, 2H), 2.84 (s, 6H), 1.30 (s, 9H).

**¹H-NMR** (Methanol-*d*₄, 300 MHz): δ = 8.07 (d, *J* = 1.9 Hz, 1H), 7.91 (s, 1H), 7.90 (s, 1H), 7.71 (d, *J* = 8.9 Hz, 1H), 7.55 (dd, *J* = 8.9, 2.1 Hz, 1H), 7.53 (d, *J* = 8.9 Hz, 1H), 7.09 (d, *J* = 8.9 Hz, 1H), 6.39 (s, 1H), 4.70 (s, 2H), 2.96 (s, 6H), 1.37 (s, 9H).

### 1-(5-(tert-Butyl)isoxazol-3-yl)-3-(2-(5-hydroxy-4-((4-methyl-1,4-diazepan-1-yl)methyl)-1H-indole-2-carbonyl)benzofuran-5-yl)urea (35a)

**C₃₂H₃₆N₆O₅ (*M* = 584.68 g/mol)**

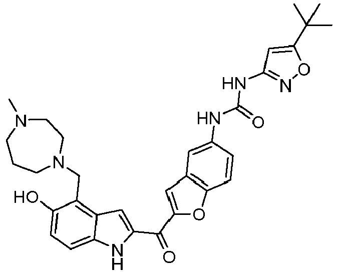

Synthesis of **35a** from **13** and 1-methyl-1,4-diazepane as described in general procedure 4A. Yield: 23%. Synthesis of **35a** from **13** and 1-methyl-1,4-diazepane as described in general procedure 4B. Yield: 64%. Additional purification was done by prep. HPLC to achieve a TFA salt of the corresponding amine.

**¹H-NMR** (DMSO-*d₆*, 300 MHz): δ = 11.85 (s, 1H), 9.66 (s, 1H), 9.11 (s, 1H), 8.09 (d, *J* = 2.2 Hz, 1H), 8.01 (s, 1H), 7.76 (d, *J* = 9.0 Hz, 1H), 7.73 (s, 1H), 7.52 (dd, *J* = 9.0, 2.2 Hz, 1H), 7.27 (d, *J* = 8.8 Hz, 1H), 6.85 (d, *J* = 8.8 Hz, 1H), 6.53 (s, 1H), 4.11 (s, 2H), 2.86 - 2.75 (m, 4H), 2.65 - 2.56 (m, 4H), 2.27 (s, 3H), 1.85 - 1.76 (m, 2H), 1.30 (s, 9H).

### 1-(2-(4-((Benzyl(methyl)amino)methyl)-5-hydroxy-1H-indole-2-carbonyl)benzofuran-5-yl)-3-(5-(tert-butyl)isoxazol-3-yl)urea (36a)

**C₃₄H₃₃N₅O₅ (*M* = 591.67 g/mol)**

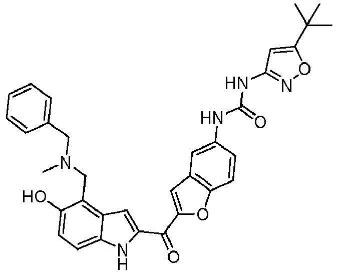

Synthesis of **36a** from **13** and *N*-methyl-1-phenylmethanamine as described in general procedure 4A. Yield: 69%.

Synthesis of **36a** from **13** and *N*-methyl-1-phenylmethanamine as described in general procedure 4B. Yield: 81%.

Additional purification was done by prep. HPLC to achieve a TFA salt of the corresponding amine. **¹H-NMR:** (DMSO-*d*₆, 300 MHz): δ = 11.87 (s, 1H), 10.41 (s, 1H), 9.60 (s, 1H), 9.02 (s, 1H), 8.12 (d, *J* = 2.2 Hz, 1H), 7.98 (d, *J* = 1.9 Hz, 2H), 7.80 - 7.71 (m, 2H), 7.52 (dd, *J* = 9.0, 2.2 Hz, 1H), 7.42 - 7.22 (m, 6H), 6.90 (d, *J* = 8.8 Hz, 1H), 6.53 (s, 1H), 4.02 (s, 2H), 3.67 (s, 2H), 2.20 (s, 3H), 1.31 (s, 9H).

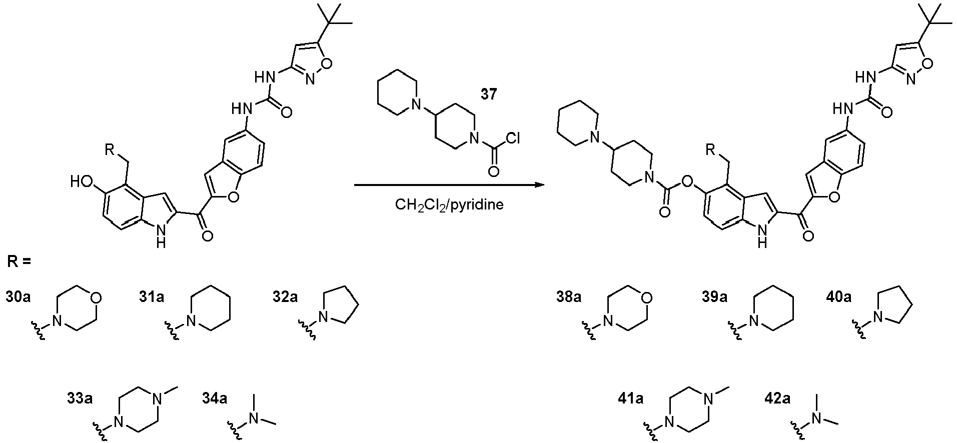

### 2-(5-(3-(5-(tert-Butyl)isoxazol-3-yl)ureido)benzofuran-2-carbonyl)-4-(morpholinomethyl)-1H-indol-5-yl [1,4'-bipiperidine]-1'-carboxylate (38a)

**C₄₁H₄₉N₇O₇ (*M* = 751.89 g/mol)**

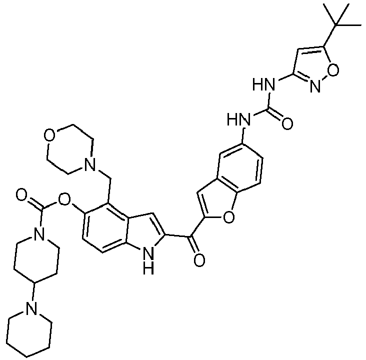

Synthesis of **38a** from **30a** as described in general procedure 5. Yield: 76%.

Additional purification was done by prep. HPLC to achieve a TFA salt of the corresponding amine.

**¹H-NMR:** (DMSO-*d*₆, 300 MHz): δ = 12.13 (d, *J* = 1.4 Hz, 1H), 9.74 (s, 1H), 9.15 (s, 1H), 8.13 (d, *J* = 1.9 Hz, 1H), 8.01 (s, 2H), 7.74 (d, *J* = 9.0 Hz, 1H), 7.51 (dd, *J* = 8.9, 2.1 Hz, 1H), 7.40 (d, *J* = 8.9 Hz, 1H), 7.06 (d, *J* = 8.9 Hz, 1H), 6.54 (s, 1H), 4.28 (d, *J* = 12.1 Hz, 1H), 4.06 (d, *J* = 10.4 Hz, 1H), 3.73 (s, 2H), 3.60 - 3.51 (m, 4H), 3.11 - 2.97 (m, 1H)), 2.91 - 2.79 (m, 1H), 2.48 - 2.45 (m, 2H), 2.44 - 2.34 (m, 4H), 1.85 - 1.76 (m, 2H), 1.66 - 1.34 (m, 11H), 1.30 (s, 9H).

**MP:** 214 - 216 °C.

### 2-(5-(3-(5-(tert-Butyl)isoxazol-3-yl)ureido)benzofuran-2-carbonyl)-4-(morpholinomethyl)-1H-indol-5-yl [1,4'-bipiperidine]-1'-carboxylate bis(2,2,2-trifluoroacetate) (38b)

**C₄₅H₅₁F₆N₇O₁₁ (*M* = 979.92 g/mol)**

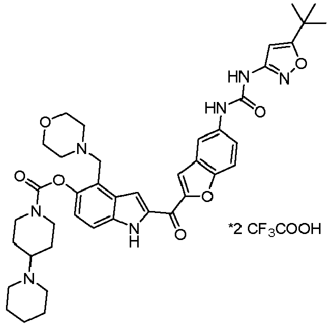

**¹H-NMR:** (DMSO-*d*₆, 300 MHz): δ = 12.45 (s, 1H), 10.27 (br s, 1H), 9.77 (s, 1H), 9.50 (br s, 1H), 9.26 (s, 1H), 8.17 (d, *J* = 2.0 Hz, 1H), 8.12 (s, 1H), 8.10 (s, 1H), 7.77 (d, *J* = 9.0 Hz, 1H), 7.64 (d, *J* = 8.6 Hz, 1H), 7.54 (dd, *J* = 9.0, 2.1 Hz, 1H), 7.23 (d, *J* = 9.0 Hz, 1H), 6.52 (s, 1H), 4.69 (s, 2H), 4.46 (d, *J* = 13.7 Hz, 1H), 4.19 (d, *J* = 11.5 Hz, 1H), 4.05 - 3.93 (m, 2H), 3.79 - 3.60 (m, 3H), 3.55 - 3.39 (m, 4H), 3.08 - 2.88 (m, 4H), 2.11 (d, *J* = 11.8 Hz, 2H), 1.93 - 1.61 (m, 9H), 1.51 - 1.37 (m, 1H), 1.30 (s, 9H).

### 2-(5-(3-(5-(tert-Butyl)isoxazol-3-yl)ureido)benzofuran-2-carbonyl)-4-(piperidin-1-ylmethyl)-1H-indol-5-yl [1,4'-bipiperidine]-1'-carboxylate (39a)

**C₄₂H₅₁N₇O₆ (M = 749.91 g/mol)**

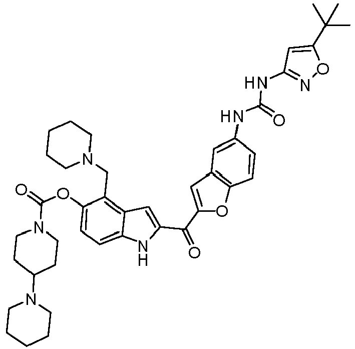

Synthesis of **38a** from **31a** as described in general procedure 5. Yield: 83%.

Additional purification was done by prep. HPLC to achieve a TFA salt of the corresponding amine.

**¹H-NMR** (DMSO-*d₆*, 400 MHz): δ = 12.09 (s, 1H), 9.65 (s, 1H), 9.28 (s, 1H), 8.12 (d, *J* = 2.1 Hz, 1H), 8.03 (d, *J* = 1.4 Hz, 1H), 7.99 (s, 1H), 7.71 (d, *J* = 9.0 Hz, 1H), 7.53 (dd, *J* = 9.0, 2.2 Hz, 1H), 7.40 (d, *J* = 9.0 Hz, 1H), 7.06 (d, *J* = 8.9 Hz, 1H), 6.53 (s, 1H), 4.30 (d, *J* = 9.9 Hz, 1H), 4.08 (d, *J* = 9.9 Hz, 1H), 3.70 (s, 2H), 3.10 - 2.98 (m, 1H), 2.92 - 2.79 (m, 1H), 2.72 - 2.55 (m, 4H), 2.44 - 2.31 (m, 4H), 2.04 - 1.86 (m, 2H), 1.64 - 1.35 (m, 15H), 1.30 (s, 9H).

**¹³C-NMR** (DMSO-*d*₆, 101 MHz): δ = 180.11, 173.21, 158.36, 153.18, 152.75, 151.65, 151.23, 149.55, 143.53, 136.11, 135.85, 135.43, 134.04, 127.87, 127.16, 123.87, 122.51, 120.80, 114.61, 112.25, 111.89, 111.09, 92.45, 61.72, 54.80, 54.10, 49.35, 43.33, 43.14, 32.43, 28.32, 27.06, 26.74, 25.66, 23.90.

**Mp:** 221 - 223 °C.

### 2-(5-(3-(5-(tert-Butyl)isoxazol-3-yl)ureido)benzofuran-2-carbonyl)-4-(piperidin-1-ylmethyl)-1H-indol-5-yl [1,4'-bipiperidine]-1'-carboxylate bis(2,2,2-trifluoroacetate) (39b)

**C₄₆H₅₃F₆N₇O₁₀ (*M* = 977.94 g/mol)**

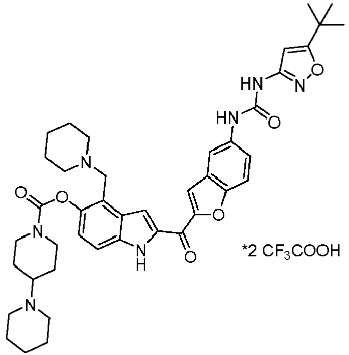

**¹H-NMR** (DMSO-*d₆*, 300 MHz): δ = 12.46 (d, *J* = 0.8 Hz, 1H), 9.77 (s, 1H), 9.65 (s, 1H), 9.58 (s, 1H), 9.27 (s, 1H), 8.17 (d, *J* = 1.9 Hz, 1H), 8.13 (s, 1H), 8.08 (s, 1H), 7.78 (d, *J* = 9.0 Hz, 1H), 7.64 (d, *J* = 8.9 Hz, 1H), 7.54 (dd, *J* = 9.0, 2.2 Hz, 1H), 7.24 (d, *J* = 9.0 Hz, 1H), 6.52 (s, 1H), 4.63 (s, 2H), 4.45 (d, *J* = 10.5 Hz, 1H), 4.19 (d, *J* = 10.5 Hz, 1H), 3.62 - 3.36 (m, 4H), 3.24 - 2.86 (m, 7H), 2.12 (d, *J* = 10.9 Hz, 2H), 1.95 - 1.60 (m, 12 H), 1.51 - 1.35 (m, 2H), 1.31 (s, 9H).

### 2-(5-(3-(5-(tert-Butyl)isoxazol-3-yl)ureido)benzofuran-2-carbonyl)-4-(pyrrolidin-1-ylmethyl)-1H-indol-5-yl [1,4'-bipiperidine]-1'-carboxylate (40a)

**C₄₁H₄₉N₇O₆ (*M* = 735.89 g/mol)**

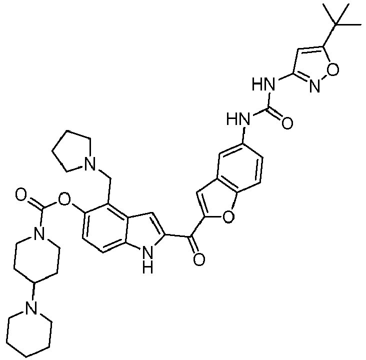

Synthesis of **40a** from **32a** as described in general procedure 5. Yield: 77%.

Additional purification was done by prep. HPLC to achieve a TFA salt of the corresponding amine.

**¹H-NMR** (DMSO-*d₆*, 400 MHz): 12.08 (d, *J* = 1.6 Hz, 1H), 9.58 (s, 1H), 9.04 (s, 1H), 8.12 (d, *J* = 2.0 Hz, 1H), 7.99 (s, 1H), 7.94 (d, *J* = 1.3 Hz, 1H), 7.74 (d, *J* = 9.0 Hz, 1H), 7.51 (dd, *J* = 9.0, 2.1 Hz, 1H), 7.39 (d, *J* = 8.9 Hz, 1H), 7.05 (d, *J* = 8.9 Hz, 1H), 6.53 (s, 1H), 4.26 (br s, 1H), 4.06 (br s, 1H), 3.86 (s, 2H), 3.10 - 2.96 (m, 1H), 2.92 - 2.78 (m, 1H), 2.47 - 2.35 (m, 4H), 1.79 (br s, 2H), 1.71 - 1.62 (m, 4H), 1.54 - 1.34 (m, 9H), 1.30 (s, 9H).

**¹³C-NMR** (DMSO-*d*₆, 101 MHz): δ = 180.2, 173.2, 158.4, 153.2, 152.7, 151.6, 151.3, 143.2, 135.9, 135.3, 134.1, 127.8, 127.2, 124.1, 122.6, 120.9, 114.7, 112.4, 112.1, 111.9, 110.7, 92.5, 61.4, 53.6, 51.0, 49.6, 43.7 + 43.6, 32.5, 28.3, 27.7 + 27.5, 26.0, 24.5, 23.2.

**Mp:** 206 - 208 °C.

### 2-(5-(3-(5-(tert-Butyl)isoxazol-3-yl)ureido)benzofuran-2-carbonyl)-4-(pyrrolidin-1-ylmethyl)-1H-indol-5-yl [1,4'-bipiperidine]-1'-carboxylate bis(2,2,2-trifluoroacetate) (40b)

**C₄₅H₅₁F₆N₇O₁₀ (*M* = 963.92 g/mol)**

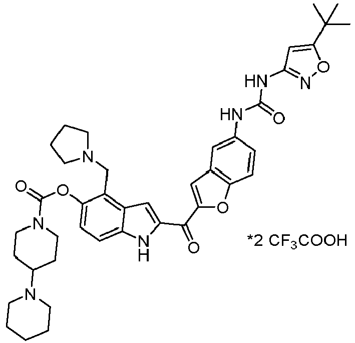

**¹H-NMR** (DMSO-*d₆*, 300 MHz): δ = 12.45 (d, *J* = 1.6 Hz, 1H), 10.13 (s, 1H), 9.86 (s, 1H), 9.77 (s, 1H), 9.42 (s, 1H), 8.21 - 8.09 (m, 3H), 7.78 (d, *J* = 9.1 Hz, 1H), 7.63 (d, *J* = 9.0 Hz, 1H), 7.56 (dd, *J* = 9.0, 2.1 Hz, 1H), 7.24 (d, *J* = 9.0 Hz, 1H), 6.53 (s, 1H), 4.72 (s, 2H), 4.48 (d, *J* = 11.0 Hz, 1H), 4.19 (d, *J* = 13.2 Hz, 1H), 3.39 - 3.35 (m, 2H), 3.34 - 2.87 (m, 7H), 2.21 - 1.61 (m, 14H), 1.52 - 1.37 (m, 1H), 1.31 (s, 9H).

### 2-(5-(3-(5-(tert-Butyl)isoxazol-3-yl)ureido)benzofuran-2-carbonyl)-4-((4-methylpiperazin-i-yl)methyl)-1H-indol-5-yl [1,4'-bipiperidine]-1'-carboxylate (41a)

**C₄₂H₅₂N₈O₆ (*M* = 764.93 g/mol)**

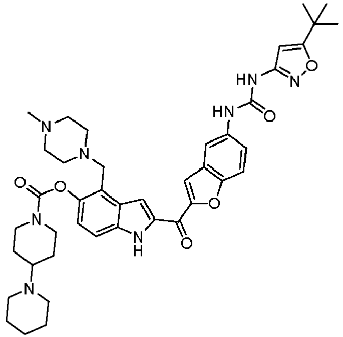

Synthesis of **41a** from **33a** as described in general procedure 5. Yield: 84%.

Additional purification was done by prep. HPLC to achieve a TFA salt of the corresponding amine.

**¹H-NMR** (DMSO-*d₆*, 400 MHz): 12.10 (d, *J* = 1.6 Hz, 1H), 9.64 (s, 1H), 9.16 (s, 1H), 8.12 (d, *J* = 2.0 Hz, 1H), 8.00 (d, *J* = 1.3 Hz, 1H), 7.99 (s, 1H), 7.74 (d, *J* = 9.0 Hz, 1H), 7.53 (dd, *J* = 9.0, 2.2 Hz, 1H), 7.39 (d, *J* = 8.9 Hz, 1H), 7.05 (d, *J* = 8.9 Hz, 1H), 6.53 (s, 1H), 4.28 (d, *J* = 9.1 Hz, 2H), 4.07 (d, *J* = 9.6 Hz, 2H), 3.72 (s, 2H), 3.10 - 2.95 (m, 1H), 2.92 - 2.78 (m, 1H), 2.64 - 2.25 (m, 12H), 2.15 (s, 3H), 1.88 - 1.76 (m, 2H), 1.59 - 1.35 (m, 9H), 1.30 (s, 9H).

**¹³C-NMR** (DMSO-*d*₆, 101 MHz): δ = 180.1, 173.2, 158.4, 153.2, 152.8, 151.6, 151.3, 143.6, 135.9, 135.4, 134.1, 127.8, 127.2, 123.3, 122.6, 120.8, 114.6, 112.3, 112.1, 112.0, 111.0, 92.5, 61.5, 54.8, 54.0, 52.6, 49.6, 45.6, 43.5, 32.4, 28.3, 27.6 + 27.3, 25.7, 24.2.

**Mp:** 212 - 215 °C.

### 2-(5-(3-(5-(tert-Butyl)isoxazol-3-yl)ureido)benzofuran-2-carbonyl)-4-((4-methylpiperazin-1-yl)methyl)-1H-indol-5-yl [1,4'-bipiperidine]-1'-carboxylate tris(2,2,2-trifluoroacetate) (41b)

**C₄₈H₅₅F₉N₈O₁₂ (*M* = 1106.98 g/mol)**

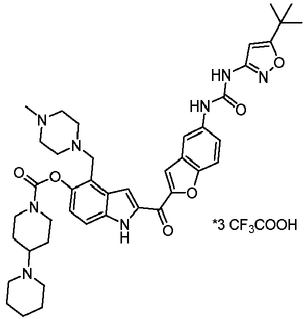

**¹H-NMR** (DMSO-*d₆*, 300 MHz): δ = 12.22 (d, *J* = 1.5 Hz, 1H), 9.76 (s, 1H), 9.76 (br s, 1H), 9.50 (s, 1H), 9.24 (s, 1H), 8.15 (d, *J* = 2.0 Hz, 1H), 8.06 (s, 1H), 7.95 (d, *J* = 1.3 Hz, 1H), 7.77 (d, *J* = 9.0 Hz, 1H), 7.53 (dd, *J* = 9.0, 2.2 Hz, 1H), 7.46 (d, *J* = 8.9 Hz, 1H), 7.11 (d, *J* = 8.9 Hz, 1H), 6.52 (s, 1H), 4.40 (d, *J* = 10.6 Hz, 1H), 4.19 (d, *J* = 10.8 Hz, 1H), 3.89 (s, 2H), 3.55 - 3.29 (m, 6H), 3.16 - 2.84 (m, 8H), 2.76 (s, 3H), 1.99 - 1.59 (m, 8H), 1.50 - 1.38 (m, 1H), 1.30 (s, 9H).

### 2-(5-(3-(5-(tert-Butyl)isoxazol-3-yl)ureido)benzofuran-2-carbonyl)-4-((dimethylamino)methyl)-1H-indol-5-yl [1,4'-bipiperidine]-1'-carboxylate (42a)

**C₃₉H₄₇N₇O₆ (*M*** = **751.89 g/mol)**

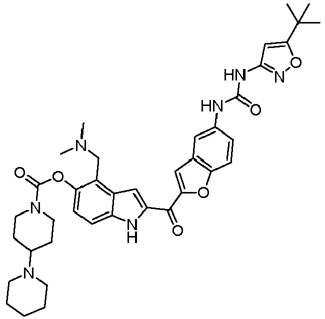

Synthesis of **42a** from **34a** as described in general procedure 5. Yield: 72%.

Additional purification was done by prep. HPLC to achieve a TFA salt of the corresponding amine.

**¹H-NMR** (DMSO-*d₆*, 400 MHz): 12.09 (d, *J* = 1.5 Hz, 1H), 9.59 (s, 1H), 9.03 (s, 1H), 8.13 (d, *J* = 2.0 Hz, 1H), 8.00 (s, 1H), 7.89 (d, *J* = 1.3 Hz, 1H), 7.75 (d, *J* = 9.0 Hz, 1H), 7.51 (dd, *J* = 9.0, 2.1 Hz, 1H), 7.40 (d, *J* = 8.9 Hz, 1H), 7.06 (d, *J* = 8.9 Hz, 1H), 6.53 (s, 1H), 4.27 (d, *J* = 9.5 Hz, 1H), 4.05 (d, *J* = 9.7 Hz, 1H), 3.63 (s, 2H), 3.10 - 2.98 (m, 1H), 2.90 - 2.77 (m, 1H), 2.49 - 2.43 (m, 4H), 2.20 (s, 6H), 1.87 - 1.73 (m, 2H), 1.58 - 1.35 (m, 9H), 1.30 (s, 9H).

**¹³C-NMR** (DMSO-*d*₆, 101 MHz): δ = 180.65, 173.70, 158.91, 153.68, 153.18, 152.09, 151.77, 143.97, 136.30, 135.83, 134.54, 128.39, 127.68, 124.23, 122.96, 121.33, 115.16, 112.93, 112.66, 112.53, 111-30, 92.95, 61.91, 55-47, 50.11, 45.89, 44.09, 40.62, 40.41, 40.20, 39.99, 39.78, 39.58, 39.37, 32.94, 28.83, 28.02, 26.51, 24.97.

**Mp:** 216 - 220 °C.

### 2-(5-(3-(5-(tert-Butyl)isoxazol-3-yl)ureido)benzofuran-2-carbonyl)-4-((dimethylamino)methyl)-1H-indol-5-yl [1,4'-bipiperidine]-1'-carboxylate bis(2,2,2-trifluoroacetate) (42b)

**C₄₃H₄₉F₆N₇O_{1O} (M** = **937.88 g/mol)**

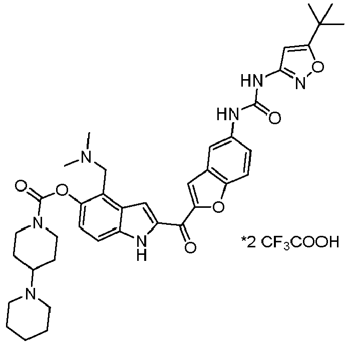

**¹H-NMR** (DMSO-*d*₆, 300 MHz): δ = 12.46 (s, 1H), 9.87 (s, 1H), 9.77 (s, 1H), 9.55 (s, 1H), 9.27 (s, 1H), 8.16 (d, *J* = 2.0 Hz, 1H), 8.14 (s, 1H), 8.10 (s, 1H), 7.78 (d, *J* = 9.0 Hz, 1H), 7.64 (d, *J* = 9.0 Hz, 1H), 7.55 (dd, *J* = 9.0, 2.1 Hz, 1H), 7.24 (d, *J* = 9.0 Hz, 1H), 6.58 (s, 1H), 6.52 (s, 1H), 4.65 (s, 2H), 4.48 (d, *J* = 11.9 Hz, 1H), 4.19 (d, *J* = 12.4 Hz, 1H), 3.20 - 2.94 (m, 6H), 2.89 (s, 6H), 2.11 (d, *J* = 10.2 Hz, 2H), 1.95 - 1.63 (m, 8H), 1.54 - 1.38 (m, 1H), 1.30 (s, 9H).

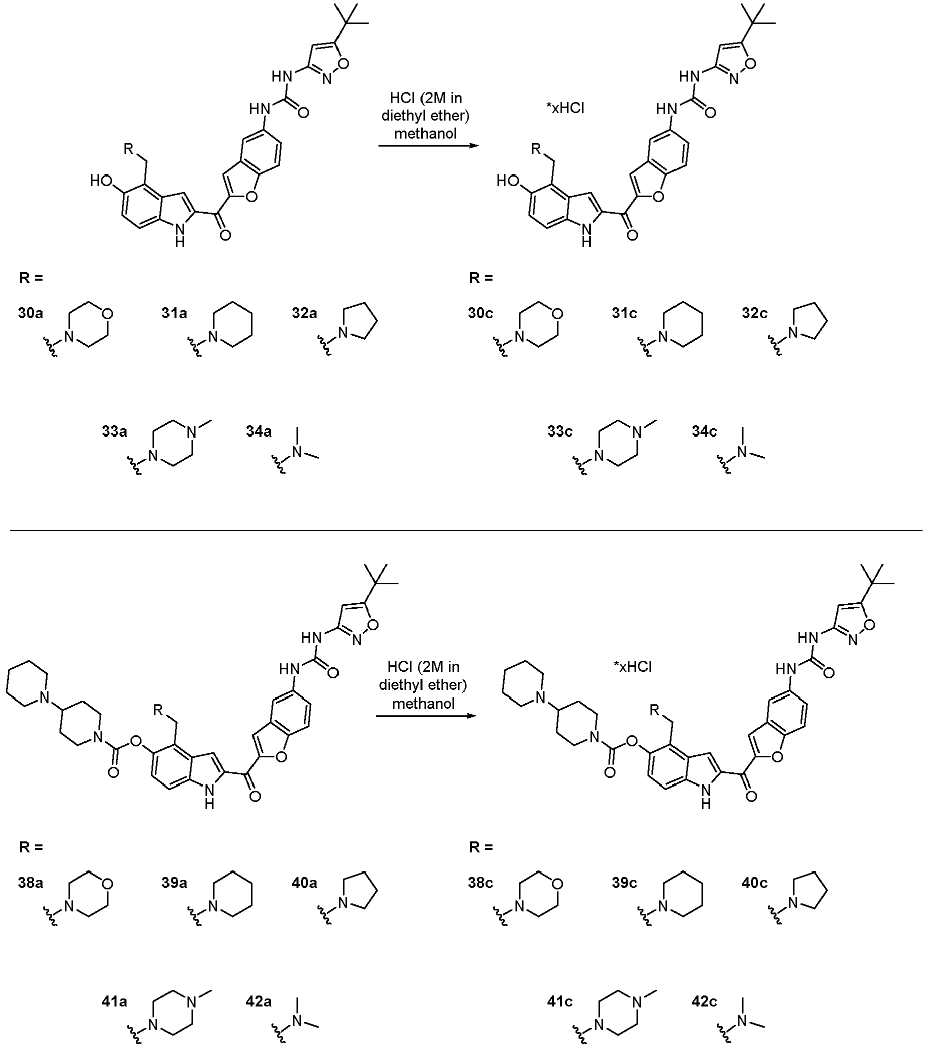

### 1-(5-(tert-Butyl)isoxazol-3-yl)-3-(2-(5-hydroxy-4-(morpholinomethyl)-1H-indole-2-carbonyl)benzofuran-5-yl)urea hydrochloride (30c)

**C₃₀H₃₂ClN₅O₆ (*M* = 594.07 g/mol)**

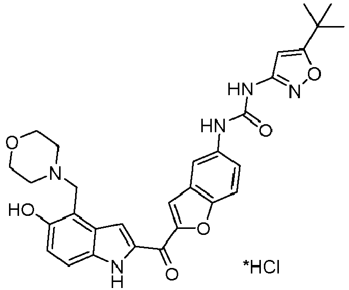

Synthesis of **30c** from **30a** as described in general procedure 6. Yield: quantitative.

**¹H-NMR** (Methanol-*d*₄, 300 MHz): δ = 8.05 (d, *J* = 1.8 Hz, 1H), 7.92 (s, 1H), 7.91 (s, 1H), 7.70 (d, *J* = 8.9 Hz, 1H), 7.53 (dd, *J* = 9.0, 2.5 Hz, 2H), 7.08 (d, *J* = 8.9 Hz, 1H), 6.37 (s, 1H), 4.75 (s, 2H), 4.16 - 3.97 (m, 2H), 3.90 - 3.71 (m, 2H), 3.61 - 3.36 (m, 4H), 1.36 (s, 9H).

**¹H-NMR** (DMSO-*d*₆, 300 MHz): δ = 12.10 (s, 1H), 10.16 (s, 1H), 9.97 (s, 1H), 9.73 (s, 1H), 9.50 (s, 1H), 8.18 (s, 1H), 8.15 (d, *J* = 2.1 Hz, 1H), 7.96 (s, 1H), 7.76 (d, *J* = 8.9 Hz, 1H), 7.51 (dd, J = 8.9, 2.3 Hz, 1H), 7.49 (d, J = 8.6 Hz, 1H), 7.13 (d, *J* = 8.9 Hz, 1H), 6.54 (s, 1H), 4.62 (s, 2H), 3.96 (d, *J* = 13.1 Hz, 2H), 3.77 (t, *J* = 11.8 Hz, 2H), 3.51 - 3.19 (m, 4H), 1.30 (s, 9H).

### 1-(5-(tert-Butyl)isoxazol-3-yl)-3-(2-(5-hydroxy-4-(piperidin-1-ylmethyl)-1H-indole-2-carbonyl)benzofuran-5-yl)urea hydrochloride (31c)

**C₃₁H₃₄ClN₅O₅ (*M*** = **592.09 g/mol)**

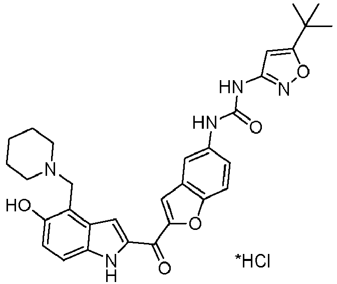

Synthesis of **31c** from **31a** as described in general procedure 6. Yield: quantitative.

**¹H-NMR** (Methanol-*d*₄, 400 MHz): δ = 8.04 (s, 1H), 7.90 (s, 1H), 7.87 (s, 1H), 7.69 (d, *J* = 8.9 Hz, 1H), 7.57 - 7.47 (m, 2H), 7.07 (d, *J* = 8.9 Hz, 1H), 6.37 (s, 1H), 4.66 (s, 2H), 3.60 (d, *J* = 12.0 Hz, 2H), 3.17 (t, *J =* 11.8 Hz, 2H), 2.02 - 1.52 (m, 6H), 1.35 (s, 9H).

**¹H-NMR** (DMSO-*d*₆, 300 MHz): δ = 12.09 (s, 1H), 9.93 (s, 1H), 9.77 (s, 1H), 9.63 (s, 1H), 9.60 (s, 1H), 8.18 (s, 1H), 8.14 (d, *J* = 1.9 Hz, 1H), 7.93 (d, *J* = 0.9 Hz, 1H), 7.76 (d, *J* = 9.0 Hz, 1H), 7.52 (dd, J = 9.1, 2.0 Hz, 1H), 7.48 (d, *J* = 9.4 Hz, 2H), 7.13 (d, *J* = 8.9 Hz, 1H), 6.54 (s, 1H), 4.54 (d, *J* = 3.8 Hz, 2H), 3.53 - 3.45 (m, 2H), 3.13 - 2.95 (m, 2H), 1.85 - 1.60 (m, 5H), 1.46 - 1.35 (m, 1H).

### 1-(5-(tert-Butyl)isoxazol-3-yl)-3-(2-(5-hydroxy-4-(pyrrolidin-1-ylmethyl)-1H-indole-2-carbonyl)benzofuran-5-yl)urea hydrochloride (32c)

**C₃₀H₃₂ClN₅O₅ (*M* = 578.07 g/mol)**

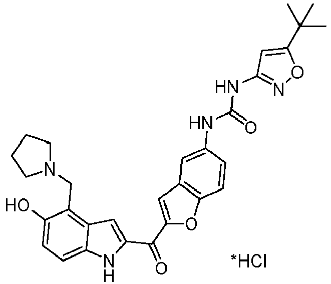

Synthesis of **32c** from **32a** as described in general procedure 6. Yield: quantitative.

**¹H-NMR** (Methanol-*d*₄, 400 MHz): δ = 8.02 (d, *J* = 1.9 Hz, 1H), 7.88 (s, 2H), 7.67 (d, *J* = 9.0 Hz, 1H), 7.54 - 7.45 (m, 2H), 7.06 (d, *J* = 8.9 Hz, 1H), 6.36 (s, 1H), 4.74 (s, 1H), 3.65 - 3.50 (m, 2H), 3.47 - 3.33 (m, 2H), 2.33 - 2.15 (m, 2H), 2.11 - 1.98 (m, 2H), 1.35 (s, 9H).

**¹H-NMR** (DMSO-*d*₆, 300 MHz): δ = 12.09 (d, *J* = 1.4 Hz, 1H), 9.94 (s, 1H), 9.88 (br s, 1H), 9.73 (s, 1H), 9.50 (s, 1H), 8.19 (s, 1H), 8.15 (d, *J* = 1.9 Hz, 1H), 7.94 (d, *J* = 1.0 Hz, 1H), 7.76 (d, *J* = 9.1 Hz, 1H), 7.52 (dd, *J* = 9.0, 2.3 Hz, 1H), 7.47 (d, *J* = 9.0 Hz, 1H), 7.12 (d, *J* = 8.9 Hz, 1H), 6.54 (s, 1H), 4.64 (d, *J* = 4.1 Hz, 2H), 3.57 - 3.40 (m, 2H), 3.29 - 3.15 (m, 2H), 2.13 - 2.00 (m, 2H), 1.97 - 1.87 (m, 2H), 1.31 (s, 9H).

### 1-(5-(tert-Butyl)isoxazol-3-yl)-3-(2-(5-hydroxy-4-((4-methylpiperazin-1-yl)methyl)-1H-indole-2-carbonyl)benzofuran-5-yl)urea dihydrochloride (33c)

**C₃₁H₃₆Cl₂N₆O₅ (*M* = 643.57 g/mol)**

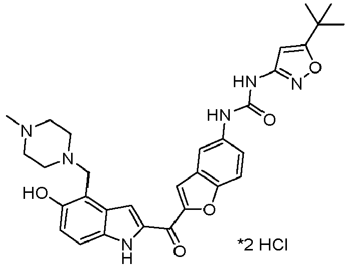

Synthesis of **33c** from **33a** as described in general procedure 6. Yield: quantitative.

**¹H-NMR** (DMSO-*d*₆, 300 MHz): δ = 12.08 (s, 1H), 11.57 (s, 1H), 9.82 (s, 1H), 9.80 (s, 1H), 8.38 (s, 1H), 8.13 (d, *J* = 1.9 Hz, 1H), 7.98 (s, 1H), 7.78 (d, *J* = 8.9 Hz, 1H), 7.51 (dd, *J* = 9.0, 2.1 Hz, 1H), 7.49 (d, *J =* 8.9 Hz, 1H), 7.14 (d, *J* = 9.0 Hz, 1H), 6.55 (s, 1H), 4.63 (s, 2H), 3.92 - 3.61 (m, 4H), 3.59 - 3.46 (m, 4H), 2.80 (s, 3H), 1.30 (s, 9H).

### 1-(5-(tert-Butyl)isoxazol-3-yl)-3-(2-(4-(dimethylamino)methyl)-5-hydroxy-1H-indole-2-carbonyl)benzofuran-5-yl)urea hydrochloride (34c)

**C₂₈H₃₀ClN₅O₅ (*M* = 552.02 g/mol)**

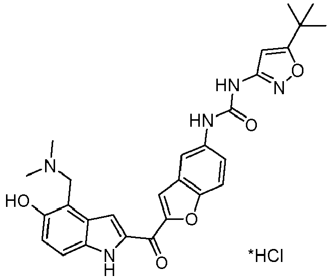

Synthesis of **34c** from **34a** as described in general procedure 6. Yield: quantitative.

**¹H-NMR** (DMSO-*d*₆, 300 MHz): δ = 12.09 (d, *J* = 1.8 Hz, 1H), 10.00 (s, 1H), 9.78 (s, 1H), 9.67 (br s, 1H), 9.64 (s, 1H), 8.17 (s, 1H), 8.13 (d, *J* = 2.0 Hz, 1H), 7.92 (d, *J* = 1.1 Hz, 1H), 7.76 (d, *J* = 9.0 Hz, 1H), 7.53 (dd, *J* = 9.0, 2.1 Hz, 1H), 7.48 (d, *J* = 8.8 Hz, 1H), 7.13 (d, *J* = 8.9 Hz, 1H), 6.54 (s, 1H), 4.58 (s, 2H), 2.83 (s, 6H), 1.30 (s, 9H).

### 2-(5-(3-(5-(tert-Butyl)isoxazol-3-yl)ureido)benzofuran-2-carbonyl)-4-(morpholinomethyl)-1H-indol-5-yl [1,4'-bipiperidine]-1'-carboxylate dihydrochloride (38c)

**C₄₁H₅₁Cl₂N₇O₇ (*M* = 824.80 g/mol)**

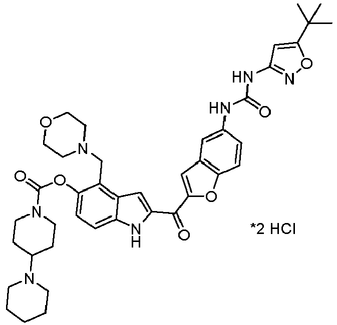

Synthesis of **38c** from **38a** as described in general procedure 6. Yield: quantitative.

**¹H-NMR** (DMSO-*d*₆, 300 MHz): δ = 12.43 (s, 1H), 10.92 (br s, 1H), 10.58 (br s, 1H), 9.77 (s, 1H), 9.62 (s, 1H), 8.34 (s, 1H), 8.21 - 8.12 (m, 2H), 7.78 (d, *J* = 9.0 Hz, 1H), 7.64 (d, *J* = 9.0 Hz, 1H), 7.52 (dd, *J* = 9.0, 2.1 Hz, 1H), 7.24 (d, *J* = 9.0 Hz, 1H), 6.55 (s, 1H), 4.68 (s, 2H), 4.50 (d, *J* = 12.4 Hz, 1H), 4.17 (d, *J* = 11.6 Hz, 1H), 4.04 - 3.79 (m, 4H), 3.78 - 3.57 (m, 2H), 3.34 - 3.06 (m, 4H), 3.04 - 2.85 (m, 3H), 2.25 - 2.13 (m, 2H), 2.00 - 1.65 (m, 9H), 1.50 - 1.38 (m, 1H), 1.30 (s, 9H).

### 2-(5-(3-(5-(tert-Butyl)isoxazol-3-yl)ureido)benzofuran-2-carbonyl)-4-(piperidin-1-ylmethyl)-1H-indol-5-yl [1,4'-bipiperidine]-1'-carboxylate dihydrochloride (39c)

**C₄₂H₅₃Cl₂N₇O₆ (*M* = 822.83 g/mol)**

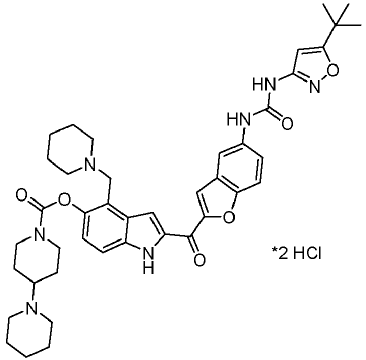

Synthesis of **39c** from **39a** as described in general procedure 6. Yield: quantitative.

**¹H-NMR** (DMSO-*d*₆, 300 MHz): δ = 12.42 (s, 1H), 10.87 (br s, 1H), 10.41 (br s, 1H), 9.89 (s, 1H), 9.85 (s, 1H), 8.35 (s, 1H), 8.15 (s, 2H), 7.77 (d, *J =* 9.0 Hz, 1H), 7.63 (d, *J =* 9.0 Hz, 1H), 7.54 (d, *J* = 8.9 Hz, 1H), 7.23 (d, *J* = 8.9 Hz, 1H), 6.55 (s, 1H), 4.58 (s, 2H), 4.16 (d, *J* = 11.7 Hz, 1H), 3.60 - 3.24 (m, 6H), 3.14 - 2.81 (m, 6H), 2.30 - 2.13 (m, 2H), 2.00 - 1.55 (m, 12H), 1.49 - 1.36 (m, 2H), 1.30 (s, 9H).

### 2-(5-(3-(5-(tert-Butyl)isoxazol-3-yl)ureido)benzofuran-2-carbonyl)-4-(pyrrolidin-1-ylmethyl)-1H-indol-5-yl [1,4'-bipiperidine]-1'-carboxylate dihydrochloride (40c)

**C₄₁H₅₁Cl₂N₇O₆ (*M* = 808.80 g/mol)**

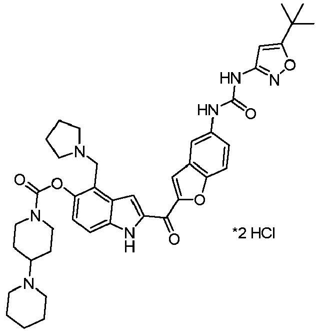

Synthesis of **40c** from **40a** as described in general procedure 6. Yield: quantitative.

**¹H-NMR** (DMSO-*d*₆, 300 MHz): δ = 12.39 (s, 1H), 10.46 (s, 1H), 9.74 (s, 1H), 9.52 (s, 1H), 8.27 (s, 1H), 8.16 (d, *J* = 1.8 Hz, 1H), 8.12 (s, 1H), 7.77 (d, *J* = 9.0 Hz, 1H), 7.59 (d, *J* = 8.6 Hz, 1H), 7.52 (dd, *J* = 9.0, 2.0 Hz, 1H), 7.21 (d, *J* = 8.7 Hz, 1H), 6.54 (s, 1H), 4.51 (s, 2H), 4.48 (d, *J* = 12.5 Hz, 1H), 4.16 (d, *J* = 12.0 Hz, 1H), 3.23 - 2.79 (m, 9H), 2.23 - 1.41 (m, 15H), 1.30 (s, 9H).

### 2-(5-(3-(5-(tert-Butyl)isoxazol-3-yl)ureido)benzofuran-2-carbonyl)-4-((4-methylpiperazin-1-yl)methyl)-1H-indol-5-yl [1,4'-bipiperidine]-1'-carboxylate trihydrochloride (41c)

**C₄₂H₅₅Cl₃N₈O₆ (*M* = 874.30 g/mol)**

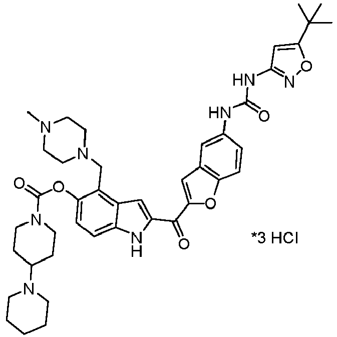

Synthesis of **41c** from **41a** as described in general procedure 6. Yield: quantitative.

**¹H-NMR** (DMSO-*d*₆, 300 MHz): δ = 12.27 (s, 1H), 10.42 (s, 1H), 9.80 (s, 1H), 9.70 (s, 1H), 8.14 (d, *J =* 2.0 Hz, 1H), 7.53 (dd, J= 9.0, 2.0 Hz, 1H), 7.51 - 7.44 (m, 1H), 7.14 (d, *J =* 8.4 Hz, 1H), 6.54 (s, 1H), 4.44 (d, *J* = 13.4 Hz, 1H), 4.17 (d, *J* = 10.2 Hz, 1H), 3.21 - 2.88 (m, 7H), 2.74 (s, 3H), 2.32 - 2.14 (m, 2H), 1.99 - 1.62 (m, 7H), 1.54 - 1.37 (m, 1H), 1.30 (s, 9H).

### 2-(5-(3-(5-(tert-Butyl)isoxazol-3-yl)ureido)benzofuran-2-carbonyl)-4-((dimethylamino)-methyl)-1H-indol-5-yl [1,4'-bipiperidine]-1'-carboxylate dihydrochloride (42c)

**C₃₉H₄₉Cl₂N₇O₆ (M = 782.76 g/mol)**

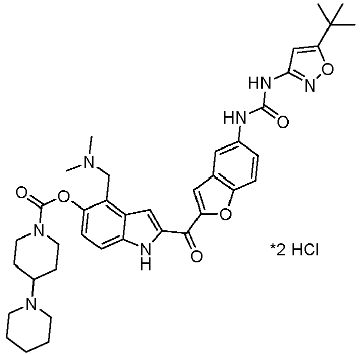

Synthesis of **42c** from **42a** as described in general procedure 6. Yield: quantitative.

**¹H-NMR** (DMSO-*d*₆, 300 MHz): δ = 12.43 (s, 1H), 10.75 (br s, 1H), 10.43 (br s, 1H), 9.82 (s, 1H), 9.78 (s, 1H), 8.43 (s, 1H), 8.15 (d, *J* = 1.9 Hz, 1H), 8.13 (s, 1H), 7.78 (d, *J* = 9.0 Hz, 1H), 7.63 (d, *J* = 9.0 Hz, 1H), 7.54 (dd, *J* = 9.0, 2.0 Hz, 1H), 7.24 (d, *J* = 9.0 Hz, 1H), 6.55 (s, 1H), 4.63 (d, *J* = 4.8 Hz, 2H), 4.52 (d, *J =* 13.1 Hz, 1H), 4.16 (d, *J =* 11.4 Hz, 1H), 3.43 (d, *J =* 9.6 Hz, 3H), 3.18 - 3.06 (m, 1H), 3.02 - 2.91 (m, 2H), 2.86 (s, 6H), 2.30 - 2.13 (m, 2H), 2.00 - 1.62 (m, 8H), 1.50 - 1.36 (m, 1H), 1.30 (s, 9H).

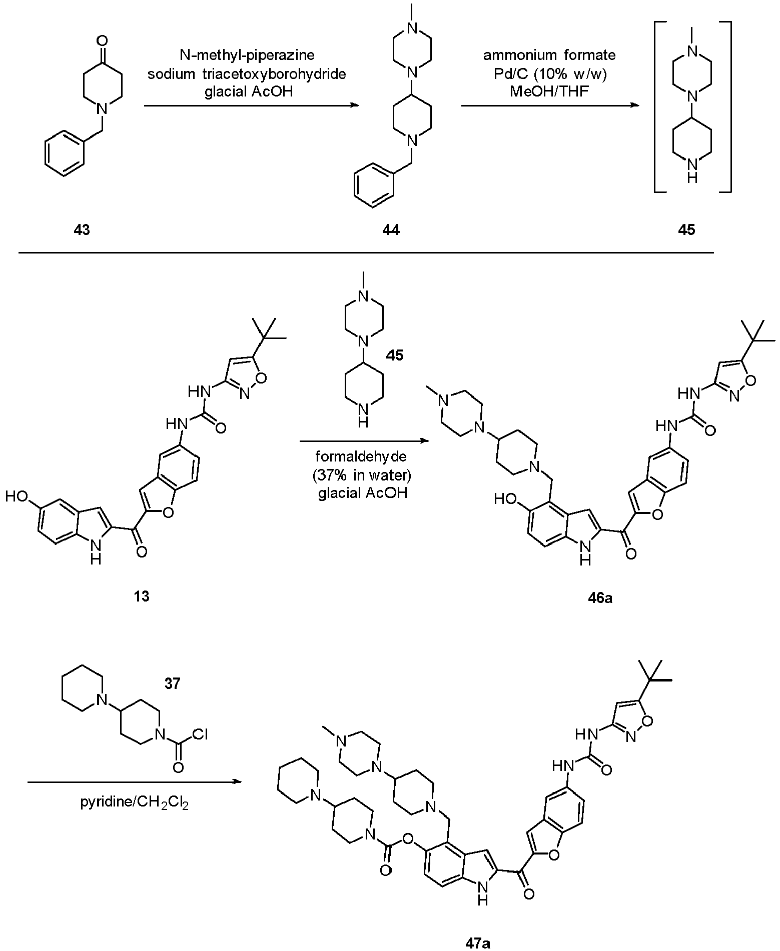

### 1-(1-Benzylpiperidin-4-yl)-4-methylpiperazine (44)

**C₁₇H₂₇N₃ (*M* = 273.42 g/mol)**

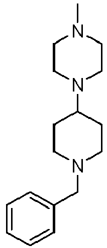

Synthesis from **43** and *N*-Methyl-piperazine. Yield: 99%.

**¹H-NMR** (CDCl₃, 300 MHz): δ = 7.34 - 7.17 (m, 5H), 3.46 (s, 2H), 2.91 (d, *J* = 11.8 Hz, 2H), 2.68 - 2.31 (m, 8H), 2.25 (s, 3H), 2.22 (tt, *J* = 11.5, 3.8 Hz, 1H), 1.93 (td, *J* = 11.9, 2.2 Hz, 2H), 1.76 (d, *J* = 12.5 Hz, 2H), 1.55 (dq, *J* = 12.1, 3.7 Hz, 2H).

**¹H-NMR** (DMSO-*d*₆, 300 MHz): δ = 7.35 - 7.18 (m, 5H), 3.41 (s, 2H), 2.80 (d, *J* = 11.7 Hz, 2H), 2.47 - 2.19 (m, 8H), 2.17 - 2.03 (m, 1H), 2.11 (s, 3H), 1.88 (td, *J* = 11.6, 1.8 Hz, 2H), 1.68 (d, *J* = 11.9 Hz, 2H), 1.36 (qd, *J* = 12.0, 3.7 Hz, 2H).

**¹H-NMR** (Methanol-*d*₄, 300 MHz): δ = 7.38 - 7.20 (m, 5H), 3.48 (s, 2H), 2.93 (d, *J* = 11.8 Hz, 2H), 2.80 - 2.30 (m, 8H), 2.26 (s, 3H), 2.20 (tt, *J* = 11.5, 3.8 Hz, 1H), 1.98 (t, *J* = 12.0 Hz, 2H), 1.84 (d, *J* = 12.3 Hz, 2H), 1.52 (qd, *J* = 12.4, 3.5 Hz, 2H).

**¹³C-NMR** (DMSO-*d*₆, 75 MHz): δ = 138.67, 128.72, 128.13, 126.80, 62.16, 61.19, 55.22, 52.60, 48.56, 45.82, 27.97.

**¹³C-NMR** (Methanol-d₆, 75 MHz): δ = 138.63, 130.65, 129.30, 128.37, 63.88, 62.96, 55.81, 53.84, 49.76, 46.00, 28.85.

### 1-Methyl-4-(piperidin-4-yl)piperazine (45)

**C₁₀H₂₁N₃ (*M* = 183.29 g/mol)**

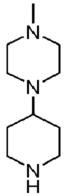

Synthesis of **45** from **44** as described in general procedure 3. Yield: 65%.

**¹H-NMR** (DMSO-*d*₆, 300 MHz): δ = 3.02 (s, 1H), 2.93 (d, *J* = 12.0 Hz, 2H), 2.48 - 2.32 (m, 6H), 2.27 (br s, 3H), 2.22 - 2.14 (m, 1H), 2.12 (s, 3H), 1.65 (d, *J* = 12.1 Hz, 1H), 1.30 - 1.12 (m, 2H).

### 1-(5-(tert-Butyl)isoxazol-3-yl)-3-(2-(5-hydroxy-4-((4-(4-methylpiperazin-1-yl)piperidin-1-yl)methyl)-1H-indole-2-carbonyl)benzofuran-5-yl)urea (46a)

**C₃₆H₄₃N₇O₅ (*M* = 653.33 g/mol)**

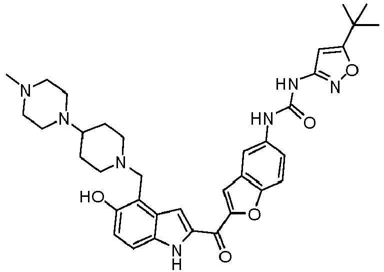

Synthesis of **46a** from **13** and **45** as described in general procedure 4A. Yield: 44%.

Synthesis of **46a** from **13** and **45** as described in general procedure 4B. Yield: 0%.

Additional purification was done by prep. HPLC to achieve a TFA salt of the corresponding amine.

**¹H-NMR** (Methanol-*d*₄, 400 MHz): δ = 7.96 (d, *J* = 1.9 Hz, 1H), 7.75 (s, 1H), 7.67 (s, 1H), 7.63 (d, *J* = 8.9 Hz, 1H), 7.48 (dd, *J* = 8.9, 2.1 Hz, 1H), 7.27 (d, *J* = 8.9 Hz, 1H), 6.87 (d, *J* = 8.9 Hz, 1H), 6.38 (s, 1H), 4.00 (s, 2H), 3.10 (d, *J* = 11.8 Hz, 2H), 2.73 - 2.37 (m, 8H), 2.33 - 2.15 (m, 7H), 1.93 (d, *J* = 11.7 Hz, 2H), 1.61 - 1.49 (m, 2H), 1.35 (s, 9H).

**¹H-NMR** (DMSO-*d*₆, 300 MHz): δ = 11.85 (s, 1H), 10.70 (br s, 1H), 9.60 (s, 1H), 9.01 (s, 1H), 8.09 (d, *J* = 2.0 Hz, 1H), 8.00 (s, 1H), 7.80 (d, *J* = 9.1 Hz, 1H), 7.76 (d, *J* = 1.0 Hz, 1H), 7.51 (dd, *J* = 9.0, 2.3 Hz, 1H), 7.26 (d, *J* = 9.1 Hz, 1H), 6.85 (d, *J* = 8.9 Hz, 1H), 6.53 (s, 1H), 3.99 (s, 2H), 3.01 (d, *J* = 11.1 Hz, 2H), 2.48 - 2.19 (m, 9H), 2.13 (s, 3H), 1.82 (d, *J* = 12.1 Hz, 2H), 1.46 (dd, *J =* 22.7, 11.8 Hz, 2H), 1.30 (s, 9H).

### 1-(5-(tert-Butyl)isoxazol-3-yl)-3-(2-(5-hydroxy-4-((4-(4-methylpiperazin-1-yl)piperidin-1-yl)methyl)-1H-indole-2-carbonyl)benzofuran-5-yl)urea tris(2,2,2-trifluoroacetate) (46b)

**C₄₂H₄₆F₉N₇O₁₁ (*M* = 995.84 g/mol)**

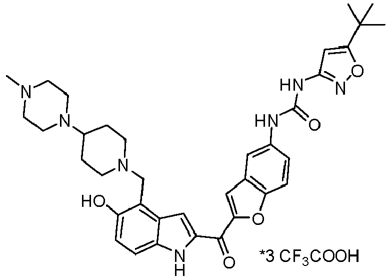

**¹H-NMR** (DMSO-*d*₆, 400 MHz): δ = 12.12 (s, 1H), 9.99 (s, 1H), 9.78 (s, 1H), 9.37 (s, 1H), 9.27 (s, 1H), 8.16 (d, *J* = 2.0 Hz, 1H), 8.07 (d, *J* = 0.5 Hz, 1H), 7.88 (d, *J* = 1.5 Hz, 1H), 7.76 (d, *J* = 9.1 Hz, 1H), 7.57 - 7.46 (m, 2H), 7.11 (d, *J* = 8.9 Hz, 1H), 6.52 (s, 1H), 4.58 (s, 2H), 3.60 (d, *J* = 11.6 Hz, 2H), 3.48 - 3.47 (m, 3H), 3.16 - 2.88 (m, 6H), 2.75 (s, 3H), 2.68 - 2.59 (m, 1H), 2.46 - 2.36 (m, 1H), 1.97 (d, *J* = 11.9 Hz, 2H), 1.69 (dd, *J* = 24.2, 12.6 Hz, 2H), 1.31 (s, 9H).

### 2-(5-(3-(5-(tert-Butyl)isoxazol-3-yl)ureido)benzofuran-2-carbonyl)-4-((4-(4-methyl-piperazin-1-yl)piperidin-1-yl)methyl)-1H-indol-5-yl[1,4'-bipiperidin]-1'-carboxylate (47a)

**C₄₇H₆₁N₉O₆ (*M* = 848.04 g/mol)**

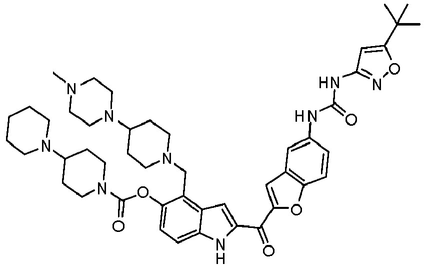

Synthesis of **47a** from **46a** as described in general procedure 5. Yield: 85%.

Additional purification was done by prep. HPLC to achieve a TFA salt of the corresponding amine. **¹H-NMR** (DMSO-*d*₆, 300 MHz): δ = 12.40 (s, 1H), 9.68 (s, 1H), 9.38 (s, 1H), 8.25 (s, 1H), 8.14 (s, 1H), 8.11 (s, 1H), 7.79 (d, *J* = 8.9 Hz, 1H), 7.63 (d, *J* = 8.6 Hz, 1H), 7.54 (d, *J* = 8.3 Hz, 1H), 7.23 (d, *J* = 8.9 Hz, 1H), 6.53 (s, 1H), 4.60 (s, 2H), 4.46 (d, *J* = 10.5 Hz, 1H), 4.16 (d, *J* = 12.1 Hz, 1H), 3.79 - 2.84 (m, 20H), 2.72 (s, 3H), 2.17 (m, 2H), 2.03 - 1.59 (m, 11H), 1.55 - 1.41 (m, 1H), 1.30 (s, 9H).

### 2-(5-(3-(5-(tert-Butyl)isoxazol-3-yl)ureido)benzofuran-2-carbonyl)-4-((4-(4-methylpiperazin-1-yl)piperidin-1-yl)methyl)-1H-indol-5-yl[1,4'-bipiperidin]-1'-carboxylate tetrakis(2,2,2-trifluoroacetate) (47b)

**C₅₅H₆₅F₁₂N₉O₁₄ (*M* = 1304.14 g/mol)**

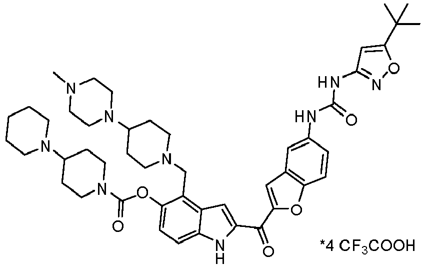

**¹H-NMR** (DMSO-*d*₆, 400 MHz): δ = 12.43 (s, 1H), 10.01 (s, 1H), 9.78 (s, 1H), 9.59 (s, 1H), 9.28 (s, 1H), 8.15 (d, *J* = 2.0 Hz, 1H), 8.11 (s, 1H), 8.06 (d, *J* = 1.3 Hz, 1H), 7.76 (d, *J* = 9.0 Hz, 1H), 7.63 (d, *J* = 9.0 Hz, 1H), 7.54 (dd, *J* = 9.0, 2.1 Hz, 1H), 7.23 (d, *J* = 8.9 Hz, 1H), 6.51 (s, 1H), 4.63 (s, 2H), 4.43 (d, *J* = 12.0 Hz, 1H), 4.18 (d, *J* = 12.3 Hz, 1H), 3.63 (s, 2H), 3.53 - 3.21 (m, 6H), 3.19 - 2.84 (m, 10H), 2.74 (s, 3H), 2.59 (s, 1H), 2.46 - 2.33 (m, 2H), 2.10 (d, *J* = 10.6 Hz, 2H), 1.98 (d, *J* = 12.4 Hz, 2H), 1.87 (d, *J* = 12.4 Hz, 2H), 1.78 - 1.60 (m, 6H), 1.50 - 1.38 (m, 1H), 1.30 (s, 9H).

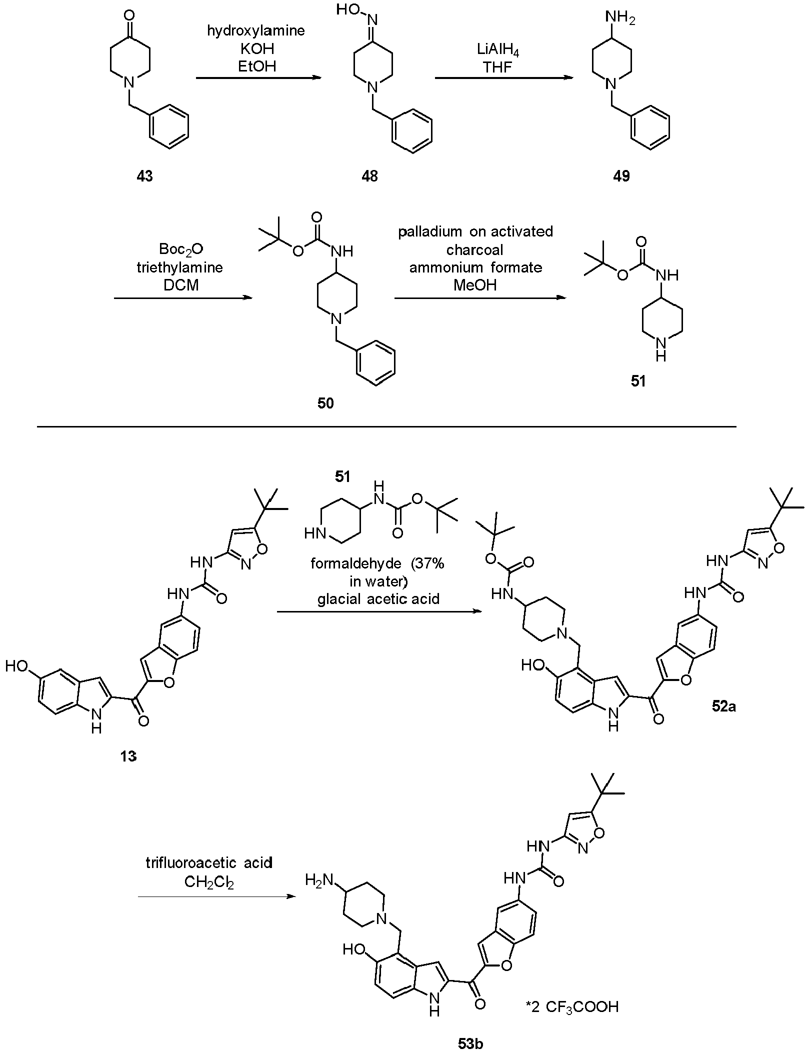

### 1-Benzylpiperidin-4-one oxime (48)

**C₁₂H₁₆N₂O (*M* = 204.27 g/mol)**

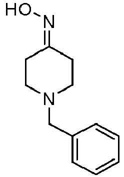

Reaction was performed similar to lit. (Luo et al. 2013). 1-Benzylpiperidin-4-one (**43**, 1.00 eq.) was added to a suspension of hydroxylamine-HCl (2.00 eq.) and potassium carbonate (2.00 eq.) in ethanol (20 mL/g). The reaction mixture was refluxed for 2 hours and the solid was filtered. The solvent was evaporated under reduced pressure and the residue was resolved in dichloromethane and KOH (20% in water). The aqueous layer was extracted with dichloromethane twice. The organic layer was washed with NaCl (sat. in water), dried (Na₂SO₄) and the solvent was evaporated under reduced pressure. Yield: 78%. **¹H-NMR** (CDCl₃, 300 MHz): δ = 7.43 (s, 1H), 7.36 - 7.24 (m, 5H), 3.55 (s, 2H), 2.65 (t, *J* = 5.8 Hz, 2H), 2.60 - 2.49 (m, 4H), 2.35 (t, *J* = 5.8 Hz, 2H).

### 1-Benzylpiperidin-4-amine (49)

**C₁₂H₁₈N₂ (*M*** = **190.29 g/mol)**

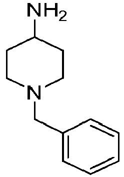

Reaction was performed as follows: A solution of 1-Benzylpiperidin-4-one oxime (**48**, 1.00 eq.) in THF (15 mL/g) was added dropwise to a suspension of lithium aluminum hydride (2.60 eq.) in tetrahydrofuran (15 mL/g) at 0 °C. The reaction mixture was refluxed overnight, and KOH (5% in water, 1 mL/g) was added. The solid was filtered over a pad of celite and the solvent was evaporated under reduced pressure. The residue was resolved in dichloromethane and washed with NaCl (sat. in water). The organic layer was dried (Na₂SO₄) and the solvent was evaporated under reduced pressure. Yield: 96%.

**¹H-NMR** (DMSO-*d*₆, 300 MHz): δ = 7.37 - 7.16 (m, 5H), 3.40 (s, 2H), 2.76 - 2.63 (m, 2H), 2.58 - 2.42 (m, 1H), 1.99 - 1.83 (m, 2H), 1.80 (br s, 2H), 1.69 - 1.58 (m, 2H), 1.31 - 1.12 (m, 2H)*.*

### tert-Butyl (1-benzylpiperidin-4-yl)carbamate (50)

**C₁₇H₂₆N₂O₂ (*M* = 290.41 g/mol)**

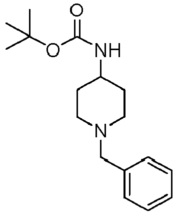

Reaction was performed as follows. A solution of 1-Benzylpiperidin-4-amine (**49**, 1.00 eq.) in dichloromethane (10 mL/g) was added dropwise to a solution of di-*tert*-butyl dicarbonate (1.10 eq.) and triethylamine (1.50 eq.) in dichloromethane (20 mL/g) at 0 °C. The reaction mixture was refluxed overnight and diluted with dichloromethane (40 mL/g). The organic layer was washed with NaHCO₃ (sat. in water) and NaCl (sat. in water), dried (Na₂SO₄) and the solvent was evaporated under reduced pressure. The crude product was purified with cc (dichloromethane: methanol 95:5). Yield: 80%.

**¹H-NMR** (DMSO-*d*₆, 400 MHz): δ = 7.35 - 7.18 (m, 5H), 6.74 (d, *J* = 7.9 Hz, 1H), 3.41 (s, 2H), 3.26 - 3.14 (m, 1H), 2.76 - 2.69 (m, 2H), 1.96 - 1.87 (m, 2H), 1.70 - 1.61 (m, 2H), 1.42 - 1.30 (m, 2H), 1.37 (s, 9H).

### tert-Butyl piperidin-4-ylcarbamate (51)

**C₁₀H₂₀N₂O₂ (*M*** = **200.28 g/mol)**

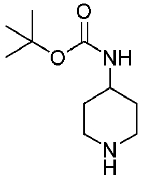

Reaction was performed as follows: To a solution of *tert*-Butyl (1-benzylpiperidin-4-yl)carbamate (**50**, 1.00 eq.) in THF:MeOH (1:1) was added ammonium formate (4.00 eq.) and palladium (5-10% on activated charcoal, 10% w/w). The reaction mixture was stirred at 40 °C for 4 hours. The reaction mixture was filtered over a pad of celite, and the solvent was evaporated under reduced pressure.

**¹H-NMR** (CDCl₃, 400 MHz): δ = 4.66 (s, 1H), 3.44 (s, 1H), 2.97 (dt, *J* = 12.6, 3.3 Hz, 2H), 2.57 (td, *J* = 12.4, 2.4 Hz, 2H), 1.85 (dd, *J* = 12.3, 3.7 Hz, 2H), 1.71 (s, 1H), 1.36 (s, 9H), 1.20 (qd, *J* = 11.5, 4.0 Hz, 2H).

### tert-Butyl (1-((2-(5-(4-(5-(tert-butyl)isoxazol-3-yl)ureido)benzofuran-2-carbonyl)-5-hydroxy-1H-indol-3-yl)methyl)piperidin-4-yl)carbamate (52a)

**C₃₆H₄₂N₆O₇ (*M*** = **670.77 g/mol)**

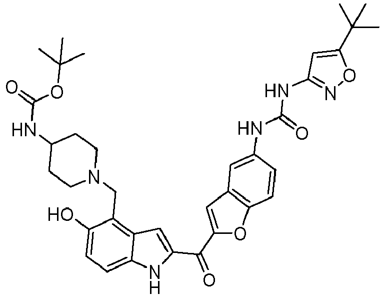

Synthesis of **52**a from **13** and **51** as described in general procedure 4A. Yield: 27%.

Synthesis of **52**a from **13** and **51** as described in general procedure 4B. Yield: 0%.

**¹H-NMR** (DMSO-*d*₆, 300 MHz): δ = 11.88 (s, 1H), 10.71 (br s, 1H), 9.63 (s, 1H), 9.06 (s, 1H), 8.10 (d, *J* = 2.2 Hz, 1H), 8.01 (s, 1H), 7.77 (d, *J* = 9.0 Hz, 1H), 7.71 (s, 1H), 7.51 (dd, *J* = 9.0, 2.2 Hz, 1H), 7.28 (d, *J* = 8.8 Hz, 1H), 6.87 (d, *J* = 8.8 Hz, 1H), 6.53 (s, 1H), 4.04 (s, 2H), 3.69 - 3.48 (m, 1H), 2.98 (d, *J* = 11.3 Hz, 2H), 2.39 - 2.21 (m, 2H), 1.84 - 1.72 (m, 2H), 1.54 - 1.41 (m, 2H), 1.37 (s, 9H), 1.30 (s, 9H).

### 1-(2-(3-((4-Aminopiperidin-1-yl)methyl)-5-hydroxy-1H-indole-2-carbonyl)benzofuran-5-yl)-4-(5-(tert-butyl)isoxazol-3-yl)urea bis(2,2,2-trifluoroacetate) (53b)

**C₃₅H₃₆F₆N₆O₉ (*M*** = **798.70 g/mol)**

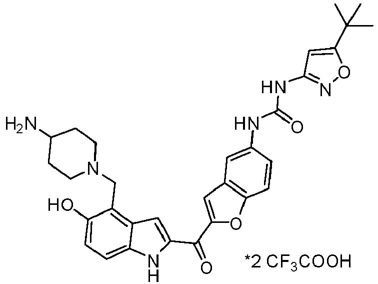

Synthesis of **53b** from **52a** described in general procedure 7. Yield: quantitative.

**¹H-NMR** (DMSO-*d*₆, 300 MHz): δ = 12.09 (s, 1H), 10.00 (br s, 1H), 9.81 (s, 1H), 9.67 (s, 1H), 9.31 (s, 1H), 8.23 - 8.09 (m, 2H), 8.06 (s, 1H), 7.85 (d, *J* = 2.1 Hz, 1H), 7.76 (d, *J* = 9.0 Hz, 1H), 7.54 (dd, *J* = 9.0, 2.3 Hz, 1H), 7.50 (d, *J* = 8.8 Hz, 1H), 7.12 (d, *J* = 8.9 Hz, 1H), 6.52 (s, 1H), 4.57 (s, 2H), 3.66 - 3.58 (m, 2H), 3.33 - 3.29 (m, 1H), 3.25 - 3.13 (m, 2H), 2.13 - 2.05 (m, 2H), 1.87 - 1.72 (m, 2H), 1.31 (s, 9H).

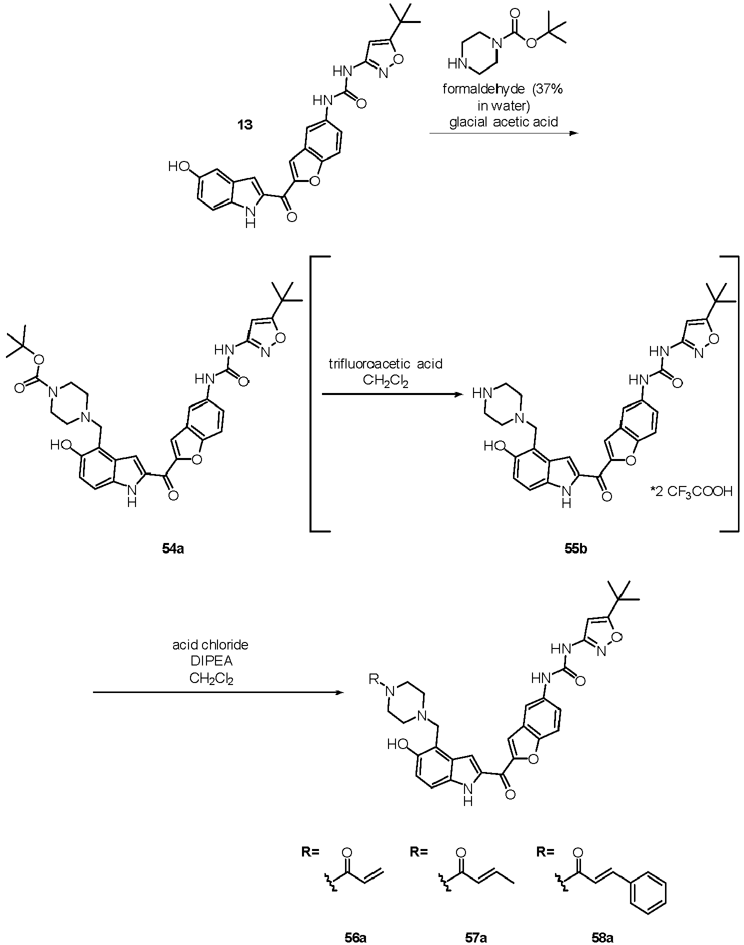

### tert-Butyl-4-((2-(5-(4-(5-(tert-butyl)isoxazol-3-yl)ureido)benzofuran-2-carbonyl)-5-hydroxy-1H-indol-3-yl)methyl)piperazine-1-carboxylate (54a)

**C₃₅H_{4O}N₆O₇ (*M* = 656.74 g/mol)**

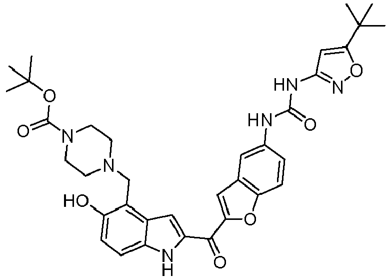

Synthesis of **54**a from **13** and 1-*boc*-piperazine as described in general procedure 4A. Yield: 27%. Synthesis of **54**a from **13** and 1-*boc*-piperazine as described in general procedure 4B. Yield: 59%.

**¹H-NMR** (DMSO-*d*₆, 400 MHz): δ = 11.83 (d, *J* = 1.8 Hz, 1H), 9.91 (br s, 1H), 9.55 (s, 1H), 8.97 (s, 1H), 8.09 (d, *J* = 2.1 Hz, 1H), 7.97 (s, 1H), 7.74 (s, 1H), 7.73 (d, *J* = 12.1 Hz, 1H), 7.49 (dd, *J* = 9.0, 2.2 Hz, 1H), 7.27 (d, *J* = 8.9 Hz, 1H), 6.89 (d, *J* = 8.8 Hz, 1H), 6.51 (s, 1H), 3.97 (s, 2H), 3.37 (s, 4H), 2.52 (s, 4H), 1.38 (s, 9H), 1.29 (s, 9H).

**¹³C-NMR** (DMSO-*d*₆, 75 MHz): δ = 180.65, 173.50, 158.89, 153.20, 152.08, 151.71, 151.35, 135.72, 134.05, 133.56, 127.97, 127.74, 121.13, 118.60, 114.91, 112.98, 112.68, 112.46, 111.94, 109.08, 92.93, 56.41, 55.22, 52.71, 46.10, 32.94, 28.82.

### 1-(5-(tert-Butyl)isoxazol-3-yl)-3-(2-(5-hydroxy-4-(piperazin-1-ylmethyl)-1H-indole-2-carbonyl)benzofuran-5-yl)urea bis(2,2,2-trifluoroacetate) (55b)

**C₃₄H₃₄F₆N₆O₉ (*M* = 784.66 g/mol)**

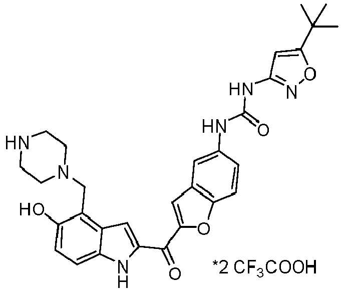

Synthesis of **55b** from **54a** described in general procedure 7. A small amount of the crude product was purified for analysis. For the synthesis of **56a**, **57a** and **58a** the crude product was used without further purification.

**¹H-NMR** (DMSO-*d*₆, 300 MHz): δ = 11.87 (s, 1H), 9.84 (s, 1H), 9.75 (s, 1H), 9.32 (br s, 2H), 8.11 (d, J = 1.9 Hz, 1H), 8.00 (s, 1H), 7.82 - 7.74 (m, 2H), 7.54 (dd, J = 9.0, 2.0 Hz, 1H), 7.29 (d, J = 8.8 Hz, 1H), 6.96 (d, J = 8.8 Hz, 1H), 6.54 (s, 1H), 3.97 (s, 2H), 3.12 - 3.02 (m, 4H), 2.78 - 2.63 (m, 4H), 1.30 (s, 9H).

### 1-(2-(4-((4-Acryloylpiperazin-1-yl)methyl)-5-hydroxy-1H-indole-2-carbonyl)benzofuran-5-yl)-3-(5-(tert-butyl)isoxazole-3-yl)urea (56a)

**C₃₃H₃₄N₆O₆ (*M*** = **610.66 g/mol)**

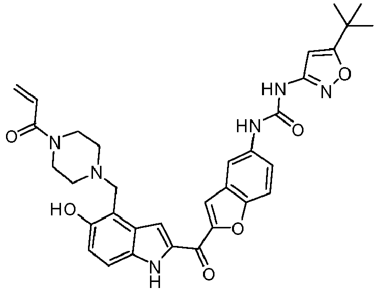

Synthesis of **56a** in two steps from **54**a and acryl acid chloride as described in general procedures 7 and 8. No additional purification was done between both steps. Yield: 32%.

Additional purification was done by prep. HPLC to achieve a TFA salt of the corresponding amine.

**¹H-NMR** (DMSO-*d*₆, 300 MHz): δ = 11.89 (s, 1H), 9.97 (br s, 1H), 9.75 (s, 1H), 9.59 (s, 1H), 9.03 (s, 1H), 8.10 (d, J = 1.8 Hz, 1H), 8.00 (s, 1H), 7.77 (s, 1H), 7.74 (d, J = 9.3 Hz, 1H), 7.50 (dd, J = 9.0, 2.0 Hz, 1H), 7.30 (d, J = 8.5 Hz, 1H), 6.92 (d, J = 8.8 Hz, 1H), 6.80 (dd, *J* = 16.7, 10.5 Hz, 1H), 6.52 (s, 1H), 6.11 (dd, *J* = 16.7, 2.2 Hz, 1H), 5.68 (dd, *J* = 10.3, 2.3 Hz, 1H), 4.02 (s, 2H), 3.73 - 3.53 (m, 4H), 2.72 - 2.55 (m, 4H), 1.30 (s, 9H).

### 1-(2-(4-((4-Acryloylpiperazin-1-yl)methyl)-5-hydroxy-1H-indole-2-carbonyl)benzofuran-5-yl)-3-(5-(tert-butyl)isoxazole-3-yl)urea 2,2,2-trifluoroacetate (56b)

**C₃₅H₃₅F₃N₆O₈ (*M* = 724.69 g/mol)**

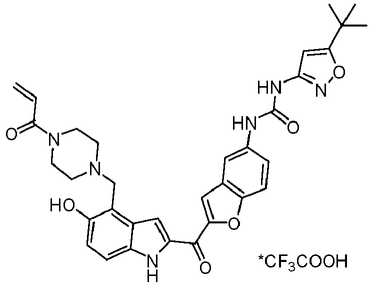

**¹H-NMR** (Methanol-*d*₄, 300 MHz): δ = 8.00 (s, 1H), 7.87 (s, 1H), 7.86 (s, 1H), 7.65 (d, *J* = 8.9 Hz, 1H), 7.50 (d, *J* = 8.9 Hz, 2H), 7.07 (d, *J* = 8.9 Hz, 1H), 6.77 (dd, *J* = 16.7, 10.6 Hz, 1H), 6.34 (s, 1H), 6.27 (dd, *J* = 16.8, 1.6 Hz, 1H), 5.81 (dd, *J* = 10.6, 1.6 Hz, 1H), 4.76 (s, 2H), 3.54 (s, 8H), 1.35 (s, 9H). **¹⁹F-NMR** (Methanol-*d*₄, 377 MHz): δ = -75.32.

### 1-(2-(4-((4-(But-2-enoyl)piperazin-1-yl)methyl)-5-hydroxy-1H-indole-2-carbonyl)benzofuran-5-yl)-3-(5-(tert-butyl)isoxazole-3-yl)urea (57a)

**C₃₄H₃₆N₆O₆ (*M* = 624.69 g/mol)**

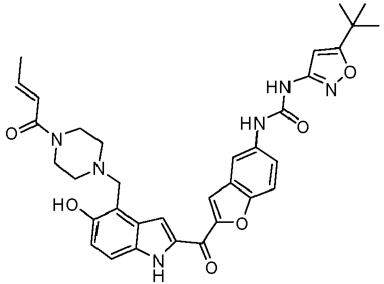

Synthesis of **57a** in two steps from **54a** and acryl acid chloride as described in general procedures 7 and 8. No additional purification was done between both steps. Yield: 28%.

Additional purification was done by prep. HPLC to achieve a TFA salt of the corresponding amine.

**¹H-NMR** (DMSO-*d*₆, 300 MHz): δ = 11.87 (d, *J* = 1.8 Hz, 1H), 9.97 (br s, 1H), 9.60 (s, 1H), 9.01 (s, 1H), 8.10 (d, J = 2.0 Hz, 1H), 7.99 (s, 1H), 7.78 - 7.71 (m, 2H), 7.50 (dd, J = 9.0, 2.2 Hz, 1H), 7.28 (d, J = 8.9 Hz, 1H), 6.91 (d, J = 8.8 Hz, 1H), 6.68 (dq, *J* = 13.4, 6.6 Hz, 1H), 6.55 - 6.44 (m, 2H), 3.99 (s, 2H), 3.68 - 3.47 (m, 4H), 2.61 - 2.49 (m, 4H), 1.82 (dd, *J* = 6.6, 0.9 Hz, 3H), 1.30 (s, 9H).

### 1-(2-(4-((4-(But-2-enoyl)piperazin-1-yl)methyl)-5-hydroxy-1H-indole-2-carbonyl)benzofuran-5-yl)-3-(5-(tert-butyl)isoxazole-3-yl)urea 2,2,2-trifluoroacetate (57b)

**C₃₆H₃₇F₃N₆O₈ (*M*** = **738.72 g/mol)**

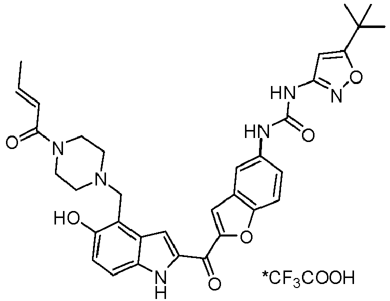

**¹H-NMR** (Methanol-*d*₄, 400 MHz): δ = 7.98 (d, *J* = 1.5 Hz, 1H), 7.85 (s, 1H), 7.84 (s, 1H), 7.64 (d, J = 8.9 Hz, 1H), 7.50 (dd, J = 8.9, 2.9 Hz, 2H), 7.06 (d, J = 8.9 Hz, 1H), 6.88 (dq, *J* = 13.7, 6.8 Hz, 1H), 6.46 (dd, *J* = 15.0, 1.6 Hz, 1H), 6.33 (s, 1H), 4.74 (s, 2H), 3.68 - 3.47 (m, 4H), 3.79 - 3.32 (m, 8H), 1.88 (dd, *J* = 6.8, 1.4 Hz, 3H), 1.34 (s, 9H).

### 1-(5-(tert-Butyl)isoxazol-3-yl)-3-(2-(4-((4-cinnamoylpiperazin-1-yl)methyl)-5-hydroxy-1H-indole-2-carbonyl)benzofuran-5-yl)urea (58a)

**C₃₉H₃₈N₆O₆ (*M* = 686.76 g/mol)**

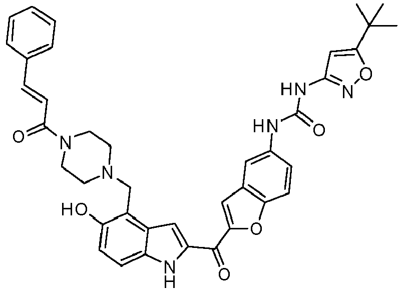

Synthesis of **58a** in two steps from **54**a and acryl acid chloride as described in general procedures 7 and 8. No additional purification was done between both steps. Yield: 35%.

Additional purification was done by prep. HPLC to achieve a TFA salt of the corresponding amine.

**¹H-NMR** (DMSO-*d*₆, 300 MHz): δ = 11.87 (d, *J* = 1.7 Hz, 1H), 9.57 (s, 1H), 8.99 (s, 1H), 8.09 (d, J = 2.1 Hz, 1H), 7.99 (d, *J* = 0.6 Hz, 1H), 7.77 (d, *J* = 1.5 Hz, 1H), 7.74 (d, *J* = 9.0 Hz, 1H), 7.71 (d, *J* = 2.2 Hz, 1H), 7.69 (d, *J* = 1.5 Hz, 1H), 7.49 (d, J = 15.3 Hz, 1H), 7.43 - 7.34 (m, 3H), 7.30 (d, J = 7.2 Hz, 1H), 7.26 (d, *J* = 13.9 Hz, 1H), 6.92 (d, J = 8.8 Hz, 1H), 6.51 (s, 1H), 4.02 (s, 2H), 3.84 - 3.71 (m, 2H), 3.69 - 3.58 (m, 4H), 2.70 - 2.55 (m, 4H), 2.61 - 2.49 (m, 4H), 1.82 (dd, *J* = 6.6, 0.9 Hz, 3H), 1.29 (s, 9H).

### 1-(5-(tert-Butyl)isoxazol-3-yl)-3-(2-(4-((4-cinnamoylpiperazin-1-yl)methyl)-5-hydroxy-1H-indole-2-carbonyl)benzofuran-5-yl)urea 2,2,2-trifluoroacetate (58b)

**C₄₁H₃₉F₃N₆O₈ (*M* = 800.71 g/mol)**

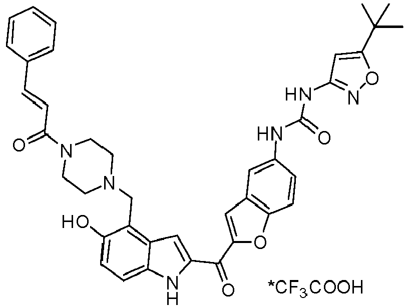

**¹H-NMR** (DMSO-*d*₆, 300 MHz): δ = 12.11 (s, 1H), 10.14 - 9.62 (m, 3H), 9.25 (s, 1H), 8.13 (d, *J* = 1.8 Hz, 1H), 8.07 (s, 1H), 7.91 (s, 1H), 7.78 - 7.68 (m, 3H), 7.57 - 7.47 (m, 3H), 7.44 - 7.35 (m, 3H), 7.29 (d, *J* = 15.4 Hz, 1H), 7.12 (d, *J* = 8.9 Hz, 1H), 6.51 (s, 1H), 4.63 (s, 2H), 3.79 - 3.44 (m, 4H), 3.30 - 2.94 (m, 4H), 1.30 (s, 9H).

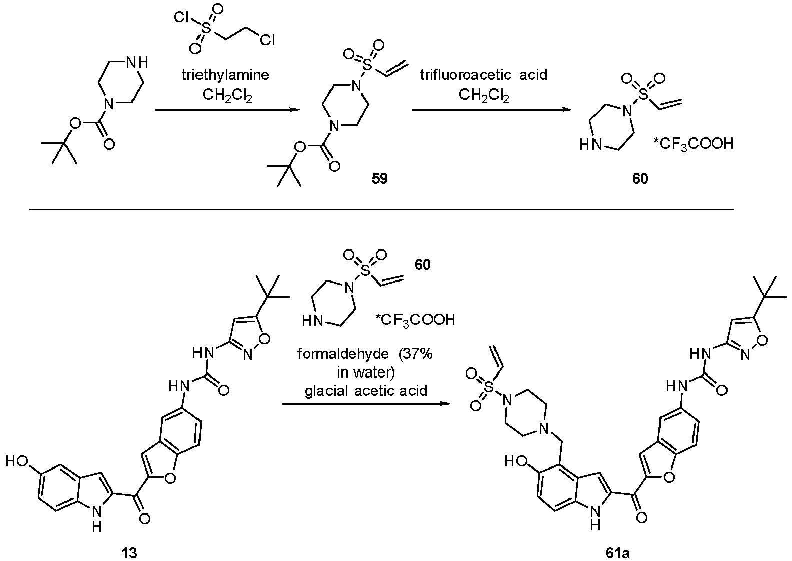

### tert-Butyl 4-(vinylsulfonyl)piperazine-1-carboxylate (59)

**C₁₁H₂₀N₂O₄S (*M* = 276.35 g/mol)**

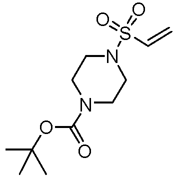

Reaction was performed similar to lit. (Li et al. 2016). A solution of 1-*boc*-piperazine and triethylamine (1.50 eq.) in dichloromethane (15 mL/g) was cooled to 0 °C and 2-Chloroethane-1-sulfonyl chloride (1.10 eq.) was added. The reaction mixture was stirred at room temperature for 1 hour before a second portion of triethylamine (1.50 eq.) was added. After the reaction was finished, water was added, and the organic layer was separated. The organic layer was washed successively with KHSO₄ (1M in water), water, NaHCO₃ (5% in water) and again water. The organic layer was dried (Na₂SO₄) and the solvent was removed under reduced pressure. The crude product was used without further purification. Yield: 47%.

### 4-(vinylsulfonyl)piperazine-1-carboxylate 2,2,2-trifluoroacetate (60)

**C₈H₁₃F₃N₂O₄S (*M*** = **290.26 g/mol)**

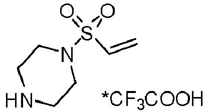

Synthesis of **60** from **59** as described in general procedure 7. Yield: quantitative.

**¹H-NMR** (DMSO-*d*₆, 300 MHz): δ = 9.02 (s, 2H), 6.92 (dd, *J* = 16.5, 10.0 Hz, 1H), 6.27 (d, *J* = 10.0 Hz, 1H), 6.18 (d, *J* = 16.5, 1H), 3.29 - 3.15 (m, 8H).

### 1-(5-(tert-Butyl)isoxazol-3-yl)-3-(2-(5-hydroxy-4-((4-(vinylsulfonyl)piperazin-1-yl)methyl)-1H-indole-2-carbonyl)benzofuran-5-yl)urea (61a)

**C₃₂H₃₄N₆O₇S (*M* = 646.72 g/mol)**

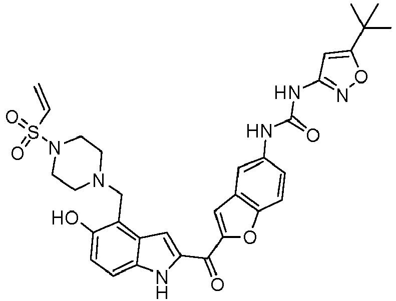

Synthesis of **61a** from **13** and **60** as described in general procedure 4A. Yield: 46%.

**¹H-NMR** (DMSO-*d*₆, 300 MHz): δ = 11.86 (s, 1H), 9.70 (br s, 1H), 9.59 (s, 1H), 9.01 (s, 1H), 8.11 (d, *J* = 2.2 Hz, 1H), 7.99 (s, 1H), 7.82 - 7.70 (m, 2H), 7.51 (dd, *J* = 9.0, 2.3 Hz, 1H), 7.28 (d, *J* = 8.8 Hz, 1H), 6.90 (d, *J* = 8.9 Hz, 1H), 6.84 (dd, *J* = 16.5, 10.5 Hz, 1H), 6.53 (s, 1H), 6.16 (d, *J* = 10.0 Hz, 1H), 6.11 (d, *J* = 16.5 Hz, 1H), 4.00 (s, 2H), 3.17 - 3.02 (m, 4H), 2.71 - 2.57 (m, 4H), 1.30 (s, 9H).

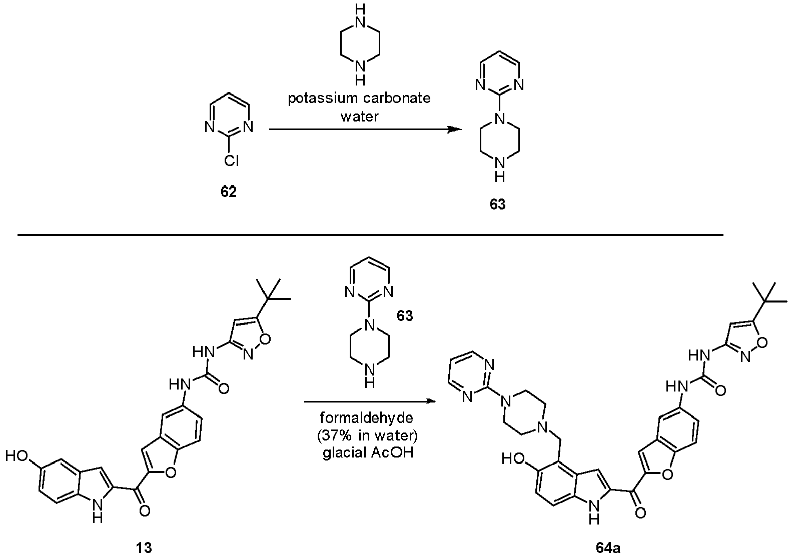

### 2-(Piperazin-1-yl)pyrimidine (63)

**C₈H₁₂N₄ (*M* = 164.21 g/mol)**

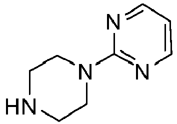

Reaction was performed as described in the following. A solution of piperazine (2.50 eq.) in water (5 mL/g) was heated to 60 °C. At this temperature 2-chloropyrimidin was added portion wise over a period of 15 min. The reaction mixture was stirred at 60 °C overnight. After completion of the reaction the reaction mixture was filtered and diluted with NaHCO₃ (sat. in water). The product was extracted with chloroform three times, the organic layer dried (Na₂SO₄) and the solvent removed under reduced pressure. Yield: 82%.

**¹H-NMR** (DMSO-*d*₆, 300 MHz): δ = 8.31 (d, *J* = 4.7 Hz, 2H), 6.54 (t, *J* = 4.7 Hz, 1H), 3.67 - 3.60 (m, 4H), 2.75 - 2.66 (m, 4H), 2.40 (br s, 1H).

### 1-(5-(tert-Butyl)isoxazol-3-yl)-3-(2-(5-hydroxy-4-((4-(pyrimidin-2-yl)piperazin-1-yl)methyl)-1H-indole-2-carbonyl)benzofuran-5-yl)urea (64a)

**C₃₄H₃₄N₈O₅ (*M* = 634.70 g/mol)**

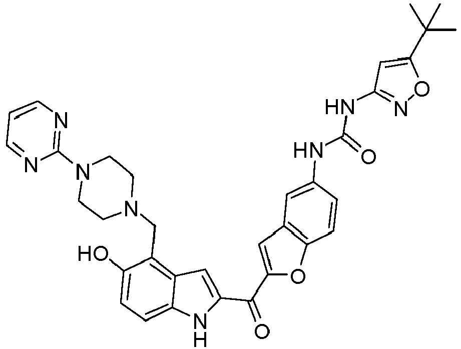

Synthesis of **64a** from **13** and **63** as described in general procedure 4A. Yield: 29%.

Synthesis of **64a** from **13** and **63** as described in general procedure 4B. Yield: 63%.

**¹H-NMR** (DMSO-*d*₆, 300 MHz): δ = 11.87 (d, *J* = 2.3 Hz, 1H), 10.12 (br s, 1H), 9.57 (s, 1H), 8.99 (s, 1H), 8.36 (d, *J* = 4.7 Hz, 2H), 8.08 (d, *J* = 2.2 Hz, 1H), 7.99 (s, 1H), 7.77 (d, *J* = 2.1 Hz, 1H), 7.73 (d, *J* = 9.0 Hz, 1H), 7.48 (dd, *J* = 9.0, 2.2 Hz, 1H), 7.29 (d, *J* = 8.8 Hz, 1H), 6.91 (d, *J* = 8.8 Hz, 1H), 6.63 (t, *J* = 4.7 Hz, 1H), 6.52 (s, 1H), 4.04 (s, 2H), 3.85 - 3.74 (m, 4H), 2.68 - 2.58 (m, 4H), 1.30 (s, 9H).

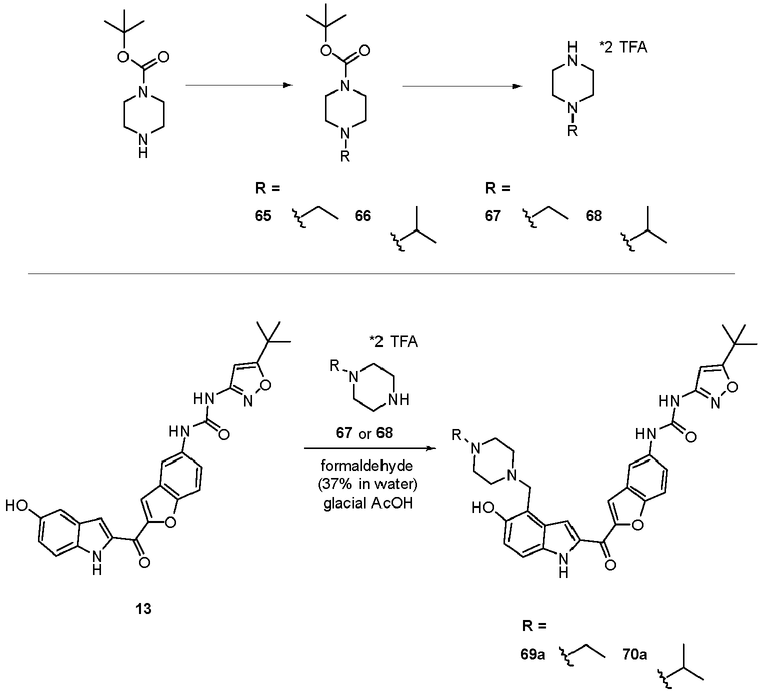

### tert-Butyl 4-ethylpiperazine-1-carboxylate (65)

**C₁₁H₂₂N₂O₂ (*M* = 214.31 g/mol)**

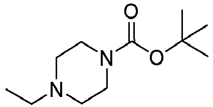

Reaction was performed as follows: A solution of 1-*boc*-piperazine in acetonitrile (20 mL/g) was cooled to 0 °C. Iodoethane (1.10 eq.) and DIPEA (1.10 eq.) were added, and the reaction mixture was stirred at room temperature overnight. After completion of the reaction ethyl acetate was added, and the organic layer was washed successively with water twice and with NaCl (sat. in water). The organic layer was dried (Na₂SO₄), and the solvent was removed under reduced pressure. The crude product was purified with cc (dichloromethane: ammonia (7M in methanol) 99:1).

**¹H-NMR** (DMSO-*d*₆, 300 MHz): δ = 3.28 (t, *J* = 5.1 Hz, 4H), 2.30 (q, *J* = 7.2 Hz, 2H), 2.27 (t, *J* = 5.1 Hz, 4H), 1.38 (s, 9H), 0.98 (t, *J* = 7.2 Hz, 3H).

### tert-Butyl 4-isopropylpiperazine-1-carboxylate (66)

**C₁₂H₂₄N₂O₂ (*M* = 228.34 g/mol)**

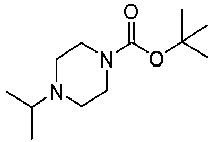

Reaction was performed similar to lit. (Baxter and Reitz 2004). To a solution of 1-boc-piperazine in a mixture of methanol and THF (1:4, 20 mL/g) was added acetone (0.8 mL/g) and glacial acetic acid (0.7 mL/g). Sodium cyanoborohydride (2.00 eq.) was added portion wise and the reaction mixture was stirred at room temperature overnight. After completion of the reaction ethyl acetate was added, and the organic layer was washed successively with water twice and with NaCl (sat. in water). The organic layer was dried (Na₂SO₄), and the solvent was removed under reduced pressure. The crude product was purified with cc (dichloromethane: ammonia (7M in methanol) 99:1).

**¹H-NMR** (DMSO-*d*₆, 300 MHz): δ = 3.27 (t, *J* = 5.1 Hz, 4H), 2.64 (p, *J* = 6.6 Hz, 1H), 2.35 (t, *J* = 5.1 Hz, 4H), 1.38 (s, 9H), 0.95 (d, *J* = 6.6 Hz, 6H).

### 1-Ethylpiperazine bis(2,2,2-trifluoroacetate) (67)

**C₁₀H₁₆F₆N₂O₄ (*M* = 342.24 g/mol)**

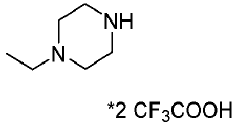

Synthesis of **67** from **65** as described in general procedure 7. The crude product was used without further purification. A small amount was purified for analytics. Yield: quantitative.

**¹H-NMR** (DMSO-*d*₆, 300 MHz): δ = 9.85 (br s, 3H), 3.42 (br s, 8H), 3.19 (q, *J* = 7.3 Hz, 2H), 1.21 (t, *J* = 7.1 Hz, 3H).

### 1-Isopropylpiperazine bis(2,2,2-trifluoroacetate) (68)

**C₁₁H₁₈F₆N₂O₄ (*M*** = **356.27 g/mol)**

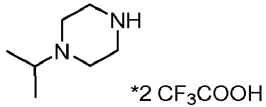

Synthesis of **66** from **68** as described in general procedure 7. The crude product was used without further purification. Yield: quantitative.

### 1-(5-(tert-Butyl)isoxazol-3-yl)-3-(2-(4-((4-ethylpiperazin-1-yl)methyl)-5-hydroxy-1H-indole-2-carbonyl)benzofuran-5-yl)urea (69a)

**C₃₂H₃₆N₆O₅ (*M* = 584.68 g/mol)**

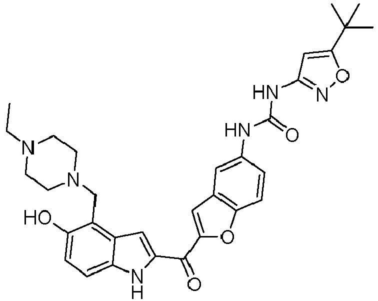

Synthesis of **69a** from **13** and **67** as described in general procedure 4A. Yield: 37%.

Synthesis of **69a** from **13** and **67** as described in general procedure 4B. Yield: 78%.

Additional purification was done by prep. HPLC to achieve a TFA salt of the corresponding amine.

**¹H-NMR** (DMSO-*d*₆, 300 MHz): δ = 11.86 (s, 1H), 10.57 (s, 1H), 9.60 (s, 1H), 9.01 (s, 1H), 8.10 (s, 1H), 8.01 (s, 1H), 7.76 (d, *J* = 9.1 Hz, 1H), 7.73 (s, 1H), 7.51 (d, *J =* 8.5 Hz, 1H), 7.26 (d, *J =* 8.7 Hz, 1H), 6.85 (d, *J* = 8.8 Hz, 1H), 6.53 (s, 1H), 4.01 (s, 2H), 2.91 - 2.01 (m, 8H), 2.33 (q, *J* = 7.1 Hz, 2H), 1.30 (s, 9H), 0.98 (t, *J =* 7.1 Hz, 3H).

### 1-(5-(tert-Butyl)isoxazol-3-yl)-3-(2-(5-hydroxy-4-((4-isopropylpiperazin-1-yl)methyl)-1H-indole-2-carbonyl)benzofuran-5-yl)urea (70a)

**C₃₃H₃₈N₆O₅ (*M*** = **598.70 g/mol)**

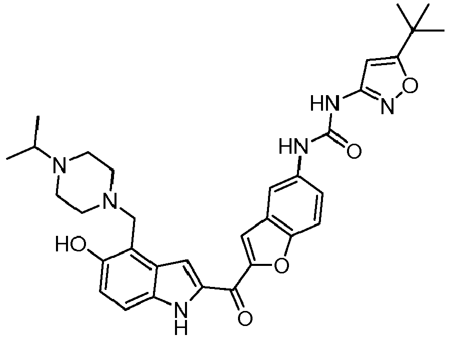

Synthesis of **70a** from **13** and **68** as described in general procedure 4A. Yield: 66%.

Synthesis of **70a** from **13** and **68** as described in general procedure 4B. Yield: 86%.

Additional purification was done by prep. HPLC to achieve a TFA salt of the corresponding amine.

**¹H-NMR** (DMSO-*d*₆, 300 MHz): δ = 11.86 (s, 1H), 10.48 (s, 1H), 9.61 (s, 1H), 9.03 (s, 1H), 8.10 (d, *J* = 2.2 Hz, 1H), 8.01 (s, 1H), 7.76 (d, *J* = 8.9 Hz, 1H), 7.73 (s, 1H), 7.51 (dd, *J* = 9.0, 2.2 Hz, 1H), 7.27 (d, *J=* 8.8 Hz, 1H), 6.86 (d, *J=* 8.8 Hz, 1H), 6.53 (s, 1H), 4.01 (s, 2H), 2.89 - 2.52 (m, 9H), 1.30 (s, 9H), 0.98 (d, *J* = 6.4 Hz, 6H).

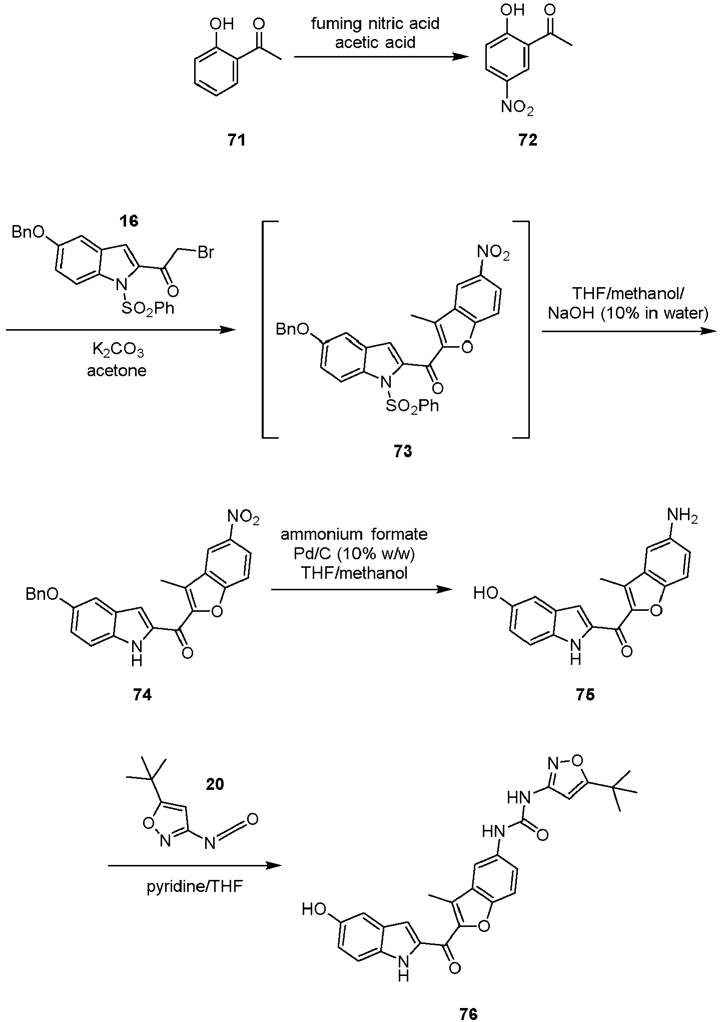

### 1-(2-Hydroxy-5-nitrophenyl)ethan-1-one (72)

**C₈H₇NO₄ (*M*** = **181.15 g/mol)**

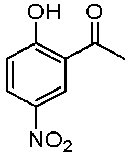

Reaction was performed as follows. 1-(2-Hydroxyphenyl)ethanone **(71)** was dissolved in glacial acetic acid (10 mL/g). After addition of fuming nitric acid (1.50 eq.), the reaction mixture was warmed up to room temperature and stirred for 16 h. After consumption of the starting material (TLC control) the reaction mixture was poured on ice water and the precipitating product was filtered. The crude product was purified by cc (SiO₂, petroleum ether:dichloromethane 4:1). Yield: 34%.

**¹H-NMR** (DMSO-*d*₆, 300 MHz): δ = 12.44 (s, 1H), 8.54 (d, *J* = 9.0 Hz, 1H), 8.30 (dd, *J* = 9.1, 2.9 Hz, 1H), 7.15 (d, *J* = 9.2 Hz, 1H), 2.67 (s, 3H).

**¹³C-NMR** (DMSO-*d*₆, 75 MHz): δ = 200.56, 164.55, 139.37, 129.84, 126.84, 122.31, 118.68, 29.51.

### (5-(Benzyloxy)-1H-indol-2-yl)(3-methyl-5-nitrobenzofuran-2-yl)methanone (74)

**C₂₅H₁₈N₂O₅ (*M*** = **426.42 g/mol)**

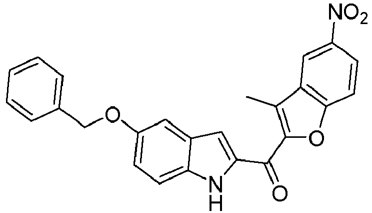

1-(2-Hydroxy-5-nitrophenyl)ethan-1-one **(72)** was dissolved in acetone (70 mL/g) and **16** (1.00 eq.) as well as K₂CO₃ (2.00 eq.) were added. The reaction mixture was heated to reflux for 4 hours and after complete consumption of the starting material (TLC control) the solid components were filtered off.

The solvent of the filtrate was removed under reduced pressure to obtain crude **73.** A mixture of THF:MeOH:NaOH (10% in water) (1:1:1, 100 mL/g) was added to the residue. The reaction mixture was heated to reflux for 4 hours. The reaction was stopped by pouring the reaction mixture on water. The resulting solid was filtered off and washed with a little amount of ethyl acetate and diethyl ether. Yield: 50% (2 steps).

**¹H-NMR** (DMSO-*d*₆, 300 MHz): δ = 11.91 (s, 1H), 8.81 (d, J = 2.3 Hz, 1H), 8.43 (dd, J = 9.1,2.4 Hz, 1H), 8.05 (d, J = 9.1 Hz, 1H), 7.80 (d, J = 1.4 Hz, 1H), 7.52 - 7.46 (m, 2H), 7.45 - 7.37 (m, 3H), 7.36 - 7.30 (m, 1H), 7.28 (d, J = 2.3 Hz, 1H), 7.09 (dd, J = 9.0, 2.4 Hz, 1H), 5.13 (s, 2H), 2.72 (s, 3H).

### (5-Amino-3-methylbenzofuran-2-yl)(5-hydroxy-1H-indol-2-yl)methanone (75)

**C₁₈H₁₄N₂O₃ (*M* = 306.32 g/mol)**

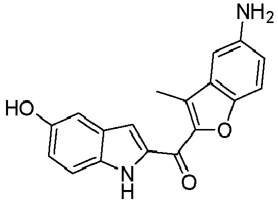

A solution of the starting material **(74)** in a mixture of THF:MeOH (2:1) (100 mL/g) was heated to reflux and ammonium formate (4.00 eq.) as well as palladium on activated charcoal (10% w/w, 10 w%) were added. After the reaction was finished, the mixture was filtered off over a pad of celite to remove the catalyst and an excess of ammonium formate. Water was added to the filtrate and the organic solvents were removed under reduced pressure. The product was extracted with ethyl acetate twice, the organic layer dried (Na₂SO₄) and the solvent removed under reduced pressure. Yield: 96%.

**¹H-NMR** (DMSO-*d*₆, 400 MHz): δ = 11.59 (d, *J=* 1.1 Hz, 1H), 8.98 (s, 1H), 7.62 (d, *J* = 1.5 Hz, 1H), 7.47 (d, *J =* 8.8 Hz, 1H), 7.33 (d, *J =* 8.8 Hz, 1H), 7.02 (d, *J* = 2.1 Hz, 1H), 6.88 (td, *J* = 9.9, 2.3 Hz, 2H), 6.81 (d, *J* = 2.1 Hz, 1H), 2.56 (s, 3H).

**¹³C-NMR** (DMSO-*d*₆, 101 MHz): δ = 174.61, 151.43, 147.70, 147.09, 145.26, 134.69, 132.67, 129.37, 128.06, 124.99, 117.91, 117.54, 113.28, 112.23, 109.91, 104.95, 102.26, 59.73, 20.74, 14.06, 9.65.

### 1-(5-(tert-Butyl)isoxazol-3-yl)-3-(2-(5-hydroxy-1H-indole-2-carbonyl)-3-methylbenzofuran-5-yl)urea (76)

**C₂₆H₂₄N₄O₅ (*M*** = **472.49 g/mol)**

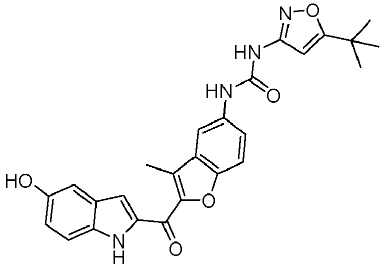

To a solution of the starting material **(75)** in THF (300 mL/g) a solution of the corresponding isocyanate (2.20 eq.) in THF was added. After addition of pyridine (25 eq.), the reaction mixture was stirred at room temperature (TLC-control). The reaction mixture was diluted with ethyl acetate and washed with HCl (1M in water) three times. The organic layer was dried (Na₂SO₄) and the solvent removed under reduced pressure. The product was crystallized from dichloromethane and petroleum ether and the solid was filtered off. Yield: 64%.

**¹H-NMR** (300 MHz, DMSO-d₆): δ = 11.69 (d, *J* = 1.8 Hz, 1H), 9.60 (s, 1H), 9.03 (s, 1H), 8.98 (s, 1H), 8.04 (d, *J* = 2.0 Hz, 1H), 7.76 (d, *J* = 8.9 Hz, 1H), 7.69 (d, *J* = 1.4 Hz, 1H), 7.48 (dd, *J* = 9.0, 2.2 Hz, 1H), 7.33 (d, *J* = 8.8 Hz, 1H), 7.03 (d, *J* = 2.2 Hz, 1H), 6.89 (dd, *J* = 8.9, 2.3 Hz, 1H), 6.55 (s, 1H), 2.64 (s, 3H), 1.30 (s, 9H).

### Example 4: Solubility of selected compound in different solvents

A very important concern of the new synthesized compounds was an increase of the water and ethanol solubility. Already known tyrosine kinase inhibitor Marbotinib **(13)** showed a not particularly good water solubility and therefore limited biological efficacy within *in vivo* applications. The prodrug concept was used for this purpose and the compound was transferred into a carbamate, that allowed the formation of a HCl salt (Sellmer et al. 2020). The problem within this concept regarding Marbotinib **(13)** and Marbotinib-Carbamat is the creation of a significantly increased molecule, extended by a large ineffective part. In an *in vivo* application the carbamate group will be split off and the prodrug is transferred into the poorly soluble drug again.

The new compounds of this invention may carry an amine group within their molecule structure, what enables the formation of an arbitrary salt and an associated increase of water or ethanol solubility without transferring the drugs into prodrugs. This led to considerable advantages and was an upgrade for the tyrosine kinase inhibitors.

### Performance of the solubility test:

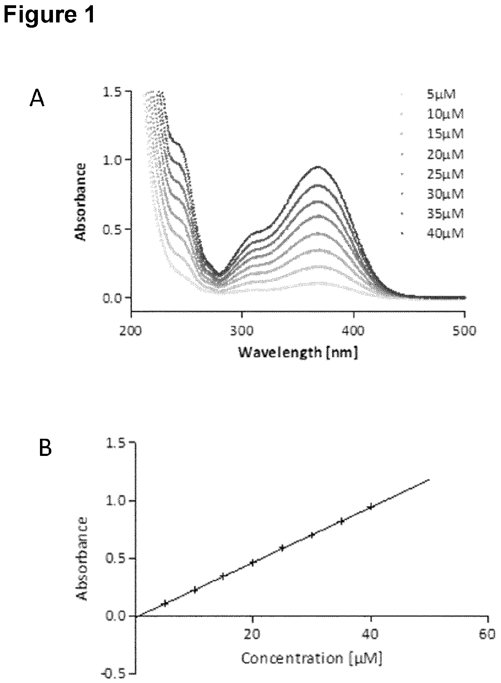

The solubility of the new compounds in ethanol and water was determined by UV-Vis spectroscopy. All UV-Vis measurements were carried out with an Agilent Cary 60 UV-Vis 2,00 between 800 - 200 nm. The absorbance was measured with a scan rate of 300.0 nm/min, a data interval of 0.50 nm and an UV-Vis Average Time of 0.10 s. For all measurements a cuvette with 10 mm light path was used. A calibration curve was plotted from several different concentrations and the solubility of the respective compound was determined by measuring the absorbance of a diluted saturated solution. As a blank curve a mixture of ultrapure water and ethanol in a ratio of 1:1 or 5:95 was recorded. For the calibration curve a 100 µM or a 10 µM solution of the compound in an ultrapure water:ethanol (1:1) or (5:95)-mixture was prepared and diluted to obtain at least 6 concentrations. The saturated solution of the respective compound was generated by shaking for at least 12 hours in ultrapure water or in ethanol in a micro test tube and subsequent centrifugation. The supernatant was taken using a syringe and filtered through a syringe filter (RC Membrane). The saturated solution was diluted with ultrapure water and ethanol to obtain an appropriate concentration in a water:ethanol (1:1) or (5:95)-mixture. The long wave absorbance maximum was used for determination of the solubility (mean values, determination in triplicate). The UV spectrum of compound **41c** in different concentrations is illustrated as an example in Figure 1A. By plotting the concentration of the solution against the absorbance values at the previously determined wavelength maximum a calibration curve was obtained (Figure 1B). By carrying out the same procedure, solubility values could be determined for other compounds as well as described.

**Table 2: Overview of the determined solubility of some selected synthesized compounds in different solvents in comparison with the solubility of Marbotinib (13) and AC220 (7), respectively.**

| | | **Solubility [g/L]** | | |
|---|---|---|---|---|
| **Compound** | **No.** | **DMSO** | **Water [g/L]** | **Ethanol [g/L]** |
| **Marbotinib** | **13** | Soluble | < 0.1 (Sellmer et al. 2020) | 0.22 |
| **Marbotinib-Carbamate*HCl** | | | 0.23 | 1.40 |
| **AC220** | **7** | 33.2 g/L | Practical Insoluble | Practical Insoluble |
| **Regetinib 4 trifluoroacetate** | **30b** | Soluble | 0.14 | 2.57 |
| **Regetinib 5** | **31a** | Soluble | <0.1 | 2.20 |
| **Regetinib 5 trifluoroacetate** | **31b** | Soluble | <0.1 | 1.89 |
| **Regetinib 6** | **32a** | Soluble | <0.1 | 0.91 |
| **Regetinib 6 trifluoroacetate** | **32b** | Soluble | <0.1 | 2.40 |
| **Regetinib 7** | **33a** | Soluble | <0.1 | 4.67 |
| **Regetinib 7 trifluoroacetate** | **33b** | Soluble | <0.1 | 3.63 |
| **Regetinib 8 trifluoroacetate** | **34b** | Soluble | <0.1 | 5.94 |
| **Regetinib 8 hydrochloride** | **34c** | Soluble | 5.77 | 2.38 |
| **Regetinib 11** | **38a** | Soluble | <0.1 | 0.89 |
| **Regetinib 11 trifluoroacetate** | **38b** | Soluble | 1.72 | 40.84 |
| **Regetinib 12** | **39a** | Soluble | <0.1 | 1.59 |
| **Regetinib 12 trifluoroacetate** | **39b** | Soluble | 12.35 | 16.91 |
| **Regetinib 13** | **40a** | Soluble | <0.1 | 1.24 |
| **Regetinib 13 trifluoroacetate** | **40b** | Soluble | 12.62 | 17.55 |
| **Regetinib 14** | **41a** | Soluble | 0.16 | 1.22 |
| **Regetinib 14 trifluoroacetate** | **41b** | Soluble | 7.80 | 6.16 |
| **Regetinib 14 hydrochloride** | **41c** | Soluble | 16.76 | 4.76 |
| **Regetinib 15** | **42a** | Soluble | <0.1 | 2.57 |
| **Regetinib 15 trifluoroacetate** | **42b** | Soluble | 13.09 | 50.34 |
| **Regetinib 15 hydrochloride** | **42c** | Soluble | 61.97 | 148.11 |
| **Regetinib 20** | **54a** | Soluble | <0.1 | 0.90 |
| **Regetinib 21** | **55b** | Soluble | <0.1 | 1.14 |
| **Regetinib 22 trifluoroacetate** | **56b** | Soluble | 0.22 | 2.54 |
| **Regetinib 27** | **69a** | Soluble | <0.1 | 0.97 |
| **Regetinib 27 trifluoroacetate** | **69b** | Soluble | 0.10 | 4.41 |
| **Regetinib 28** | **70a** | Soluble | <0.1 | 0.92 |
| **Regetinib 28 trifluoroacetate** | **70b** | Soluble | 0.37 | 1.82 |

As shown in Table 2, advantageously, all the compounds exhibit an increased water and ethanol solubility compared to Marbotinib **(13).** Advantageously, by transferring the drugs into TFA or HCl salts the solubility can be further enhanced to exceed the solubility of the HCl salt of prodrug Marbotinib-Carbamate. Quizartinib **(7)** exhibited no or at most very poor solubility in both solvents.

In summary, advantageously, the present invention provides compounds with increased solubility. Furthermore, the biological availability of the compounds is increased without creating a pharmacologically less valuable prodrug.

### Example 5: Biological evaluation - Biological activity of the novel TKi against recombinant human FLT3 (ITD) and FLT3 (D835Y) and in proliferation assay with MV4-11 cells (FLT3-ITD positive)

Biological evaluation of the new synthesized compounds was done by means of determining the kinase activity of the tyrosine kinase inhibitors against FLT3-ITD, FLT3-D835Y and in a proliferation assay with FLT3-ITD positive human MV4-11 cells (Table 3).

### Performance of the enzymatic and cellular assays:

Compounds were tested in 10-dose IC₅₀ mode with a 3-fold serial dilution starting at 10 µM. Control compound, staurosporine, was tested in 10-dose IC₅₀ mode with 4-fold serial dilution starting at 20 µM. Reactions were carried out at 10 µM ATP by Reaction Biology.

Cell proliferation was assessed with Cell Titer-Glo^{®} Viability Assay (Promega; Madison, WI, United States) according to the manufacturer's instructions. For the Cell Titer-Glo^{®} Assay, MV4-11 cells were incubated with the test compounds or staurosporine diluted in DMSO 1:3 starting with a concentration of 1 mM in 384 well plates for 72 h. Luminescence was recorded by Envision 2104 Multilabel Reader (PerkinElmer, Santa Clara, CA, United States).

**Table 3: Comparison of the IC50 values of the novel compounds with Marbotinib (13) and Quizartinib (7) in enzymatic assays and comparison of the IC50 values with Marbotinib (13) in cellular proliferation assay.**

| **Compound** | **No** | **Structure** | **IC₅₀ FLT3 (ITD) [nM]** | **IC₅₀ FLT3 (D835Y) [nM]** | **IC₅₀ MV4-11 cells [nM]** |
|---|---|---|---|---|---|
| **Marbotinib** | **13** | 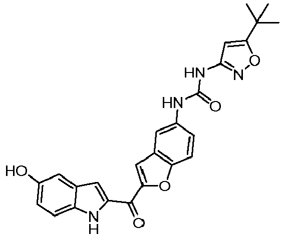 | 2.3 (Sellmer et al. 2020) | 4.84 (Sellmer et al. 2020) | 0.64 (Sellmer et al. 2020) |
| **Regetinib 4 hydrochloride** | **30c** | 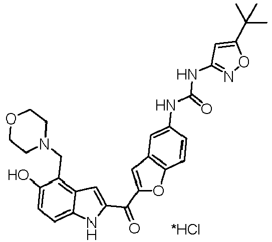 | 2.00 | n.d. | n.d. |
| **Regetinib 7** | **33a** | 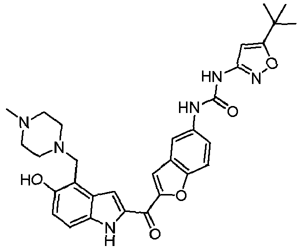 | 0.81 | 3.83 | n.d. |
| **Regetinib 8** | **34a** | 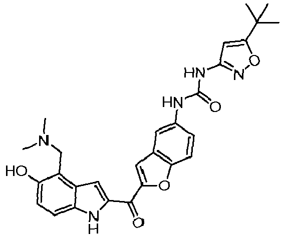 | 1.51 | n.d. | n.d. |
| **Regetinib 11 trifluoroacetate** | **38b** | 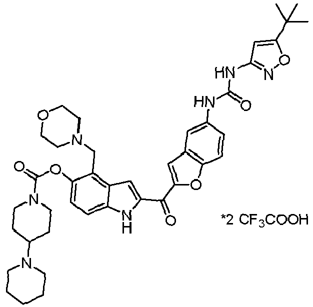 | 1.29 | 6.63 | 0.38 |
| **Regetinib 11 hydrochloride** | **38c** | 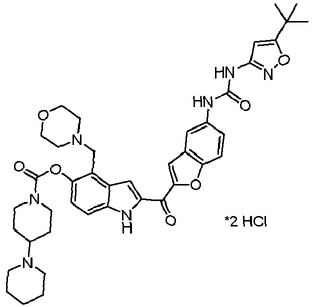 | 1.51 | n.d. | n.d. |
| **Regetinib 12** | **39a** | 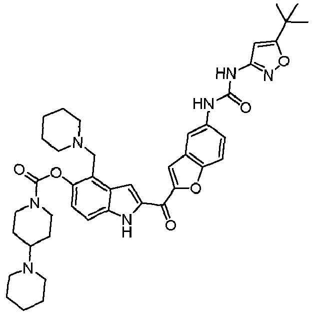 | 1.14 | n.d. | n.d. |
| **Regetinib 13** | **40a** | 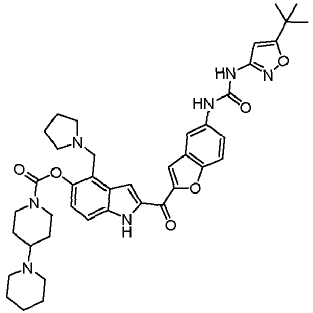 | 1.15 | n.d. | n.d. |
| **Regetinib 14** | **41a** | 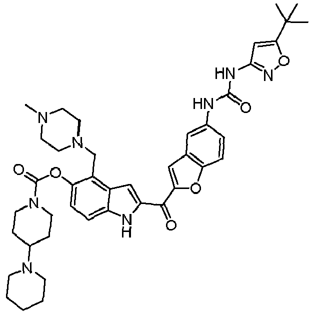 | 0.70 | 2.46 | n.d. |
| **Regetinib 14 trifluoroacetate** | **41b** | 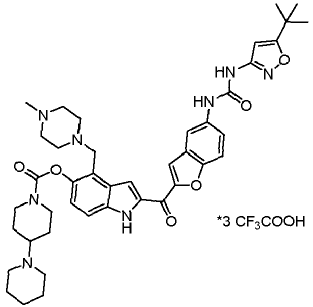 | 0.94 | 1.71 | 1.42 |
| **Regetinib 14 hydrochloride** | **41c** | 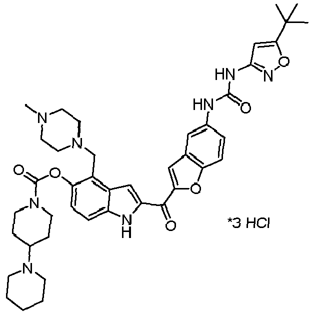 | 0.50 | n.d. | n.d. |
| **Regetinib 15** | **42a** | 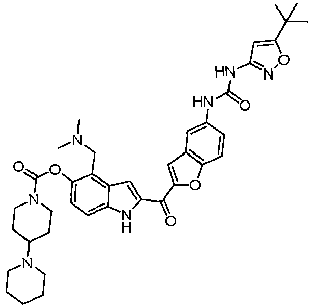 | 1.23 | n.d. | n.d. |
| **Regetinib 15 trifluoroacetate** | **42b** | 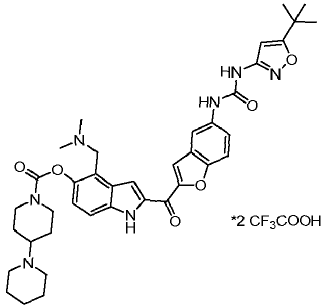 | 0.81 | 0.62 | 0.48 |
| **Regetinib 21** | **55b** | 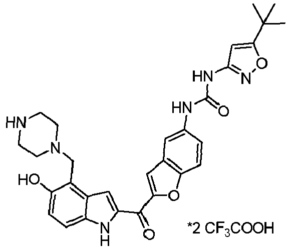 | 3.36 | 9.55 | 0.42 |
| **Regetinib 22 trifluoroacetate** | **56b** | 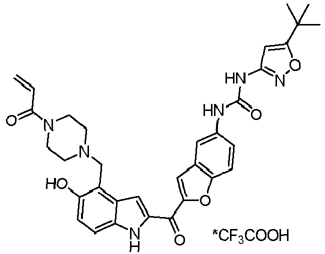 | 1.47 | 10.8 | 0.17 |
| **Regetinib 23** | **57a** | 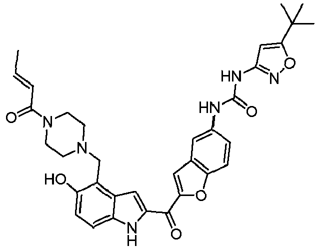 | 4.36 | n.d. | 0.24 |
| **Regetinib 27** | **69a** | 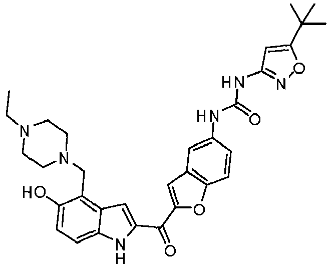 | 1.28 | 4.23 | 0.13 |
| **Regetinib 28** | **70a** | 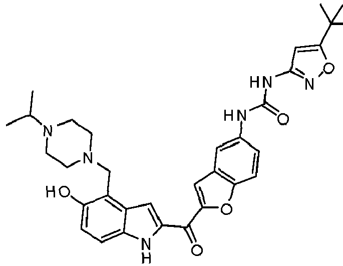 | 1.98 | 2.60 | 0.31 |
| **Quizartinib** | **7** | | 4.2 (Sellmer et al. 2020) | 137 (Sellmer et al. 2020) | |
| **Staurosporine** | | | 1.20 | 0.14 | 0.06 |

In summary, the data indicate improved on-target efficacy and beneficial effects compared to Marbotinib **(13).** The compounds of the invention are very potent and show inhibition of FLT3-ITD and FLT3-D835Y in a low nanomolar or even sub-nanomolar range. Much better or at least comparable IC₅₀ values in the performed enzymatic assays compared to Marbotinib **(13)** and Quizartinib **(7)** were shown for compounds of the invention. Furthermore, much better or at least comparable IC₅₀ values in the performed cellular assay with MV4-11 cells compared to Marbotinib **(13)** were shown for compounds of the invention. Particularly large advantages compared to Quizartinib **(7)** were found for the inhibition of the TKD mutant FLT3-D835Y, whereas the new substances showed much lower IC₅₀ values. Apart from that there are a few compounds that show inhibition almost as strong or even stronger as the unselective kinase inhibitor and control compound Staurosporine in enzymatic assays as well as in the cellular proliferation assay.

### Example 6: Phosphorylation assay in cellular systems

To show the inhibitory impact of novel compounds in a cellular content, the cellular kinase activity on c-KIT and FLT3-ITD was investigated.

### Performance of the phosphorylation assay in cellular systems (c-KIT):

In the cellular c-KIT phosphorylation assay the human acute megakaryoblastic leukemia cell line Mo7e was used, which expresses endogenously a high level of wild-type c-KIT. Stimulation of these cells with human stem cell factor (SCF) results in receptor tyrosine autophosphorylation. Mo7e cells were plated in RPMI supplemented with 20% FCS and 10 ng/ml GM-CSF in multiwell cell culture plates. After serum- and growth factor-starvation overnight cells were incubated with compounds (90 min at 37°C) in serum-free medium. Cells treated with 1,0E-05 M Staurosporine were used as low control. For stimulation, cells were treated with 100 ng/ml SCF for 3 min at room temperature. After cell lysis quantification of receptor autophosphorylation was assessed in 96 well plates via sandwich-ELISA using a kinase specific capture antibody and an anti-phosphotyrosine detection antibody.

The results obtained for c-KIT in the cellular phosphorylation assay is compiled in Table 4:

**Table 4: IC₅₀ values for the inhibition of c-KIT by a selection of novel compounds in phosphorylation assays with Sunitinib (4) used as reference compound.**

| **Compound** | **No** | **Structure** | **IC₅₀ [nM]** |
|---|---|---|---|
| **Regetinib 7 trifluoroacetate** | **33b** | 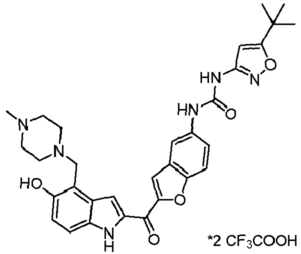 | 3.2 |
| **Regetinib 8 trifluoroacetate** | **34b** | 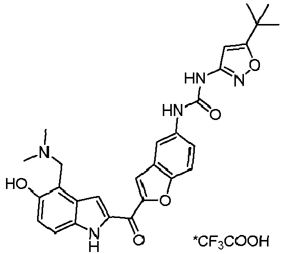 | 1.8 |
| **Regetinib 14 trifluoroacetate** | **41b** | 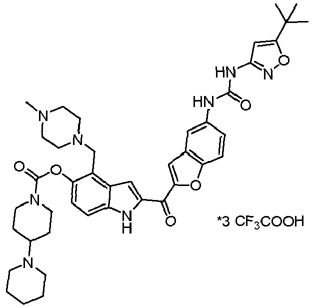 | 6.4 |
| **Regetinib 15 trifluoroacetate** | **42b** | 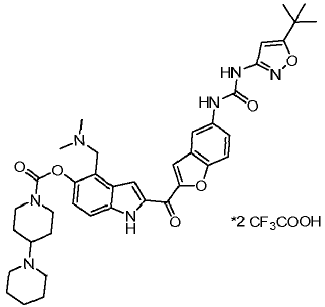 | 3.2 |
| **Sunitinib** | **4** | | 0.89 |

All four compounds inhibited phosphorylation within c-KIT cells much lower than proven and commercially available first-generation inhibitor Sunitinib **(5),** showing much higher IC₅₀ values in phosphorylation assay.

### Performance of the phosphorylation assay in cellular systems (FLT3-ITD):

In the cellular FLT3-ITD phosphorylation assay the murine embryonal fibroblast cell line (MEF) was used, which expresses a high level of exogenously introduced full-length human FLT3-ITD. Stimulation of these cells with human FLT3-Ligand results in receptor tyrosine autophosphorylation. MEF-FLT3-ITD cells were plated in DMEM supplemented with 10% FCS in multiwell cell culture plates. After serum-starvation overnight cells were incubated with compounds (90 min at 37°C) in serum-free medium. Cells treated with 1,0E-05 M Staurosporine were used as low control. For stimulation, cells were treated with 50 ng/ml FLT3-ligand for 5 min at room temperature. After cell lysis quantification of receptor autophosphorylation was assessed in 96 well plates via sandwich-ELISA using a kinase specific capture antibody and an antiphosphotyrosine detection antibody. The results obtained for FLT3-ITD in the cellular phosphorylation assay is compiled in Table 5:

**Table 5: IC₅₀ values for the inhibition of FLT3-ITD by a selection of novel compounds in phosphorylation assays with Sunitinib (4) used as reference compound.**

| **Compound** | **No** | **Structure** | **IC₅₀ FLT3-ITD [nM]** |
|---|---|---|---|
| **Regetinib 7 trifluoroacetate** | **33b** | 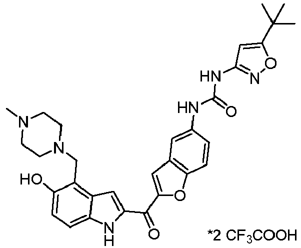 | 6.9 |
| **Regetinib 8 trifluoroacetate** | **34b** | 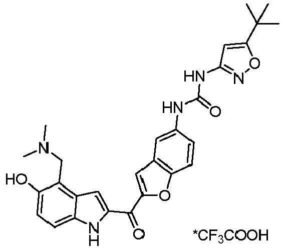 | 3.1 |
| **Regetinib 14 trifluoroacetate** | **41b** | 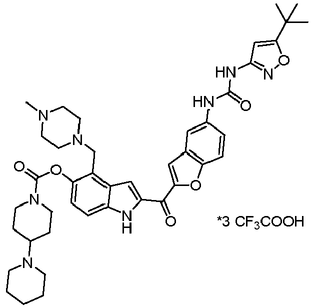 | 8.6 |
| **Regetinib 15 trifluoroacetate** | **42b** | 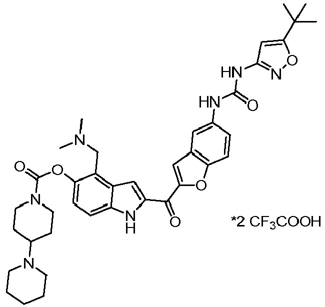 | 2.5 |
| **Sunitinib** | **4** | | 49 |

All four compounds inhibited phosphorylation in cells carrying FLT3-ITD mutation much better than proven and commercially available first-generation inhibitor Sunitinib **(4),** showing much lower IC₅₀ values in phosphorylation assay. In summary, on the one hand the new compounds are able to inhibit phosphorylation of FLT3-ITD in a cellular system much better than multikinase inhibitor Sunitinib **(4),** whereas they are not able to inhibit phosphorylation of c-KIT in a cellular system as strong as the reference compound (Sunitinib, **4).**

### Example 7: Selectivity in cell viability assay

For a more extensive investigation of the inhibitory impact of the new compounds in a cellular content, proliferation assays with further FLT3-overexpressing cell lines were performed (Table 6).

### Performance of the phosphorylation assay in cellular systems (c-KIT):

EOL-1 (acute myeloid eosinophilic leukemia), MOLM-13 (acute myeloid leukemia), MONO-MAC-1 (acute monocytic leukemia), and MONO-MAC-6 (acute monocytic leukemia). In vitro screening was performed by Charles River using the CellTiter-Blue^{®} Cell Viability Assay.

**Table 6: IC50 values obtained from Cell Titer Blue^{®} Cell Viability Assay with four hematological cell lines.**

| *Cell line* | *IC₅₀ [µM]* | | | | |
|---|---|---|---|---|---|
| | *EOL-1* | *MOLM-13* | *MONO-MAC-1* | *MONO-MAC-6* | *Geomean* |
| Regetinib 7 trifluoroacetate (33b) | 0.00005 | 0.00109 | 0.1000 | 0.00431 | 0.00223 |
| Regetinib 8 trifluoroacetate (34b) | 0.00002 | 0.00020 | 0.1000 | 0.00065 | 0.00074 |
| Regetinib 14 trifluoroacetate (41b) | 0.00016 | 0.00156 | 0.1000 | 0.00520 | 0.00337 |
| Regetinib 15 trifluoroacetate (42b) | 0.00004 | 0.00067 | 0.1000 | 0.00111 | 0.00130 |
| Marbotinib (13) (Sellmer et al. 2020) | <0.001 | <0.001 | <0.001 | | |
| Quizartinib (7) (Sellmer et al. 2020) | 0.0001 | 0.007 | 0.006 | 0.004 | |
| Midostaurin (1) (Sellmer et al. 2020) | 0.006 | 0.014 | 0.03 | 0.000003 | |

All five highly sensitive cell lines EOL-1, MOLM-13, MV4-11, MONO-MAC-1, MONO-MAC-6 exhibit FLT3 overexpression. All four tested compounds showed strong activity in the selected AML cell lines.

### Example 8: In vivo MTD study in non-Tumor bearing NOD/SCID mice

Since many compounds of this invention showed outstanding results compared to Marbotinib **(13)** and Quizartinib **(7)** in enzymatic assays, proliferation assay with MV4-11 cells and especially regarding solubility, some *in vivo* studys were performed analyzing the MTD (Maximum tolerated dose) and efficacy in subcutaneous MV4-11 CDX model in NOD (Non-Obese Diabetic)/SCID (Severe Combined Immunodeficiency) mice.

### Performance of the in vivo MTD study:

For an *in vivo* MTD (Maximum Tolerated Dose) study of **34c** 15 mice (*Mus musculus*) were used in 5 groups, which differ in dose concentration and dosing route. Group information and dosing details can be seen in the following Table 7.

**Table 7: Dosing Scheme of the in vivo MTD study.**

| | **Group** | | | | |
|---|---|---|---|---|---|
| | **1** | **2** | **3** | **4** | **5** |
| **Number of mice** | 3 | 3 | 3 | 3 | 3 |
| **Dose [mg/kg]** | 20 | 50 | 75 | 20 | 50 |
| **Dose volume [mL/kg]** | 10 | 10 | 10 | 10 | 10 |
| **Dose concentration [mg/mL]** | 2.0 | 5.0 | 7.5 | 2.0 | 5.0 |
| **Route** | Po | po | po | ip | ip |
| **Dosing frequency** | Daily (5 days) | Daily (5 days) | Daily (5 days) | Daily (5 days) | Daily (5 days) |

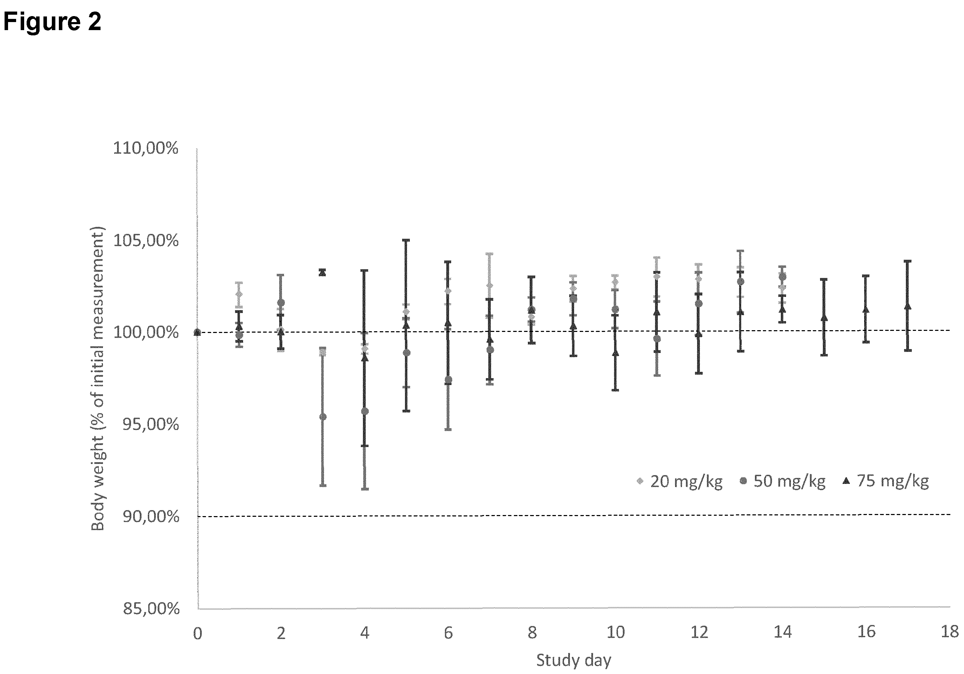

All mice were 6-8 weeks at study initiation, all were female. Acclimatization occurred for at least 14 days. Data about behavior and physical appearance was recorded at least once daily. Dosing of **34c** was done as a solution in deionized water, initiated on day 1 and continued for 5 days. One mouse in the 50 mg/kg po group and one in the 75 mg/kg po group lost more weight than 10% in the dosing phase of the study. The weight loss in the 50 mg/kg group was observed after receiving three doses of **34c** and after receiving four doses of **34c** in the 50 mg/kg group. A dosing break was made until the end of dosing for both mice and after a couple of days during the 10-day observation period both mice regained their weight. The body weight was measured daily for all three oral treatment groups for the 5 days of dosing and the following 10-day observational period. The mean relative body weight of the oral treatment group can be seen in Figure 2. In general, there were no clinical signs observed in the 20 mg/kg and the 50 mg/kg po treatment groups, while for the mouse of the 75 mg/kg treatment group which lost weight, swelling of the right hind limb and foot was noticed. Since this observation was made only after dosing, it can be considered it was not caused by the compound.

### Example 9: In vivo efficacy study in subcutaneous MV4-11 CDX model in NOD/SCID mice

Based on the results of MTD study dosing of **34c** for the efficacy study was adjusted and Midostaurin **(1)** as well as Quizartinib **(7)** were used as reference compounds.

### Performance of the in vivo efficacy study:

*In vivo* anti-tumor efficacy of **34c** was determined using MV4-11 bearing NOD (Non-Obese Diabetic)/SCID (Severe Combined Immunodeficiency) mice. 33 mice (*Mus musculus,* 25 mice and 30% excess) were used in 5 groups, where all mice were randomly assigned into treatment groups.

All mice were 6-8 weeks at study initiation, all were female. Acclimatization occurred for at least 14 days. Data about behavior and physical appearance was recorded at least once daily. The tumor was examined three times a week and body weight was determined daily for two weeks and three times a week for the duration of the study. Tumor volume was estimated for the duration of the study by measuring the tumor in two dimensions using electronic calipers and the formula 0.5 (L×W²).

Compound **34c** was dosed in two different concentrations, Midostaurin **(1)** and Quizartinib **(7)** were used as reference compounds. All compounds were administered orally. Treatment with compound **34c** was started when tumors reached a mean volume of 100-200 mm³. Dosing of **34c** as well as Midostaurin **(1)** and Quizartinib **(7)** was initiated one day after randomization and was continued for 5 days. After 5 days of dosing the mice were observed for three more weeks. Dosage of **34c** was done in deionized water as a vehicle, Midostaurin **(1)** in 5% DMSO + 45% PEG 300 + ddH₂O and Quizartinib **(7)** in 0.5% methylcellulose. **34c** was diluted with deionized water to reach the required concentration, while solvent was added to Midostaurin **(1)** individually and in order and the mixture was used within 30 min after mixing. Quizartinib **(7)** was treated with the corresponding vehicle and the mixture was sonicated for 5-10 min until a fine suspension was achieved. Further dosage information can be seen in following Table 8.

**Table 8: Dosing Scheme of the in vivo efficacy study in subcutaneous MV4-11 CDX model in NOD/SCID mice.**

| | **Group** | | | | |
|---|---|---|---|---|---|
| | **1** | **2** | **3** | **4** | **5** |
| **Number of mice** | 5 | 5 | 5 | 5 | 5 |
| **Compound** | Vehicle (Water) | **34c** | **34c** | 1 | 7 |
| **Compound dose [mg/kg]** | 0 | 20 | 50 | 50 | 50 |
| **Dose volume [mL/kg]** | 10 | 10 | 10 | 10 | 10 |
| **Dose concentration [mg/mL]** | 0 | 2.0 | 5.0 | 5.0 | 5.0 |
| **Route** | po | po | po | po | po |
| **Dosing frequency** | Daily days) | Daily days) | Daily days) | Daily days) | Daily days) |

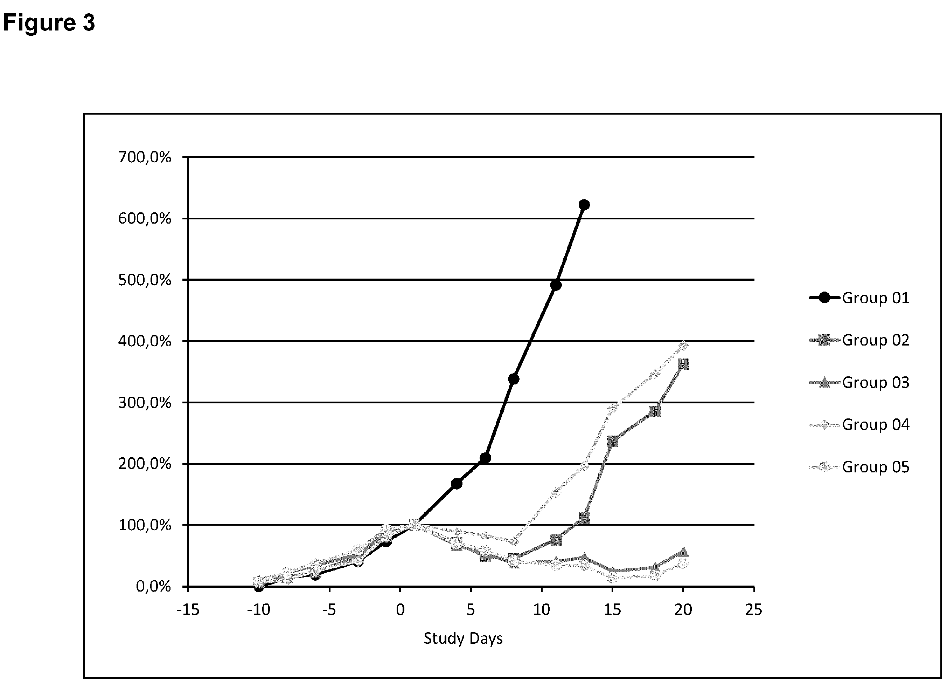

Advantageously, the results of tumor inhibition of **34c** were remarkable since the compound (Treatment group 3) was similarly active as Quizartinib **(7,** Treatment group 5) within the same compound doses (50 mg/kg) and much more active than Midostaurin **(1,** Treatment group 4) when evaluating the relative tumor volume. Even the 20 mg/kg treatment group (Treatment group 2) showed better results regarding tumor volume measurements than Midostaurin **(1,** dosed with 50 mg/kg). An overview over the progression of the relative tumor volume of all groups is shown in Figure 3.

Furthermore, advantageously, compound **34c** was better tolerated when evaluating death counts and the mice of both groups (Treatment groups 2 and 3) showed less relative body weight loss compared to the mice treated with Midostaurin **(1,** Treatment group 4) or Quizartinib (7, Treatment group 5). Only 1 mouse of treatment group 1 and 2 had to be sacrificed after 20 days of study. Mice of the Midostaurin group had to be sacrificed after study day 5 (1 mouse) and 8 (1 mouse) and a high weight loss could be observed for all mice of this group. Mice of the Quizartinib group had to be sacrificed after study day 6 (2 mice) and also a higher weight loss could be observed compared to both treatment groups of **34c.**

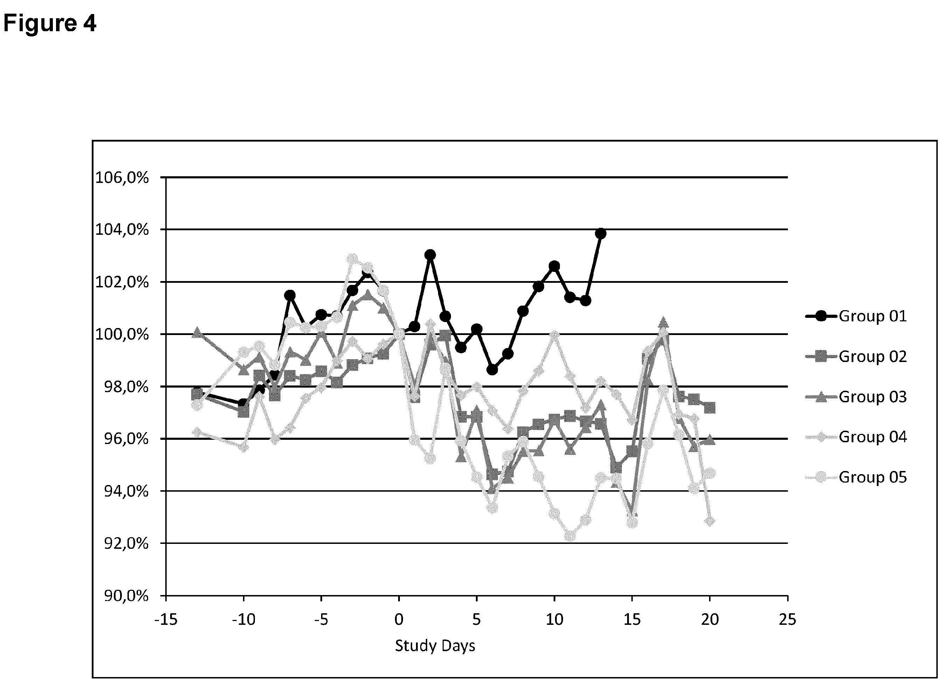

An overview over the progression of the relative body weight of all groups is shown in Figure 4.

In summary, advantageously, compound **34c** achieved outstanding results in *in vivo* efficacy study. **34c** can be dosed in lower concentrations than Midostaurin **(1)** and in much lower doses than Marbotinib **(13)** with very good results regarding efficacy. The compound is compatible with oral admission and is very tolerable within different doses. The interaction of good tolerability up to 50 mg/kg and good efficacy even with only 20 mg/kg gavage is very beneficial for **34c** in its role for use as an FLT3 inhibitor.

### Example 10: Comparison between midostaurin (PKC-412), marbotinib, and regetinib.

### Midostaurin (PKC-412) and Marbotinib

WO2019/034538A1 shows a comparison of marbotinib and midostaurin, particularly with respect to their in vivo efficacy (see Kaplan-Meier curve of Figure 13 of WO2019/034538A1). Marbotinib and midostaurin were dosed perorally at 50 mg/kg body weight for 5 days. In the marbotinib group, the first death was observed two days before the first death in the midostaurin group. Furthermore, the last death within the midostaurin group was observed 2 days after the last death in the marbotinib group. Thus, it may be concluded that, in this series of tests, marbotinib showed a decreased efficiency compared to midostaurin.

### Midostaurin (PKC-412) and 34c

### Survival

A molecule of the new regetinib series **(34c)** was compared with midostaurin regarding its in vivo efficacy. Midostaurin was dosed perorally at 50 mg/kg body weight for 5 days. **34c** was studied in two different groups that differed in terms of dose. The first group was dosed perorally at 20 mg/kg body weight for 5 days, while the second was dosed at 50 mg/kg.

The first death (day 6) in the midostaurin group occurred well before the first death in the **34c** (20 mg/kg) group (day 9). A second death in the midostaurin group was observed as early as on day 9. No deaths were observed in the higher-dose **34c** group (50 mg/kg) until day 36.

| **Group** | ***Day of study*** | | | | | |
|---|---|---|---|---|---|---|
| | ***5*** | ***6*** | ***8*** | ***13*** | ***26*** | ***36*** |
| **34c (20 mg/kg)** | 5 | 5 | 5 | 4 | 4 | 0 |
| **34c (50 mg/kg)** | 5 | 5 | 5 | 5 | 5 | 5 |
| **Midostaurin** | 5 | 4 | 4 | 3 | 3 | 0 |

Conclusively, **34c** showed a significantly better efficacy and tolerability in terms of overall survival than midostaurin in this series of tests. Advantageously, **34c** has a significantly increased efficacy compared to marbotinib.

### Tumor volume

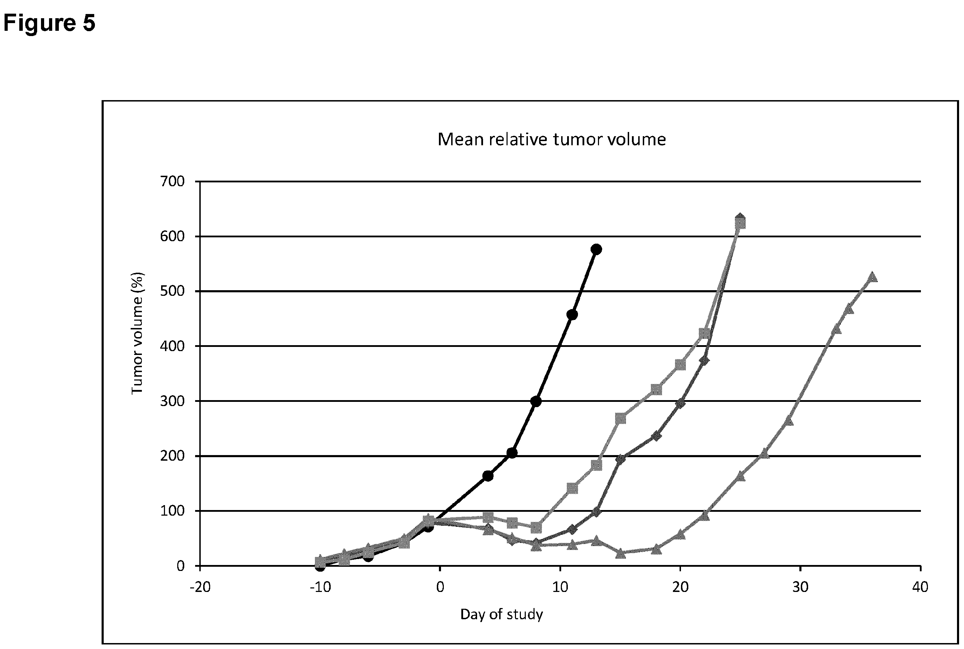

The change in relative tumor volume in the **34c** (20 mg/kg), **34c** (50 mg/kg), and midostaurin (50 mg/kg) groups was observed (Figure 5).

The first curve (•) shows the development of the mean relative tumor volume in mice that received no drug. The second curve (▪) shows the development of the mean relative tumor volume in the group of mice treated with midostaurin (50 mg/kg). The third curve (ó) shows the development of the mean relative tumor volume in the **34c** group (20 mg/kg). It can be seen that, advantageously, **34c** shows a higher efficiency than midostaurin (particularly in terms of tumor volume) even at significantly lower doses (20 compared to 50 mg/kg). The fourth curve (Δ) is the plot of the mean relative tumor volume in the group of mice treated with **34c** (50 mg/kg). The results show a significantly increased efficiency of **34C** compared to midostaurin (50 mg/kg). Conclusively, **34c** showed a significantly higher efficiency than midostaurin when treated with the same doses. Furthermore, advantageously, the efficiency of **34c** was comparable to the efficiency of midostaurin when applying **34c** at significantly lower doses.

### Example 11: Comparison between quizartinib, marbotinib, and regetinib.

### Quizartinib (AC220) and Marbotinib carbamate

Previous studies have compared quizartinib (AC220) and marbotinib carbamate (Beyer et. al. - Cell Chem Biol 2022). Quizartinib (AC220) was administered at doses of 10 mg/kg and 50 mg/kg. Marbotinib carbamate was administered at doses of 50 mg/kg and 75 mg/kg. Even in the group treated with 10 mg/kg quizartinib, the first death was observed later than in the group treated with 50 mg/kg marbotinib carbamate. The groups dosed at 50 mg/kg showed that in the marbotinib-carbamate group the first deaths were observed well before (about 7 days) the first deaths in the quizartinib group. In WO2019/034538A1, marbotinib at various doses was compared with quizartinib (AC220) with respect to in vivo efficiency. Significantly lower efficiency was observed in the marbotinib groups compared to the quizartinib groups, even when quizartinib was dosed at only 10 mg/kg.

### Quizartinib (AC220) and 34c

### Survival

A molecule of the new regetinib series **(34c)** was compared with quizartinib (AC220) regarding in vivo efficiency. Quizartinib was dosed perorally at 50 mg/kg body weight for 5 days. **34c** was studied in two groups; the first group was dosed perorally at 20 mg/kg body weight for 5 days, and the second was dosed at 50 mg/kg. In the quizartinib group, the first two deaths (day 7) occurred well before the first death in the **34c** (20 mg/kg) group (day 9). In the **34c** group with the higher dose (50 mg/kg), no deaths were noted until day 36. Numbers in the table indicate the number of living subjects at the respective day of the study.

| Group | *Day of study* | | | | | |
|---|---|---|---|---|---|---|
| | *5* | *6* | *8* | *13* | *26* | *36* |
| **34c** (20 mg/kg) | 5 | 5 | 5 | 4 | 4 | 0 |
| **34c** (50 mg/kg) | 5 | 5 | 5 | 5 | 5 | 5 |
| Quizartinib | 5 | 5 | 3 | 3 | 3 | 3 |

Conclusively, **34c** showed a significantly better efficiency and tolerability (in terms of overall survival) than quizartinib. These results indicate that **34c** has a significantly increased efficiency compared to marbotinib and marbotinib carbamate.

### Tumor volume

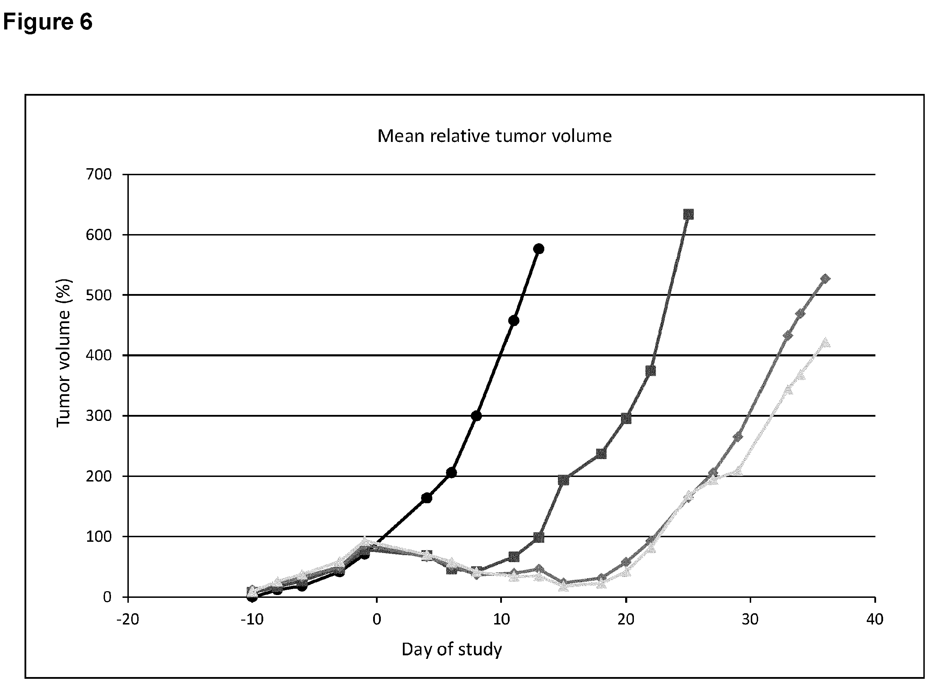

The change in relative tumor volume in the **34c** (20 mg/kg), **34c** (50 mg/kg), and quizartinib (AC220) (50 mg/kg) groups was observed (Figure 6).

The first curve (•) shows the development of the mean relative tumor volume in mice that received no drug. The fourth curve (Δ) shows the mean relative tumor volume in the group of mice treated with quizartinib (50 mg/kg). The second curve (▪) shows the development of the mean relative tumor volume in the group of mice treated with **34c** (20 mg/kg), and the third curve (◊) shows mice treated with **34c** (50 mg/kg). As demonstrated, **34c** has an efficiency (in terms of tumor volume) comparable to the efficiency of quizartinib at the same dose. Furthermore, according to the study on the tolerability of the active ingredient **34c,** the use of a higher dose of **34c** of up to 75 mg/kg is possible, which leads to a further significant increase in efficacy.

### Example 12: Marbotinib carbamate and 34c - prodrug concept.

Marbotinib carbamate was synthesized to improve the solubility and bioavailability of the drug using the prodrug concept. For this purpose, the molecule was significantly enlarged and an inactive part was added. When cleaving the carbamate group in vivo, the prodrug becomes the insoluble drug. Advantageously, the preparation of a prodrug to improve solubility is absolutely not necessary in the case of **34c,** since the molecule itself exhibits very good water and ethanol solubility. For example, the compound of the invention may have an amino group that enables salt formation and exhibits very good water and ethanol solubility.

When using a prodrug, there is still the question of whether the prodrug ligand (in this case the carbamate part) is cleaved quickly enough, cleanly and completely, and thus whether a high level of efficacy can be achieved. It must also be ensured that the prodrug ligand (carbamate moiety) also leaves the body safely, quickly and without problems. With the choice of the prodrug ligand, the disease state, the dose and the duration of the therapy must also be taken into account. An incomplete clearance process may lead to unexpected results or unexpected toxic metabolites could occur. Due to all these mentioned aspects, a prodrug formation using a carbamate brings significantly increased complexity and additional challenges. Advantageously, prodrug formation can be circumvented with a compound of the invention, such as **34c,** because the molecule provides good solubility. Advantageously, the compound of the invention may be used as an active drug without prodrug formation.

### REFERENCES

Bandini, Marco; Eichholzer, Astrid (2009): Catalytic functionalization of indoles in a new dimension. In: Angewandte Chemie (International ed. in English) 48 (51), S. 9608-9644. DOI: 10.1002/anie.200901843.

Harriman, G.: FUNCTIONALIZED HETEROCYCLES AS CHEMOKINE RECEPTOR MODULATORS.

Heldin, Carl-Henrik (1995): Dimerization of cell surface receptors in signal transduction. In: Cell 80 (2), S. 213-223. DOI: 10.1016/0092-8674(95)90404-2.

Isgör Y G et. al. (2008): Novel Aminomethylindole Derivatives as Inhibitors of pp6oc-Src Tyrosine Kinase: Synthesis and Biological Activity. In: Chem Biol Drug Des 72, S. 599-604. Online verfügbar unter http://arxiv.org/pdf/2008.00734v1.

Laras, Y.; Garino, C.; Dessolin, J.; Week, C.; Moret, V.; Rolland, A.; Kraus, J-L (2009): New N(4)-substituted piperazine naphthamide derivatives as BACE-1 inhibitors. In: Journal of enzyme inhibition and medicinal chemistry 24 (1), S. 181-187. DOI: 10.1080/14756360802048939.

Mahboobi, Siavosh; Dove, Stefan; Sellmer, Andreas; Winkler, Matthias; Eichhorn, Emerich; Pongratz, Herwig et al. (2009): Design of chimeric histone deacetylase- and tyrosine kinase-inhibitors: a series of imatinib hybrides as potent inhibitors of wild-type and mutant BCR-ABL, PDGF-Rbeta, and histone deacetylases. In: Journal of medicinal chemistry 52 (8), S. 2265-2279. DOI: 10.1021/jm800988r.

Mahboobi, Siavosh; Teller, Steffen; Pongratz, Herwig; Hufsky, Harald; Sellmer, Andreas; Botzki, Alexander et al. (2002): Bis(1H-2-indolyl)methanones as a novel class of inhibitors of the platelet-derived growth factor receptor kinase. In: Journal of medicinal chemistry 45 (5), S. 1002-1018. DOI: 10.1021/jm010988n.

Monti, S. A.; Johnson, W. O. (1970): Position selective mannich reactions of some 5- and 6-hydroxyindoles. In: Tetrahedron 26 (15), S. 3685-3694. DOI: 10.1016/80040-4020(01)92947-3.

Sellmer, Andreas; Pilsl, Bernadette; Beyer, Mandy; Pongratz, Herwig; Wirth, Lukas; Elz, Sigurd et al. (2020): A series of novel aryl-methanone derivatives as inhibitors of FMS-like tyrosine kinase 3 (FLT3) in FLT3-ITD-positive acute myeloid leukemia. In: European journal of medicinal chemistry 193, S. 112232. DOI: 10.1016/j.ejmech.2020.112232.

The features of the present invention disclosed in the specification, the claims, and/or in the accompanying figures may, both separately and in any combination thereof, be material for realizing the invention in various forms thereof.

## Claims

1. A compound having the general formula I: 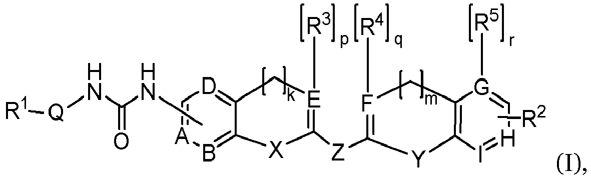 wherein
**A, B, D, H,** and **I** are independently selected from CH and N, preferably CH;
**E, F,** and **G** are independently selected from C and N, preferably C;
**k** and **m** are independently selected from o and 1, preferably 0;
**p, q,** and **r** are independently selected from 0 and 1, wherein at least one of **p, q** and **r** is 1, preferably each of **p, q** and **r** is 1; wherein, if p is 1, then **E** is C, and if p is 0, then **F** is N; wherein, if q is 1, then **F** is C, and if q is 0, then **F** is N; wherein, if r is 1, then **G is** C, and if r is 0, then **G** is N;
**X** and **Y are** independently selected from NH, O, and S, preferably from NH and O; wherein, preferably, X is O and/or Y is NH;
**Z** is selected from C=O, C=S, and CHOH, preferably is C=O;
**Q** is selected from the group consisting of five- or six-membered aromatic systems and five- or six-membered heteroaromatic systems, preferably is isoxazole;
**R¹** is selected from hydrogen and alkyl, preferably C₁-C₆ alkyl, more preferably *tert*-butyl;
**R²** is selected from the group consisting of hydrogen, alkyl, preferably C₁-C₆ alkyl, OH, ester, carbamate, alkylamino, preferably C₁-C₆ alkylamino, alkoxy, preferably C₁-C₆ alkoxy, 2-hydroxysuccinyl, an amino acid residue, preferably a basic amino acid residue, and any structure of the following group **R⁶** 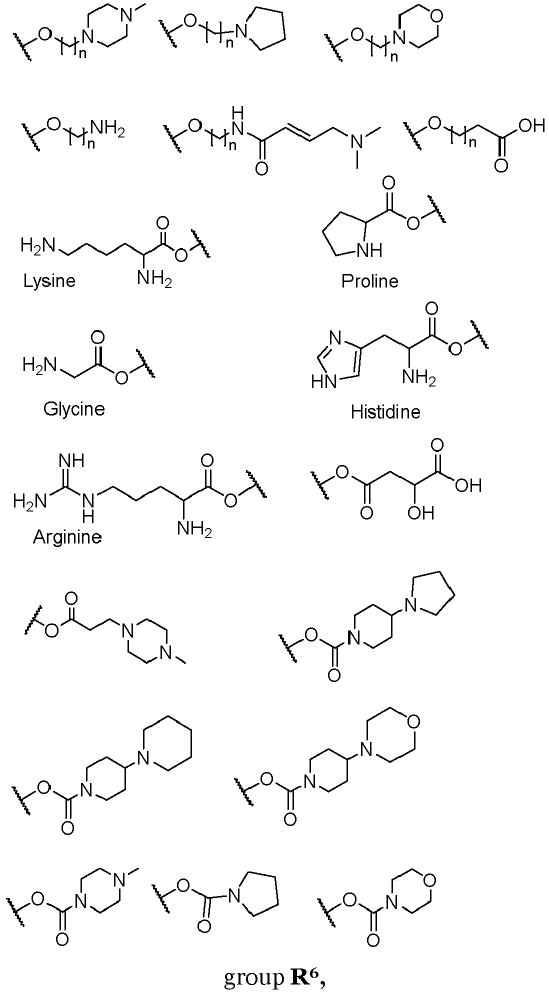
wherein **n** is independently selected from 0, 1, 2, 3, 4, 5, and 6,
wherein each of said alkyl, OH, ester, carbamate, alkylamino, and alkoxy is optionally substituted with a substituent selected from the group consisting of amino, alkylamino, cyclic alkylamino, cyclic diaminoalkyl, preferably piperazinyl or 1-alkylpiperazinyl, and heterocyclic alkylamino, preferably morpholinyl;
**R³** is selected from the group consisting of hydrogen, alkyl, preferably C₁-C₆ alkyl, alkylamino, preferably C₁-C₆ alkylamino, cyclic alkylamino, cyclic diaminoalkyl, such as piperazinyl or 1-alkylpiperazinyl, heterocyclic alkylamino, such as morpholinyl, and any structure of the following group **R⁷** 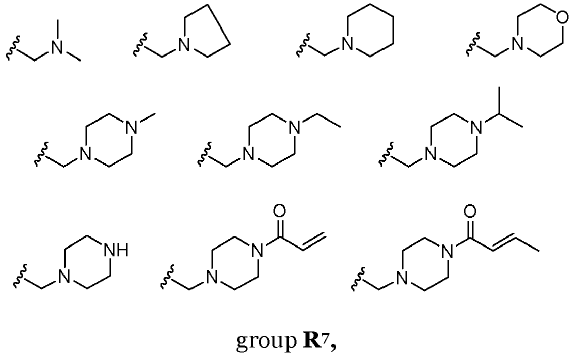
wherein each of said alkyl, alkylamino, cyclic alkylamino, cyclic diaminoalkyl, and heterocyclic alkylamino is optionally substituted;
**R⁴** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁸** 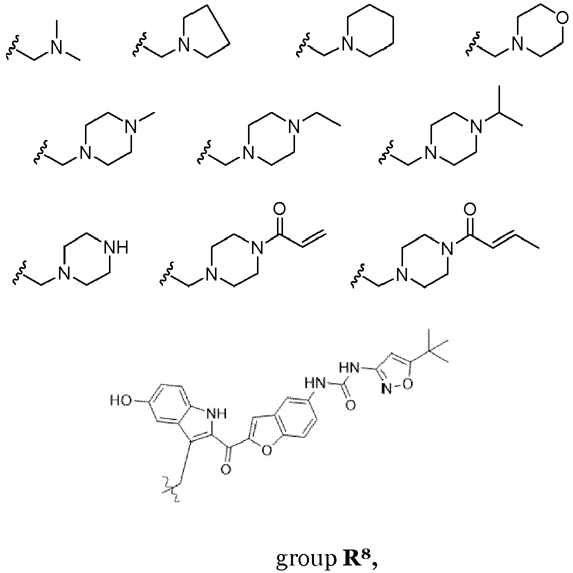
wherein each of said alkyl, alkylamino, amino, dialkylamino, cyclic alkylamino, cyclic diaminoalkyl, and heterocyclic alkylamino is optionally substituted;
**R⁵** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁹** 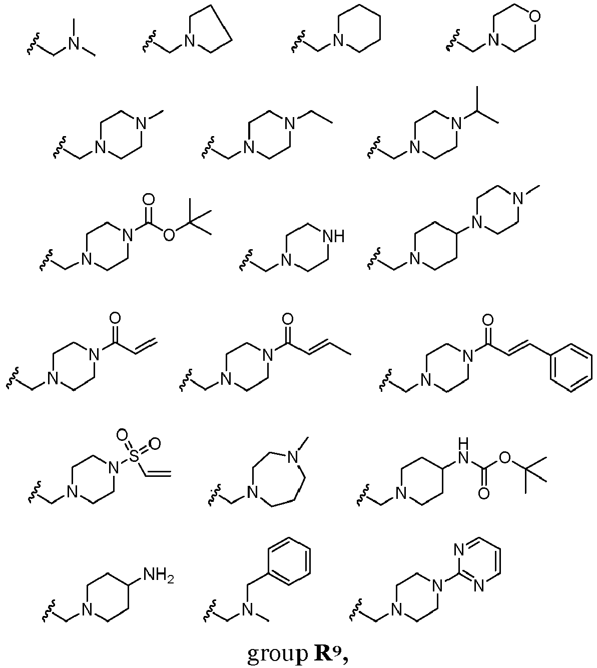
wherein, preferably, **R⁵** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R¹⁰** 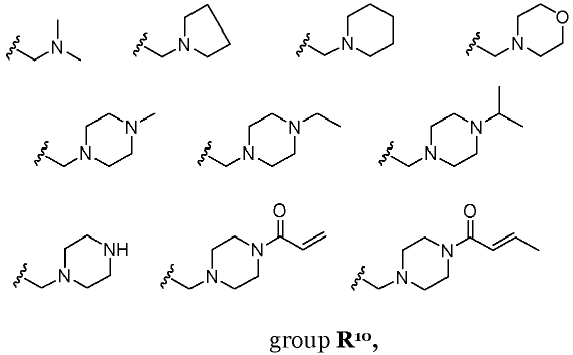
wherein each of said alkyl, alkylamino, amino, dialkylamino, cyclic alkylamino, cyclic diaminoalkyl, and heterocyclic alkylamino is optionally substituted;
under the proviso that at least one of **R³**, **R⁴**, and **R⁵** is not hydrogen;
and pharmaceutically acceptable salts or solvates thereof.

2. The compound according to claim 1 having the general formula II 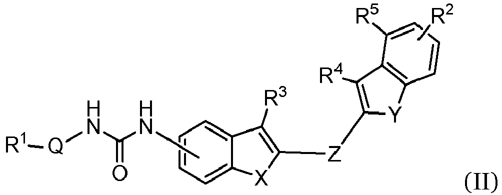 wherein
**X** and **Y are** independently selected from NH, O, and S, preferably from NH and O; wherein, preferably, X is O and/or Y is NH;
**Z** is selected from C=O, C=S, and CHOH, preferably is C=O;
**Q** is selected from the group consisting of five- or six-membered aromatic systems and five- or six-membered heteroaromatic systems, preferably isoxazole;
**R¹** is selected from hydrogen and alkyl, preferably C₁-C₆ alkyl, more preferably tert-butyl;
**R²** is selected from the group consisting of hydrogen, alkyl, preferably C₁-C₆ alkyl, OH, ester, carbamate, alkylamino, preferably C₁-C₆ alkylamino, alkoxy, preferably C₁-C₆ alkoxy, 2-hydroxysuccinyl, an amino acid residue, preferably a basic amino acid residue, and any structure of the following group **R⁶** 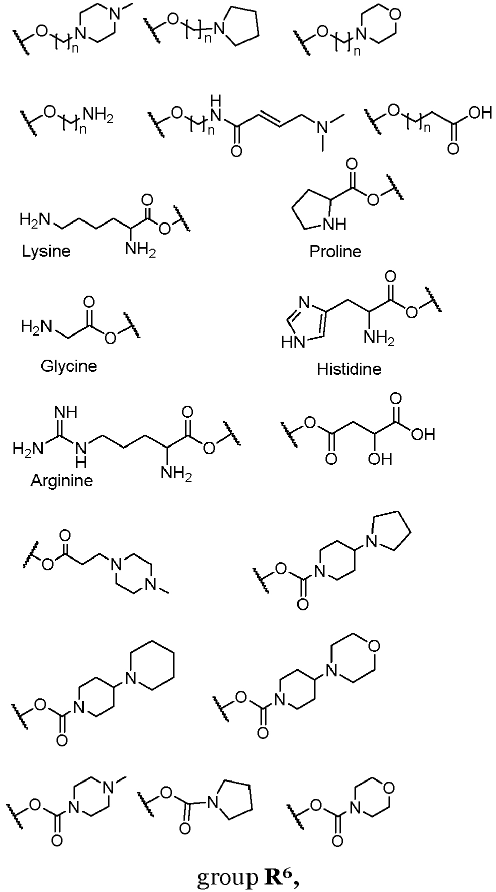
wherein **n** is independently selected from 0, 1, 2, 3, 4, 5, and 6,
wherein each of said alkyl, OH, ester, carbamate, alkylamino, and alkoxy is optionally substituted with a substituent selected from the group consisting of amino, alkylamino, cyclic alkylamino, cyclic diaminoalkyl, preferably piperazinyl or 1-alkylpiperazinyl, and heterocyclic alkylamino, preferably morpholinyl;
**R³** is selected from the group consisting of hydrogen, alkyl, preferably C₁-C₆ alkyl, alkylamino, preferably C₁-C₆ alkylamino, cyclic alkylamino, cyclic diaminoalkyl, such as piperazinyl or 1-alkylpiperazinyl, heterocyclic alkylamino, such as morpholinyl, and any structure of the following group **R⁷** 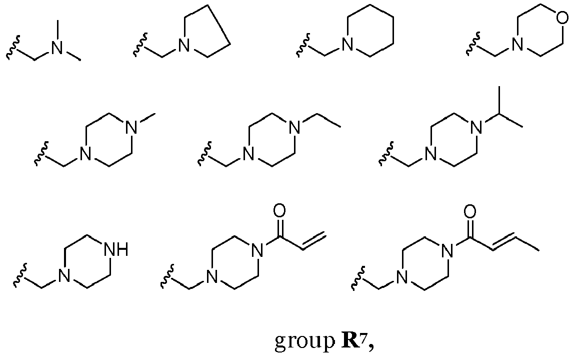
wherein each of said alkyl, alkylamino, cyclic alkylamino, cyclic diaminoalkyl, and heterocyclic alkylamino is optionally substituted;
**R⁴** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁸** 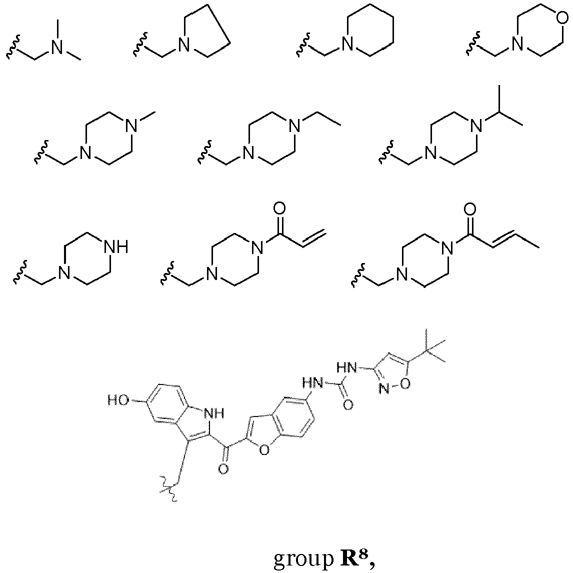
wherein each of said alkyl, alkylamino, amino, dialkylamino, cyclic alkylamino, cyclic diaminoalkyl, and heterocyclic alkylamino is optionally substituted;
**R⁵** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁹** 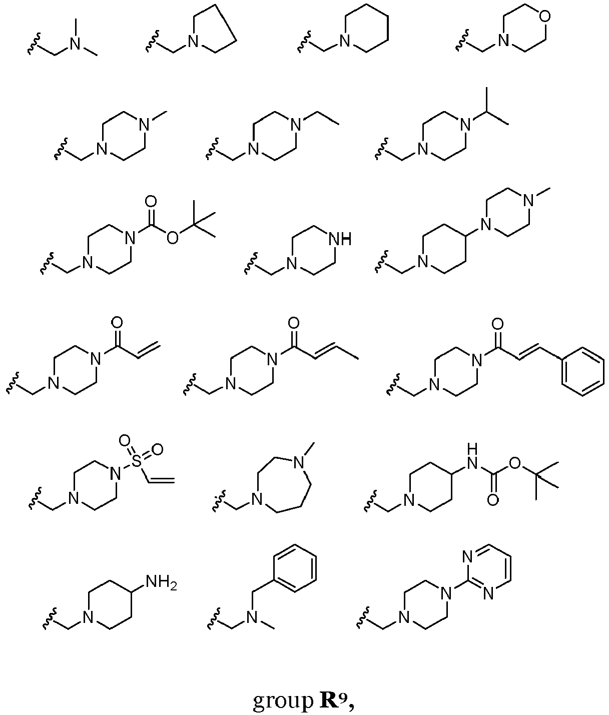
wherein, preferably, **R⁵** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R¹⁰** 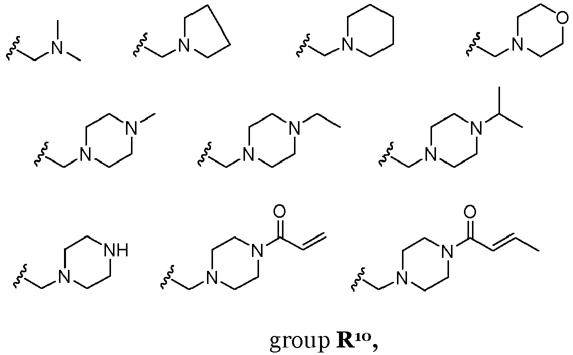
wherein each of said alkyl, alkylamino, amino, dialkylamino, cyclic alkylamino, cyclic diaminoalkyl, and heterocyclic alkylamino is optionally substituted;
under the proviso that at least one of **R³**, **R⁴,** and **R⁵** is not hydrogen;
and pharmaceutically acceptable salts or solvates thereof.

3. The compound according to claim 1 or 2, wherein
**Q** is isoxazole; and/or
**R¹** is selected from C₁-C₆ alkyl, preferably is tert-butyl.

4. The compound according to any one of the foregoing claims, wherein
**X** is O;
**Y** is NH; and/or
**Z** is C=O;
wherein, preferably,
**Q** is isoxazole;
**R¹** is selected from C₁-C₆ alkyl, preferably is tert-butyl;
**X** is O;
**Y** is NH; and
**Z** is C=O.

5. The compound according to any one of the foregoing claims, wherein **R²** is OH, carbamate, alkylamino, or an amino acid residue; preferably is OH or carbamate, more preferably is OH or

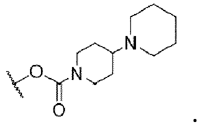

6. The compound according to any one of the foregoing claims, wherein each of p, q, and r is 1, and wherein **R³** is selected from hydrogen and alkyl, preferably selected from hydrogen and C₁-C₆ alkyl, more preferably selected from hydrogen and methyl; under the proviso that, if **R³** is hydrogen, then at least one of **R⁴** and **R⁵** is not hydrogen.

7. The compound according to any one of the foregoing claims having the general formula III 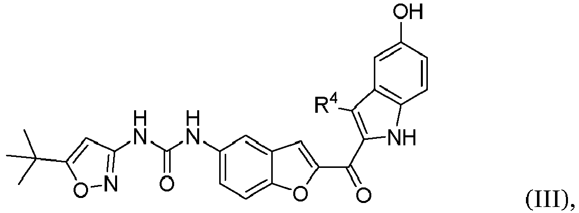 wherein
**R⁴** is selected from the group consisting of alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁸** 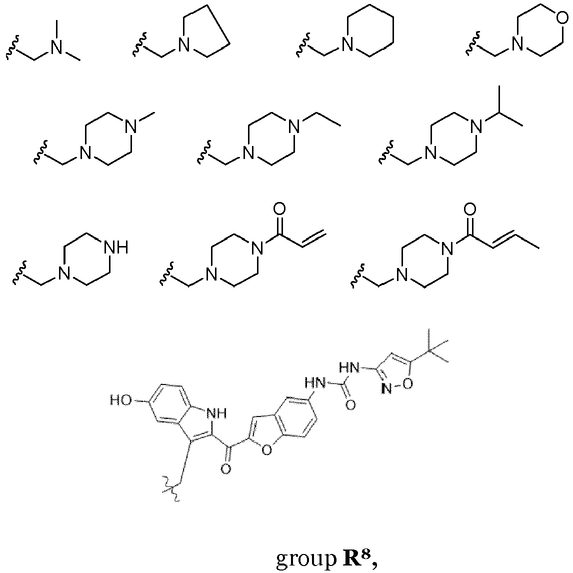
wherein each of said alkyl, alkylamino, amino, dialkylamino, cyclic alkylamino, cyclic diaminoalkyl, and heterocyclic alkylamino is optionally substituted;
wherein, preferably, **R⁴** is selected from alkyl, preferably C₁-C₆ alkyl, more preferably methyl, and any structure of the above group **R⁸**;
wherein, more preferably, **R⁴** is selected from alkyl, preferably C₁-C₆ alkyl, more preferably methyl, and any structure of the following group **R¹¹** 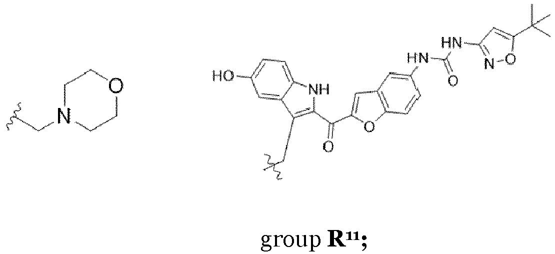
and pharmaceutically acceptable salts or solvates thereof.

8. The compound according to any one of claims 1-6 having the general formula IV 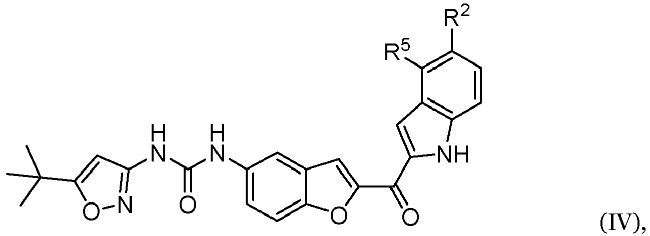 wherein
**R²** is selected from the group consisting of OH and carbamate, preferably is selected from OH 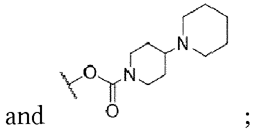
**R⁵** is selected from the group consisting of alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R⁹** 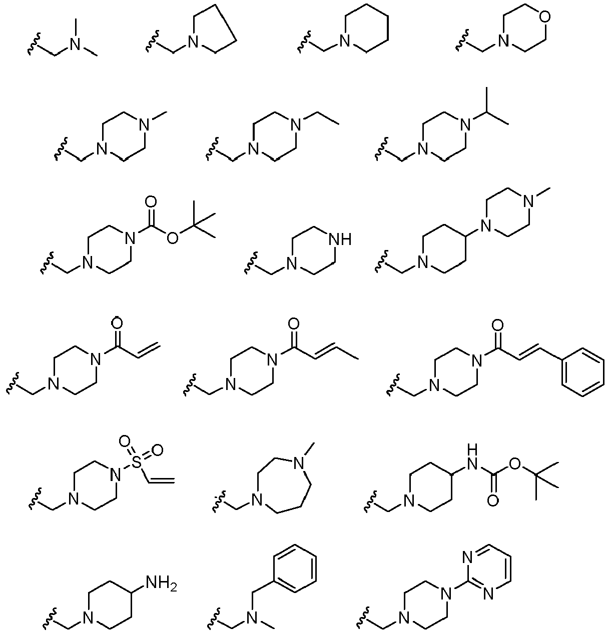 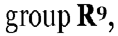
wherein, preferably, **R⁵** is selected from the group consisting of hydrogen, alkyl, preferably C₁-C₆ alkyl, alkylamino, preferably C₁-C₆ alkylamino, amino, dialkylamino, cyclic alkylamino, such as pyrrolidinyl or piperidinyl, cyclic diaminoalkyl, such as piperazinyl or 1-alkylpiperazinyl, heterocyclic alkylamino, such as morpholinyl, and any structure of the following group **R¹⁰** 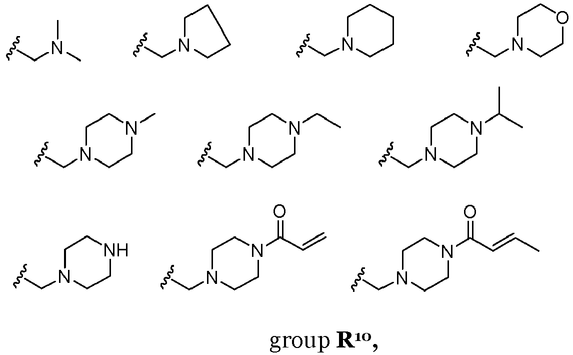
wherein each of said alkyl, alkylamino, amino, dialkylamino, cyclic alkylamino, cyclic diaminoalkyl, and heterocyclic alkylamino is optionally substituted;
wherein, more preferably, **R⁵** is selected from any structure of the above group **R⁹**;
wherein, even more preferably, **R⁵** is selected from any structure of the above group **R¹⁰**; and pharmaceutically acceptable salts or solvates thereof.

9. The compound according to any one of the foregoing claims, wherein **R³** is hydrogen; and wherein
**R⁵** is hydrogen, and **R⁴** is selected from alkyl, preferably C₁-C₆ alkyl, more preferably methyl, and any structure of the following group **R⁸** 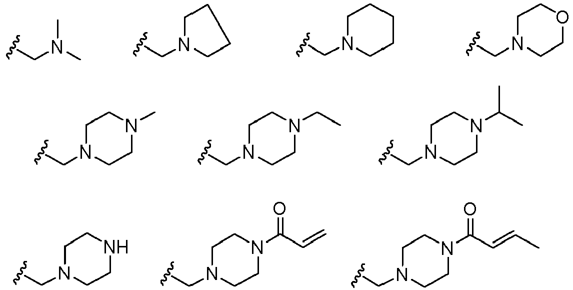 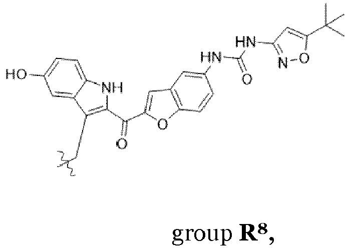
**R⁴** preferably being selected from alkyl, preferably C₁-C₆ alkyl, more preferably methyl, and any structure of the following group **R¹¹** 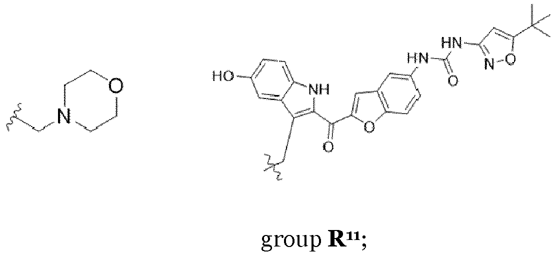 or
**R⁴** is hydrogen, and **R⁵** is selected from any structure of the following group **R⁹** 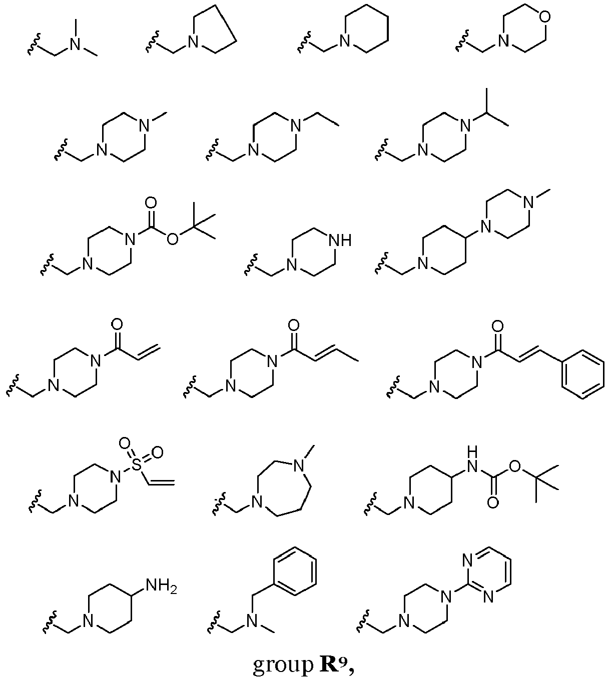 wherein, preferably, **R⁵** is selected from any structure of the following group **R¹⁰** 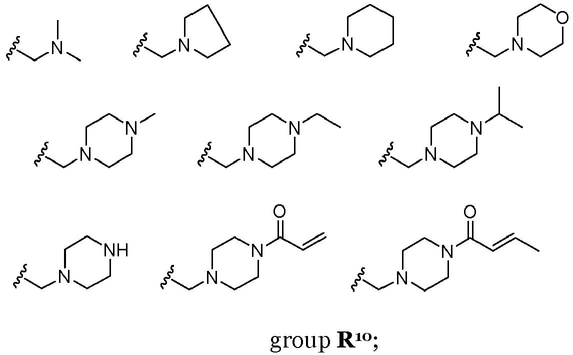
and pharmaceutically acceptable salts or solvates thereof.

10. The compound according to any one of the foregoing claims, wherein said salts are selected from hydrochlorides, trifluoroacetates, sulfates, phosphates, mesylates, tosylates, formiates, and acetates, preferably selected from hydrochlorides and trifluoroacetates; and/or, if **R²** = OH, a salt of the respective phenolate comprising sodium, potassium, calcium, zinc or another pharmaceutically acceptable cation forming a soluble salt.

11. The compound according to any one of the foregoing claims, wherein said compound has any one of the structures as shown hereafter:
| cpd. no. | structure | cpd. no. | structure |
|---|---|---|---|
| **24a** | 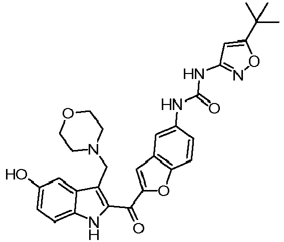 | **33c** | 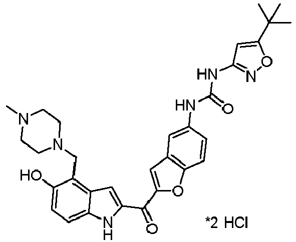 |
| **24b** | 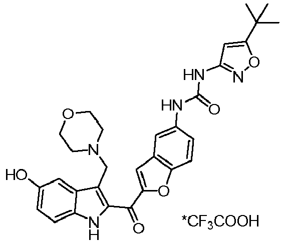 | **34c** | 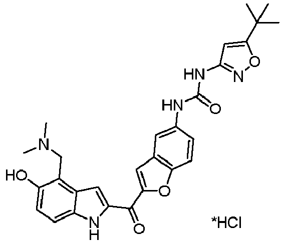 |
| **26** | 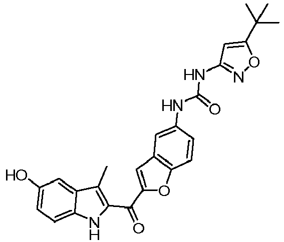 | **38c** | 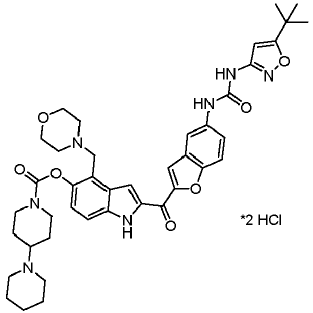 |
| **29** | 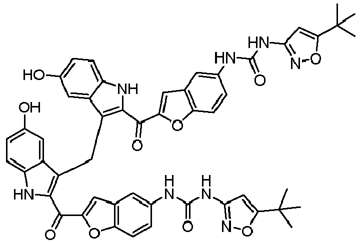 | **39c** | 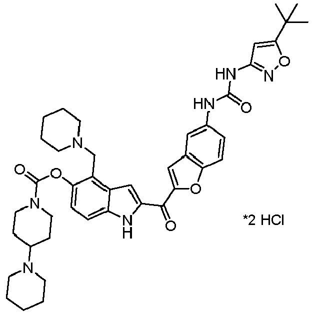 |
| **30a** | 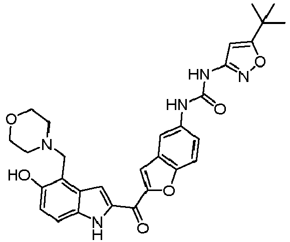 | **40c** | 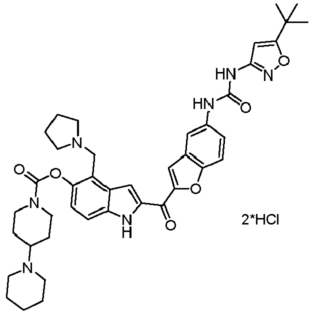 |
| **30b** | 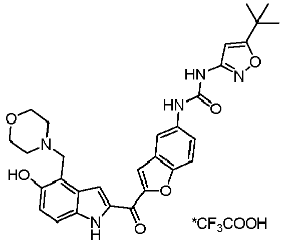 | **41 c** | 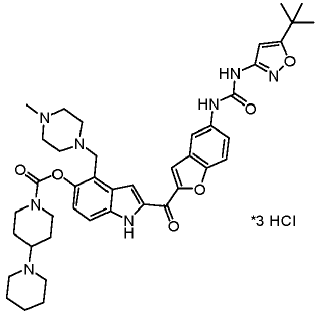 |
| **31a** | 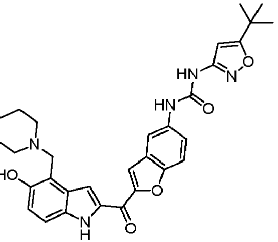 | **42c** | 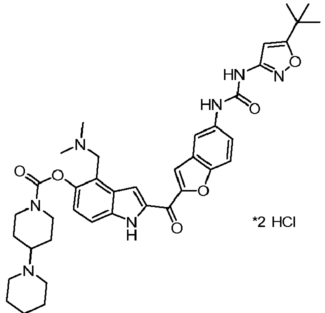 |
| **31b** | 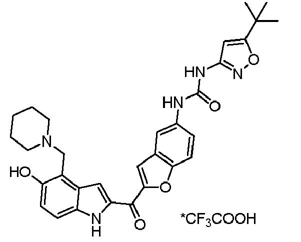 | **46a** | 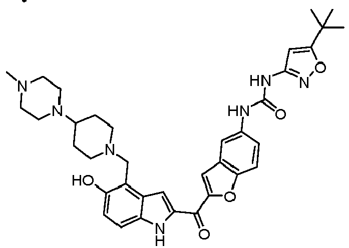 |
| **32a** | 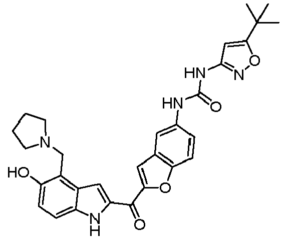 | **46b** | 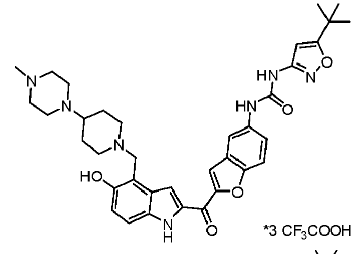 |
| **32b** | 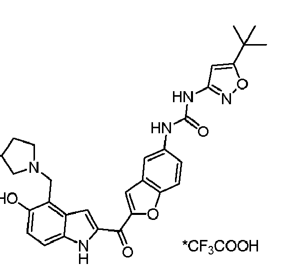 | **47a** | 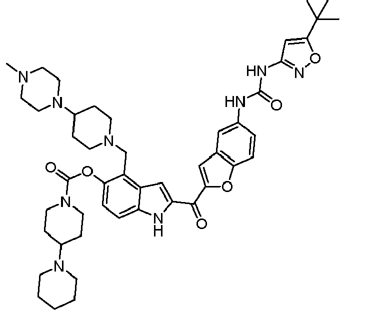 |
| **33a** | 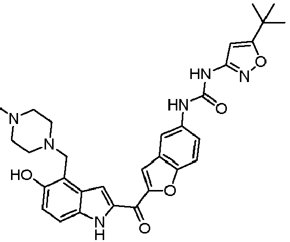 | **47b** | 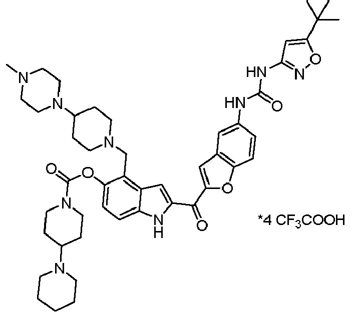 |
| **33b** | 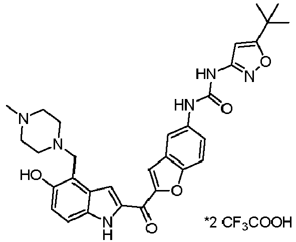 | **52a** | 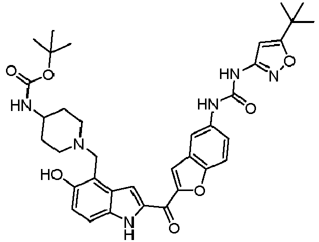 |
| **34a** | 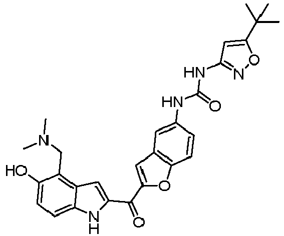 | **53b** | 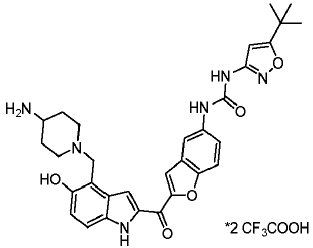 |
| **34b** | 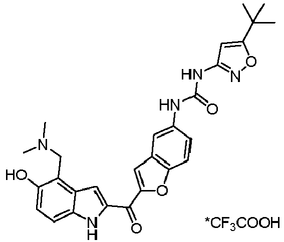 | **54a** | 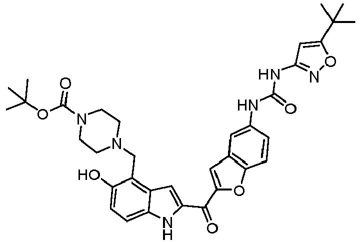 |
| **35a** | 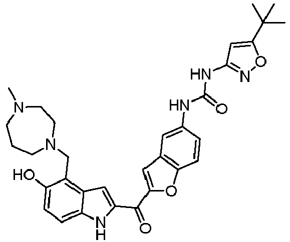 | **55b** | 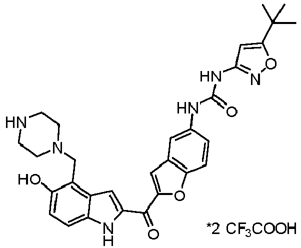 |
| **36a** | 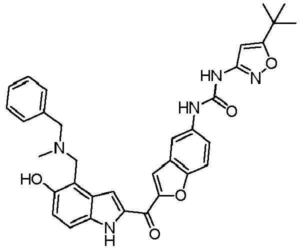 | **56a** | 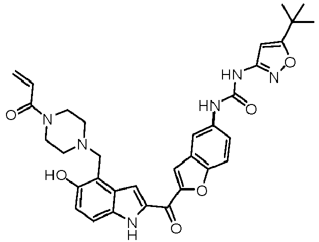 |
| **38a** | 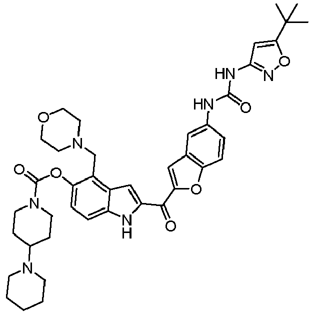 | **56b** | 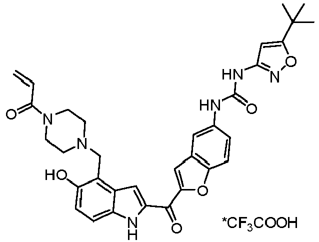 |
| **38b** | 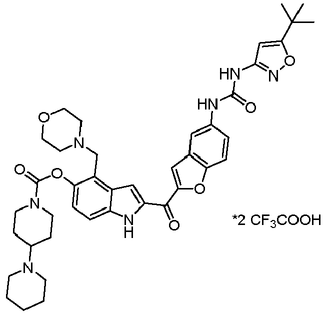 | **57a** | 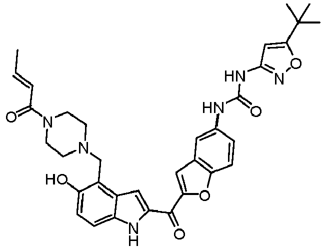 |
| **39a** | 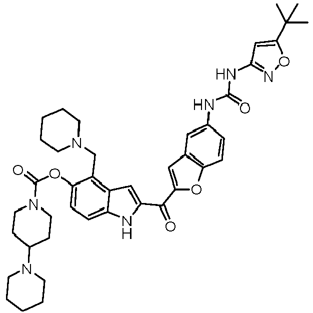 | **57b** | 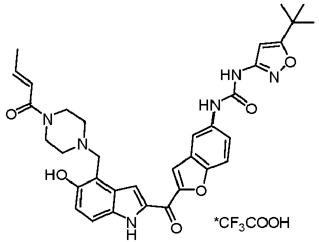 |
| **39b** | 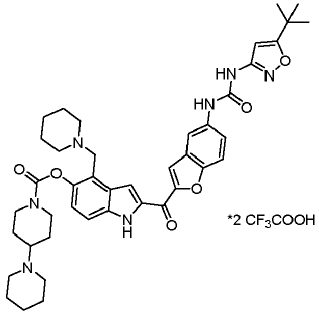 | **58a** | 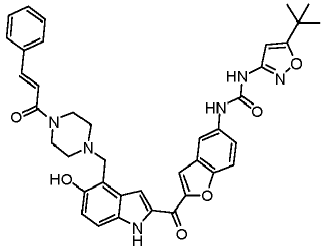 |
| **40a** | 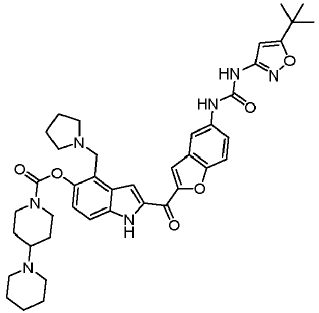 | **58b** | 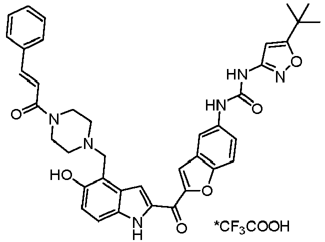 |
| **40b** | 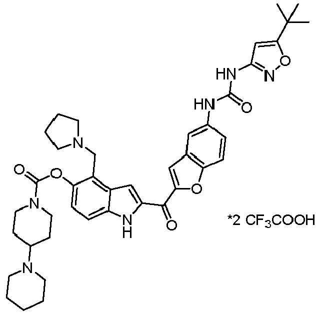 | **61a** | 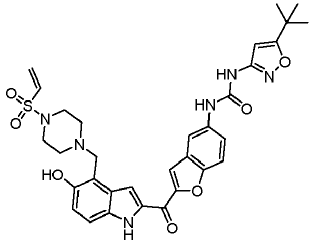 |
| **41a** | 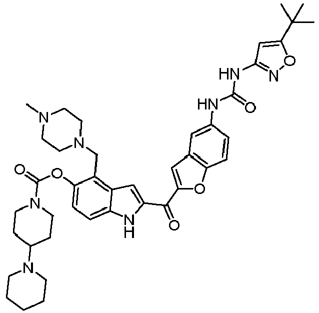 | **64a** | 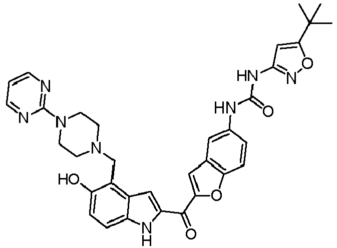 |
| **41b** | 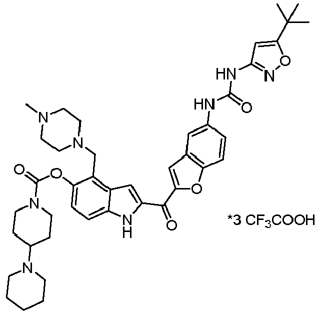 | **69a** | 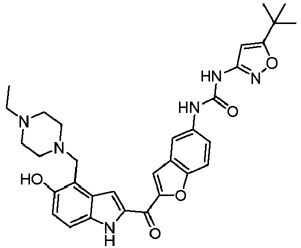 |
| **42a** | 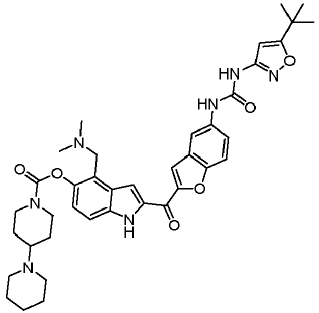 | **69b** | 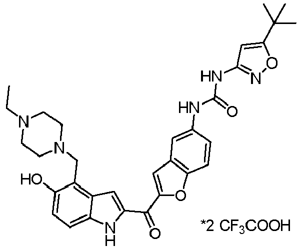 |
| **42b** | 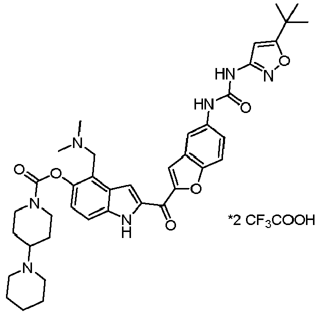 | **70a** | 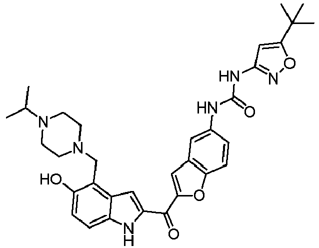 |
| **30c** | 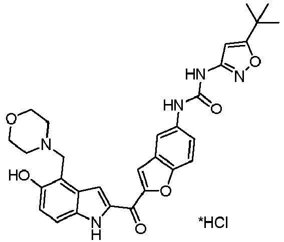 | **70b** | 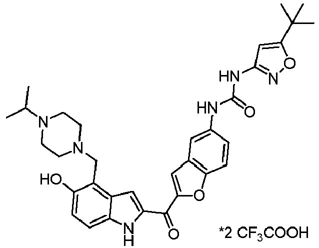 |
| **31C** | 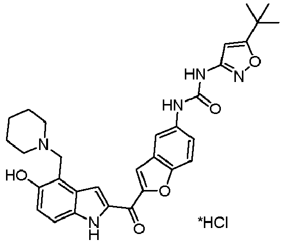 | **76** | 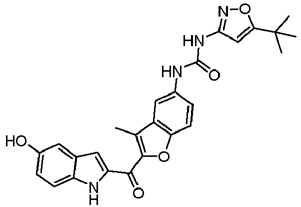 |
| **32c** | 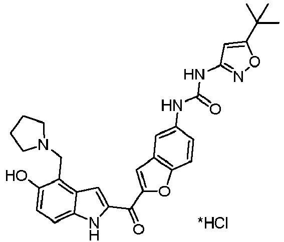 | | |
or a pharmaceutically acceptable salt or solvate thereof.

12. A composition, preferably pharmaceutical composition, comprising a compound or a pharmaceutically acceptable salt thereof, as defined in any one of the foregoing claims, and a pharmaceutically acceptable excipient and/or carrier;
wherein, optionally, said composition further comprises a further active agent, preferably a drug.

13. The compound according to any one of claims 1-11 or the composition according to claim 12 for use in medicine.

14. The compound or composition for use according to claim 12 for use in a method of preventing or treating cancer, preferably blood cancer, more preferably leukemia, even more preferably acute myeloid leukemia (AML);
wherein, optionally, said method of preventing or treating cancer comprises administering said compound or said composition to a patient, preferably a patient **characterized by** a mutation in the *Flt3* gene.

15. A method of preparing a compound as defined in any one of claims 1-11, comprising the steps:
(1a) heating a solution of a starting material, preferably a starting material having a structure represented by 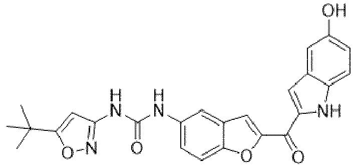 , in a solvent, preferably dimethylformamide, to a temperature in a range of from 60 °C to 100 °C, preferably of from 70 °C to 90 °C, more preferably of about 80 °C; and
adding an aldehyde, preferably formaldehyde, paraformaldehyde, or acetaldehyde, and a primary or secondary amine, preferably dimethylamine, piperidine, pyrrolidine, morpholine, or 1-methylpiperazine, to said solution;
(1b) deprotecting a benzyloxy group of a first intermediate compound obtained in step (1a);
(1c) reduction of a nitro group of said first intermediate compound;
(1d) reaction of a second intermediate compound obtained in step (1b) and/or (1c) with an isocyanate, preferably an isocyanate having a structure represented by 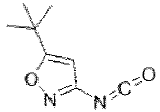 ; and
(1e) obtaining a compound as defined in any one of claims 1-11;
or comprising the steps:
(2aa) heating a solution of a starting material, preferably a starting material having a structure represented by 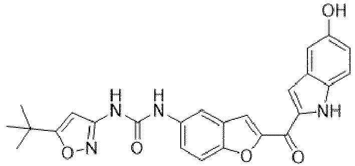 , in a solvent, preferably dimethylformamide or ethanol, to a temperature in a range of from 40 °C to 100 °C, preferably of from 50 °C to 90 °C, more preferably of from about 60 °C to about 80 °C; and adding an aldehyde, preferably formaldehyde, paraformaldehyde, or acetaldehyde, and a primary or secondary amine, preferably dimethylamine, piperidine, pyrrolidine, morpholine, or 1-methylpiperazine, to said solution; or
(2ab) mixing, preferably stirring, a primary or secondary amine and an aldehyde, preferably formaldehyde, paraformaldehyde, or acetaldehyde, dissolved in a carboxylic acid, preferably glacial acetic acid, and adding a starting material, preferably a starting material having a structure represented by 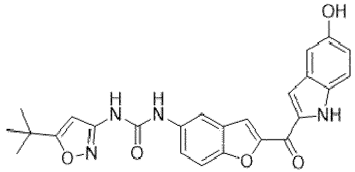 , dissolved in a carboxylic acid, preferably glacial acetic acid; and
(2b) adding to a solution obtained in step (2aa) or in step (2ab), optionally in a mixture of dichloromethane:pyridine, a solution of [1,4'-bipiperidine]-1'-carbonylchloride, optionally in a mixture of dichloromethane:pyridine; and
(2c) obtaining a compound as defined in any one of claims 1-11;
or comprising the steps:
(3a) acidic cleavage of a tert-butoxycarbonyl group of 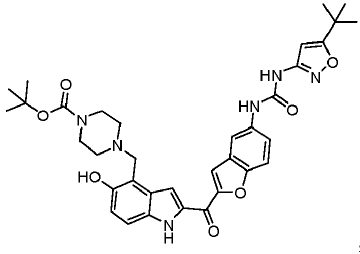 , preferably using trifluoro acetic acid;
(3b) reaction with an acid chloride or an acidic anhydride, preferably with acryloyl chloride or crotonic acid chloride; optionally in the presence of a base, preferably triethylamine, diisopropylethylamine, or pyridine; and
(3c) obtaining a compound as defined in any one of claims 1-11;
or comprising the steps:
(4a) hydrogenolytic cleavage of a benzyloxy group of a compound and reduction of a nitro group of said compound; wherein, optionally, said compound has a structure represented by 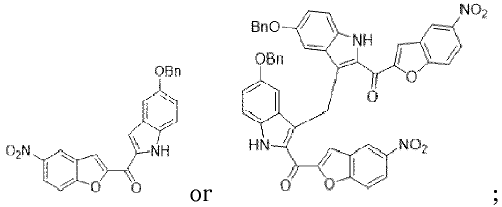
(4b) reaction of said compound with an isocyanate, preferably an isocyanate having a structure represented by 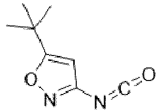 , to obtain an urea derivative of said compound; wherein, optionally, said urea derivative has a structure represented by 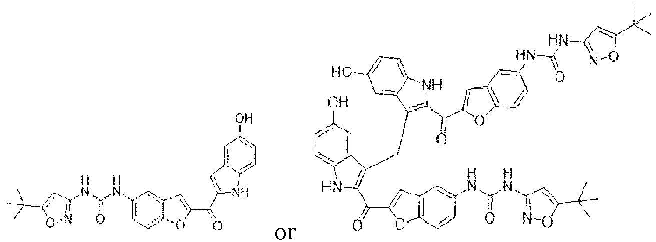 ; and
(4c) obtaining a compound as defined in any one of claims 1-11;
or comprising the steps:
(5a) aromatic nitration of a starting material, preferably a compound having a structure represented by 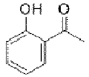 , to obtain a first intermediate compound, preferably a first intermediate compound having a structure represented by 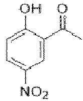 ; wherein, preferably, said aromatic nitration is performed using nitric acid and acetic acid;
(5b) reaction of 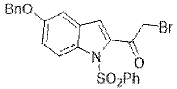 with said first intermediate compound in the presence of a base, preferably K₂CO₃, to obtain a second intermediate compound, preferably a second intermediate compound having a structure represented by 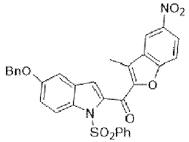 ;
(5c) cleavage of a phenylsulfonyl-protection group of said second intermediate compound by a base, preferably NaOH, to obtain a third intermediate compound, preferably a third intermediate compound having a structure represented by 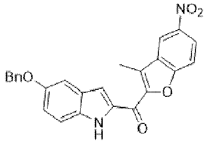 ;
(5d) catalytic hydrogenolytic cleavage of a benzyloxy group of said third intermediate compound and reaction of a nitro group of said third intermediate compound, preferably to obtain a fourth intermediate compound having a structure represented by 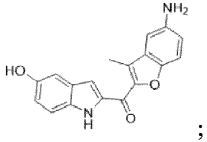
(5e) reaction with an isocyanate, preferably an isocyanate having a structure represented by 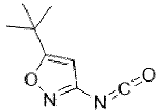 , to obtain an urea derivative, preferably a compound having a structure represented by 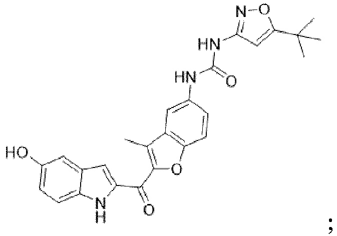 and
(5f) obtaining a compound as defined in any one of claims 1-11;
or comprising the steps:
(6a) transformation of a compound to a pharmaceutically acceptable salt by treatment with an acid, preferably an acid selected from the group consisting of hydrochloric acid, trifluoroacetic acid, and a sulfonic acid such as methanesulfonic acid; and
(6b) obtaining a compound as defined in any one of claims 1-11.
